(19) Europäisches Patentamt
European Patent Office
Office européen des brevets

(11) EP 2 900 638 B1

(12) FASCICULE DE BREVET EUROPEEN

(45) Date de publication et mention de la délivrance du brevet:
**04.01.2017 Bulletin 2017/01**

(21) Numéro de dépôt: **13766559.2**

(22) Date de dépôt: **27.09.2013**

(51) Int Cl.:
***C07D 213/81*** (2006.01) ***C07C 311/16*** (2006.01)
***C07D 417/04*** (2006.01) ***C07D 249/06*** (2006.01)
***C07D 249/08*** (2006.01) ***C07D 271/06*** (2006.01)
***C07D 277/26*** (2006.01) ***C07D 277/30*** (2006.01)
***C07D 277/56*** (2006.01) ***C07D 277/66*** (2006.01)
***C07D 285/08*** (2006.01) ***C07D 285/12*** (2006.01)
***C07D 291/04*** (2006.01) ***A61K 31/41*** (2006.01)
***A61K 31/4427*** (2006.01)

(86) Numéro de dépôt international:
**PCT/EP2013/070169**

(87) Numéro de publication internationale:
**WO 2014/049107 (03.04.2014 Gazette 2014/14)**

# (54) COMPOSES UTILISABLES DANS LE TRAITEMENT DES INFECTIONS MYCOBACTERIENNES

VERBINDUNGEN ZUR VERWENDUNG BEI DER BEHANDLUNG MYKOBAKTERIELLER INFEKTIONEN

COMPOUNDS FOR USE IN THE TREATMENT OF MYCOBACTERIAL INFECTIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.09.2012 FR 1202568**

(43) Date de publication de la demande:
**05.08.2015 Bulletin 2015/32**

(73) Titulaire: **Université de Droit et de la Santé de Lille 2**
**59800 Lille (FR)**

(72) Inventeurs:
- **WILLAND, Nicolas**
  **F-59000 Lille (FR)**
- **DEPREZ, Benoit**
  **F-59000 Lille (FR)**
- **BAULARD, Alain**
  **B-7520 Tournai (BE)**
- **BRODIN, Priscille**
  **F-75014 Paris (FR)**
- **SPERANDO, Olivier**
  **F-92390 Villeneuve-la-Garenne (FR)**
- **VILLERET, Vincent**
  **B-7861 Wannebecq (BE)**
- **VILLEMAGNE, Baptiste**
  **F-42170 Saint-Rambert (FR)**

(74) Mandataire: **Latscha Schöllhorn Partner AG**
**Austrasse 24**
**4051 Basel (CH)**

(56) Documents cités:
**EP-A1- 0 915 086**
**EP-A1- 1 024 134**
**WO-A1-2004/024702**
**WO-A1-2004/026848**
**WO-A1-2012/028676**
**WO-A2-2008/011131**
**US-A1- 2009 239 810**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

**Description**

La présente invention concerne des composés pour leur utilisation dans le traitement des infections bactériennes et mycobactériennes comme, par exemple, la tuberculose, la lèpre et les infections mycobactériennes atypiques.

La présente invention concerne également de nouveaux composés utilisables en tant que médicament en particulier en tant que médicament pour le traitement d'infections bactériennes et mycobactériennes comme, par exemple, la tuberculose, la lèpre et les infections mycobactériennes atypiques.

La présente invention concerne également des compositions pharmaceutiques comprenant en tant que principe actif au moins un des composés précités et éventuellement un antibiotique activable par la voie de l'EthA.

La présente invention concerne également des produits (kits) contenant au moins un des composés précités et au moins un antibiotique activable selon la voie de l'EthA comme produits de combinaison pour une utilisation simultanée, séparée ou étalée dans le temps en thérapie antituberculeuse, antilépromateuse ou antimycobactérienne générale.

La tuberculose tue 2 millions de personnes chaque année dans le monde. L'épidémie de sida et l'émergence de souches multi résistantes aux antibiotiques contribue à aggraver l'impact de cette maladie, considérée par l'Organisation Mondiale de la Santé comme responsable d'une épidémie mondiale de plus en plus dangereuse et comme une urgence sanitaire au niveau planétaire.

Un nombre croissant de souches de *Mycobacterium tuberculosis* est aujourd'hui caractérisé par une multi résistance aux antibiotiques de première ligne que sont l'isoniazide (INH) et la rifampicine (RIF). Ces antibiotiques présentent un indice thérapeutique élevé (l'indice thérapeutique d'un principe actif est le rapport de la dose thérapeutique à la dose toxique) et doivent alors être remplacés par des antibiotiques de seconde ligne comme l'éthionamide (ETH) auxquels les souches ne sont pas résistantes mais qui ont le désavantage de présenter un indice thérapeutique plus faible.

Une stratégie consistant à augmenter l'activité de l'éthionamide (ETH) en l'associant à un composé particulier a déjà été envisagée. En effet, l'ETH est une prodrogue qui est transformée *in vivo* en une forme thérapeutiquement active par l'enzyme EthA (voir l'article "Activation of the prodrug ethionamide is regulated in mycobacteria" 2000 Journal of Biological Chemistry). Les résistances observées à l'ETH viennent du fait que le répresseur transcriptionnel EthR de *M. tuberculosis* contrôle l'expression de l'enzyme EthA et limite voire empêche la transformation de l'ETH en substance thérapeutiquement active. La stratégie précitée est basée sur la combinaison de l'ETH et d'un composé qui inhibe EthR et supprime ainsi le contrôle de *M. tuberculosis* sur EthA. Ainsi le document "Ethionamide Boosters Combining Bioisosteric Replacement and Structure-Based Drug Design to Solve Pharmacokinetic Issues in a series of potent 1,2,4-Oxadiazole EthR Inhibitors" publié en 2012 dans la revue Journal of Medicinal Chemistry divulgue des composés susceptibles de potentialiser l'efficacité de l'éthionamide en inhibant EthR de M. *tuberculosis.*

Un but de la présente invention est de proposer de nouveaux composés susceptibles de potentialiser l'activité des antibiotiques activables par la voie EthA, en particulier l'éthionamide, par inhibition de EthR.

Un autre but de la présente invention est de proposer des composés tels que précités qui permettent en combinaison avec un antibiotique activable par la voie de l'EthA d'obtenir à dose d'antibiotique égale une meilleure efficacité ou qui permettent de réduire la dose d'antibiotique précitée pour obtenir une efficacité donnée.

Un autre but de la présente invention est de proposer des composés tels que précités qui soient simples et peu coûteux à produire.

Un autre but de la présente invention est de proposer des composés tels que précités qui soient solubles de manière satisfaisante dans un fluide biologique.

Un autre but de la présente invention est de proposer des composés tels que précités qui sont susceptibles d'être actifs en particulier par voie orale et/ou qui engendrent moins d'effets secondaires.

Pour atteindre au moins un des objectifs précités, la présente invention propose donc des composés de formule générale (I) :

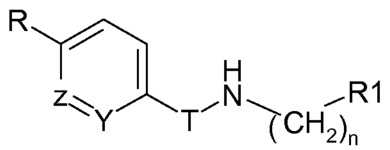

(I)

dans laquelle :

- Y et Z sont choisis indépendamment l'un de l'autre parmi CH et N ;
- T est choisi parmi CO ou $SO_2$ ;
- n est un entier supérieur ou égal à 1 et inférieur ou égal à 3 ;

R1 représente un groupement choisi parmi les chaînes alkyles en C1-C3, linéaires ou ramifiées, les chaînes alkyles en C1-C3 linéaires ou ramifiées et substituées, en particulier les chaînes alkyles en C1-C3 linéaires ou ramifiées et substituées par au moins un atome de fluor, les groupements cycliques en C3-C6, le groupement cyano, le groupement azido, les chaînes alkoxy en C1-C3, un groupement phényle non substitué, un groupement phényle substitué par un, deux ou trois substituants sélectionnés indépendamment les uns des autres parmi les chaînes alkyles linéaires ou ramifiées en C1-C3, le trifluorométhyl, les chaînes alkoxy linéaires ou ramifiées en C1-C3, les halogènes (F, Cl, Br, I), les hétérocycles à 6 sommets comportant 1 ou 2 atomes d'azote dans le cycle et les hétérocycles aromatiques à 5 sommets ;
R est choisi parmi les groupements azido, cyano, 2-benzothiazolyl, et les groupements (R-1) à (R-10) suivants :

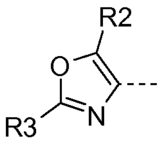
(R-1)

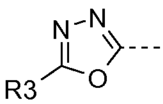
(R-2)

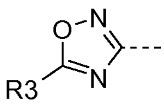
(R-3)

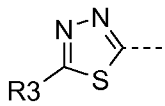
(R-4)

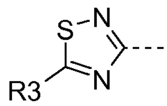
(R-5)

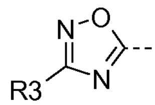
(R-6)

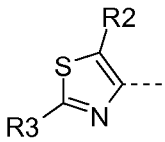
(R-7)

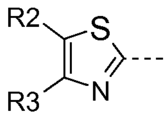
(R-8)

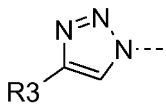
(R-9)

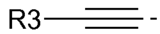
(R-10)

dans lesquels :

R2 est choisi parmi H, F, Cl, Br, I ;
R3 est un groupement sélectionné parmi H, les chaînes alkyles en C1-C6, linéaires ou ramifiées, les chaînes alkyles en C1-C6 linéaires ou ramifiées et substituées par au moins un atome de fluor, les groupements cycliques en C3-C6, le groupement cyanométhyl, le groupement azidométhyl, les chaînes alkoxy linéaires ou ramifiées en C1-C4, les groupements hydroxyalkyles en C1-C4, les groupements alkyle méthyl ester en C1-C4, les groupements alkyle methylcarbonylamino en C1-C4, les groupements alkyle méthylsulfone en C1-C4, le groupement phényle non substitué, un groupement phényle substitué par un, deux ou trois substituant(s) sélectionnés indépendamment les uns des autres parmi les chaînes alkyles en C1-C3, le trifluorométhyl, les chaînes alkoxy en C1-C3, les hétérocycles aromatiques comme, par exemple le 2-pyridinyle, le 3-pyridinyle, le 4-pyridinyle, les hétérocycles aromatiques substitués, en particulier les hétérocycles substitués par une chaîne alkyle linéaire ou ramifiée en C1-C5, les hétérocycles aromatiques substitués par au moins un atome de fluor, en particulier les hétérocycles aromatiques substitués par un, par deux ou par trois atomes de fluor ou par un groupement choisi parmi les groupements (II-a et II-b) suivants :

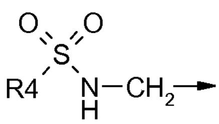
(II-a)

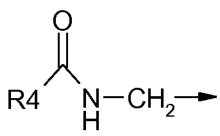
(II-b)

dans lesquels R4 est un groupement choisi parmi H, les chaînes alkyles linéaires ou ramifiées en C1-C4, le groupement phényle, un groupement phényle substitué par au moins un atome d'halogène, en particulier substitué par un atome d'halogène, un groupement phényle substitué par une chaîne alkyle linéaire ou ramifiée en C1-C4, un groupement phényle substitué par une chaîne alkoxy linéaire ou ramifiée en C1-C4 et un groupement phényle substitué par un groupement trifluorométhyl ;
pour leur utilisation en tant que médicament, en particulier pour leur utilisation dans le traitement des infections bactériennes et mycobactériennes, notamment dans le traitement de la tuberculose, de la lèpre ou des mycobactérioses atypiques.

Par «groupements alkyle méthyl ester en C1-C4 », on entend, au sens de la présente invention, que le radical alkyl comporte de 1 à 4 atomes de carbone et que le radical méthyle est lié à l'un quelconque des groupements R-1 à R-10.

Dans toute la description de la présente invention, les termes « chaînes alkyles substituées » représentent, quel que soit le nombre de carbone de la chaîne considérée, les chaînes alkyles dont au moins un hydrogène lié à un atome de carbone est substitué ; lorsque la chaîne comporte au moins deux substituants, ces substituants peuvent être liés au même atome de carbone ou à des atomes de carbone différents.

Lorsque le substituant de la chaîne alkyle est explicitement indiqué, la position de ce dernier n'est pas limitée selon la présente invention.

Lorsqu'au moins deux substituant(s) de la chaîne alkyle sont explicitement indiqués, ces derniers peuvent être liés au même carbone ou à des carbones différents.

Les termes « chaînes alkoxy » désignent toutes les chaînes alkoxy comportant le nombre de carbone indiqué et un atome d'oxygène reliant deux atomes de carbone de la chaîne, quelle que soit la position de ce dernier.

On définit ici les mycobactérioses atypiques comme étant les mycobactérioses causées par au moins une mycobactérie autre que *M. Tuberculinum* et en particulier les mycobactérioses impliquant *M. Kansasii.*

Dans toute la présente demande, les chaînes précitées qui sont substituées par au moins un atome d'halogène peuvent être en particulier substituées par un atome d'halogène, deux atomes d'halogène ou trois atomes d'halogène. Les atomes d'halogène peuvent être différents les uns des autres sur une même chaîne. Les substituants halogénés sont choisis indépendamment les uns des autres. Ils peuvent substituer un même atome de carbone ou des atomes de carbone différents.

La présente invention concerne également des composés qui correspondent à la formule (III) suivante :

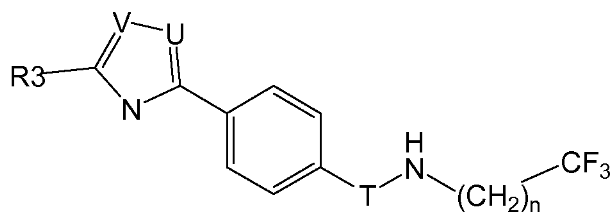

(III)

dans laquelle U est choisi parmi CH et N ; et V est choisi parmi O ou S ; n, T et R3 étant tels que précédemment définis.

La présente invention concerne également des composés de formule (IV) suivante :

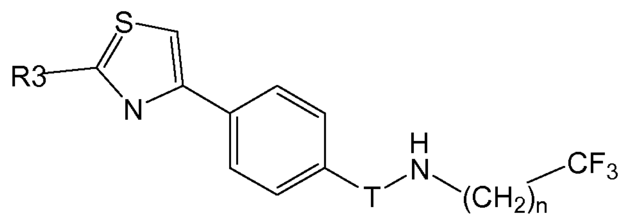

(IV)

dans laquelle n, T et R3 sont définis comme précédemment.

Avantageusement, T = CO et R3 est choisi parmi les groupements suivants :$-CH_2SO_2R'$ où le radical R' est un groupement tertiobutyle, méthyle, isobutyle, isopentyle, isohexyle, 4-pyridinyle et 4-pyridinyle substitué par une chaîne alkyle linéaire en C1-C4.

Avantageusement, R3 peut être, quels que soient n et T, un groupement 4-pyridinyle substitué par un groupe méthyle ou une chaîne éthyle. Dans ce cas, avantageusement, le cycle est substitué en position 3, c'est-à-dire en position ortho par rapport à l'atome d'azote du groupement pyridinyle.

La présente invention concerne également les composés précités pour leur utilisation en tant que médicament, en particulier, pour leur utilisation dans le traitement des infections bactériennes et mycobactériennes, notamment dans le traitement de la tuberculose, de la lèpre ou de mycobactérioses atypiques.

La présente invention concerne également une composition pharmaceutique comprenant en tant que principe actif au moins un composé tel que précité, en particulier un composé de formule générale (III) ou (IV) et/ou au moins un composé de formule générale (I) telle que précitée et un excipient pharmaceutiquement acceptable.

Au sein des compositions pharmaceutiques conformes à l'invention, le ou les composés utilisés en tant qu'ingrédient(s) actif(s) peuvent être utilisés en une quantité permettant d'administrer des doses unitaires comprises entre 0,3 mg et 1 g environ. Au sein des compositions pharmaceutiques conformes à l'invention et lorsqu'il(s) est(sont) présent(s), le ou les antibiotiques activables par la voie enzymatique de l'EthA sont avantageusement utilisés en une quantité permettant d'administrer des doses unitaires égales ou inférieures aux doses habituellement recommandées par l'OMS (WHO, Treatment of tuberculosis: Guidelines for National Programmes. 2003; WHO/CDS/TB2003.313.), les organisations sanitaires nationales ou non gouvernementales, ou les laboratoires pharmaceutiques compétents.

L'Homme du Métier est à même de choisir un ou plusieurs excipients pharmaceutiquement acceptables en fonction de la voie d'administration de la composition pharmaceutique. Bien entendu, l'Homme du Métier veillera à cette occasion à ce que le ou les excipients utilisés soient compatibles avec les propriétés intrinsèques attachées à la composition conforme à la présente invention. En outre, la forme du médicament ou de la composition pharmaceutique (par exemple, une solution, une suspension, une émulsion, des comprimés, des gélules, des suppositoires, etc...) dépendra de la voie d'administration choisie.

Ainsi, au sens de la présente invention, le médicament ou la composition pharmaceutique peut être administré(e) par n'importe quelle voie appropriée, par exemple par la voie orale, anale, locale (topique, par exemple), systémique, intraveineuse, intramusculaire ou mucosale, ou bien en utilisant un patch, ou encore sous forme encapsulée dans, ou immobilisée sur, des liposomes, des microparticules, des microcapsules, associée à des nanoparticules et analogues. On peut notamment citer, à titre d'exemples non limitatifs d'excipients appropriés pour une administration par voie orale, le talc, le lactose, l'amidon et ses dérivés, la cellulose et ses dérivés, les polyéthylèneglycols, les polymères d'acide acrylique, la gélatine, le stéarate de magnésium, des matières grasses animales, végétales ou synthétiques, les dérivés de la paraffine, les glycols, les stabilisants, les conservateurs, les anti-oxydants, les agents mouillants, les anti-agglomérants, les dispersants, les émulsionnants, les agents modifiants du goût, les agents de pénétrations, de solubilisation, etc....Les techniques de formulation et d'administration des médicaments et compositions pharmaceutiques sont bien connues dans la technique ici considérée, l'Homme du Métier pouvant notamment se référer à l'ouvrage Remington's Pharmaceutical Sciences, dernière édition.

La présente Invention a également pour objet l'utilisation d'au moins un composé selon l'invention pour la fabrication d'un médicament destiné à la prévention et/ou au traitement des infections bactériennes, de préférence mycobactériennes, et tout particulièrement de la tuberculose, de la lèpre ou de mycobactérioses atypiques.

Avantageusement, la composition pharmaceutique comprend en outre, en tant que principe actif, au moins un antibiotique choisi parmi les antibiotiques activables par la voie enzymatique de l'EthA. L'éthionamide, le prothionamide, l'isoxyl et le thiacétazone sont des exemples d'antibiotiques activables par la voie EthA. La présente invention n'est néanmoins pas limitée à ces antibiotiques.

Au sens de la présente invention, le terme « traitement » désigne le traitement curatif et/ou le traitement prophylactique des infections précitées. Le terme « traitement » englobe toute amélioration de l'état du patient, en particulier toute diminution de la quantité de bactéries présentes dans un moins un site d'infection du patient.

On définit au sens de la présente invention, un antibiotique activable par la voie de l'EthA, toute substance qui, au moins *in vitro* réagit avec l'enzyme EthA pour produire une substance ayant des propriétés antibiotiques. L'Homme du Métier est à même de déterminer si un antibiotique est activable par la voie de l'EthA, par exemple, en appliquant la méthode décrite dans la publication suivante : "Activation of the prodrug ethionamide is regulated in mycobacteria" 2000, Journal of Biological Chemistry.

***PARTIE EXPERIMENTALE***

**Synthèse micro-ondes**

La synthèse micro-ondes a été réalisée sur un four micro-ondes CEM Discover™.

**Analyse par chromatographie sur couche mince**

Les chromatographies sur couche mince (CCM) ont été réalisées sur des plaques d'aluminium recouvertes d'une couche de 0,25mm d'épaisseur de gel de silice 60 $F_{254}$ Merck.

**Analyses LC-MS**

Les analyses par LC-MS ont été réalisées sur deux appareils :

- Un appareil Varian 1200ws triple quadrupole. La détection UV se fait aux longeurs d'ondes 215 et 254nm.
- Un appareil Waters Alliance Micromass ZQ 2000. La détection se fait à l'aide d'un système Waters 2996 diode array.

Ces appareils sont équipés d'une colonne C18 TSK-gel Super ODS 2μm (50 x 4.6mm) ou d'une colonne XBridge C18 (Waters) 5μm (50x4.6mm). Les volumes d'injection sont de 20μL.
Deux gradients ont été utilisés :

- 10min : 100% $H_2O$ / 0.1% HCOOH jusqu'à 95% ACN / 0.1% HCOOH en 7 minutes avec un débit de 1 $mL.min^{-1}$.
- 5min : 100% $H_2O$/0.1 % HCOOH jusqu'à 95% ACN / 0.1% HCOOH en 3 minutes avec un débit de 2$mL.min^{-1}$.

<u>Méthode a :</u> Varian, gradient 10min, colonne SODS
<u>Méthode b :</u> Waters, gradient 10min, colonne SODS
<u>Méthode c :</u> Waters, gradient 5min, colonne XBridge
<u>Méthode d :</u> Waters, gradient 10min, colonne XBridge

**Analyses RMN**

Les spectres de résonance magnétique nucléaire (RMN) ont été effectués sur un spectromètre Brücker DRX 300MHz. Les déplacements chimiques δ sont exprimés en ppm par rapport au TMS et les constantes de couplage J en Hz (Hertz). Les pics sont décrits selon le modèle : δ (nature du massif, nombre de protons par intégration, J).
Les abréviations suivantes ont été utilisées pour la multiplicité des signaux : s=singulet, d=doublet, t=triplet, q=quadruplet, qn=quintuplet, sx=sextuplet, sp=septuplet, n=nonuplet, br=broad (large).

**Purification par chromatographie sur colonne de silice prépaquée**

Les chromatographies sur colonne de silice prépaquées ont été réalisées sur des colonnes de marque AIT Chromato de granulométrie 20 à 40 μm ou Grace Reveleris de granulométrie 40μm, à l'aide d'une pompe Flashmart.

**Purification par Chromatographie Liquide Haute Pression (HPLC)**

Les purifications par HPLC préparative ont été réalisées à l'aide d'un appareil Varian ProStar utilisant une colonne Omnisphere 10 $C_{18}$ 250mm x 41.4mm Dynamax. Le gradient utilisé part d'un mélange (20% d'Acétonitrile/80% d'Eau/0.1% d'Acide formique) jusqu'à un mélange (100% Acétonitrile/0.1% Acide formique). La détection est réalisée aux longueurs d'onde 215 et 254 nm.

**Point de fusion**

Les points de fusion des produits recristallisés ont été mesurés par la méthode des capillaires, sur un appareil Büchi B-540.

## Scheme 1:

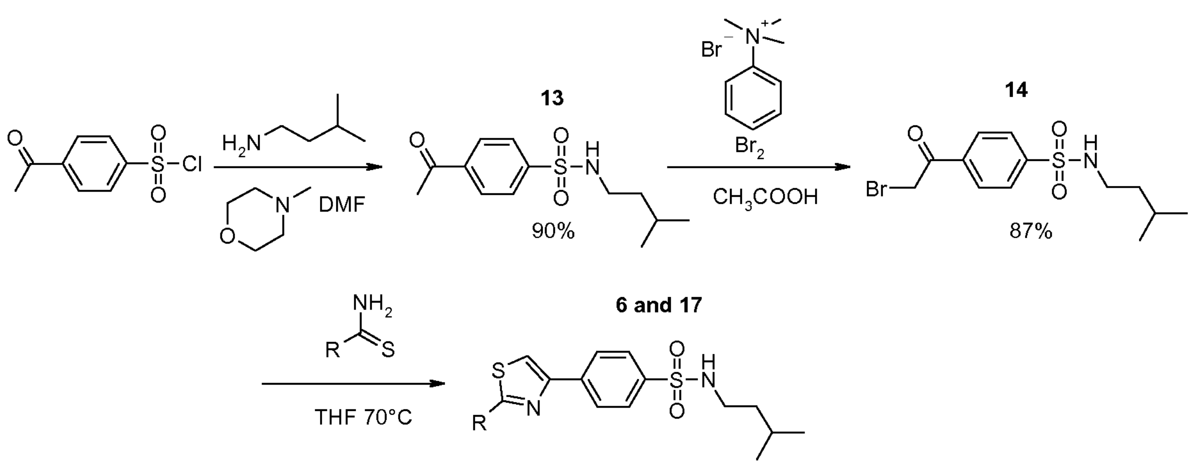


**Composé 13 : 4-acétyl-N-isopentylbenzènesulfonamide**

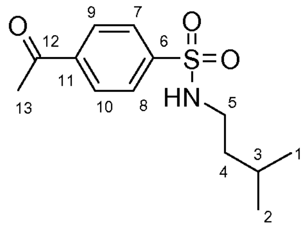

3g (1 eq.) de chlorure d'acide 4-acétylbenzènesulfonique sont ajoutés à une solution de 2.39mL (1.5eq.) de 3-Méthyl-butylamine et 7.54mL (5eq.) de N-méthylmorpholine dans 200mL de DMF sur tamis moléculaire. Le mélange est agité pendant 3h à TA. Le DMF est évaporé sous pression réduite. Le résidu est repris dans l'acétate d'éthyle puis lavé à l'aide d'une solution aqueuse d'HCl 1 N (2x) et à la saumure (1x). Les phases organiques sont séchées sur sulfate de magnésium puis concentrées sous pression réduite pour donner 3.34g (90%) de poudre beige.

LC-MS : $t_R$ = 5.61 min (méthode a) ; m/z : $[M+H]^+$ = 270
CCM : $R_f$ = 0.44 (AcOEt 3:7 Ether de Pétrole)
RMN $^1H$ ($CD_3OD$, 300MHz) : $\delta$ ppm = 8.16 (d, 2H, $^3J$ = 8.67Hz, 9+10); 7.95 (d, 2H, $^3J$ = 8.67Hz, 7+8); 2.89 (t, 2H, $^3J$ = 7.16Hz); 2.65 (s, 3H, 13); 1.61 (n, 1H, $^3J$ = 6.72Hz, 3); 1.32 (q, 2H, $^3J$ = 7.08Hz, 4); 0.84 (d, 6H, $^3J$ = 6.62Hz, 1+2)

**Composé 14 : 4-(2-bromoacétyl)-N-isopentylbenzènesulfonamide**

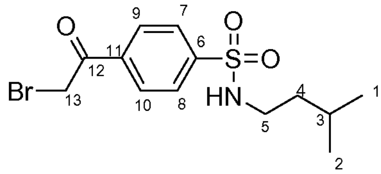

4.466g (1 eq.) de tribromure de triméthylphénylammonium sont ajoutés à une solution de 3.2g (1eq.) de 4-acétyl-N-isopentylbenzènesulfonamide (13) dans 150mL d'acide acétique glacial. Le mélange réactionnel est agité pendant 3h à TA. L'acide acétique est évaporé sous pression réduite, le solide jaune obtenu est repris dans l'acétate d'éthyle puis lavé à l'eau (2x) et à la saumure (1x). La phase organique est séchée sur sulfate de magnésium puis évaporée sous pression réduite pour donner 4.02g (quantitatif) d'une huile jaune.

LC-MS : $t_R$ = 6.26min (méthode a) ; m/z : $[M+H]^+$ = 349
RMN $^1H$ ($CD_3OD$, 300MHz) : $\delta$ ppm = 8.18 (d, 2H, $^3J$ = 8.70Hz, 7-8); 7.98 (d, 2H, $^3J$ = 8.70Hz, 9-10); 4.71 (s, 2H, 13); 2.90 (t, 2H, $^3J$ = 7.10Hz, 5); 1.62 (n, 1H, $^3J$ = 6.70Hz, 3); 1.32 (q, 2H, $^3J$ = 6.90Hz, 4); 0.84 (d, 6H, $^3J$ = 6.60Hz, 1+2)

**Composé 6 : 4-(2-méthylthiazol-4-yl)-N-isopentylbenzènesulfonamide**

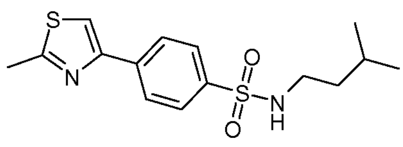

LC-MS : $t_R$ = 6.4min (méthode a) ; m/z : $[M+H]^+$ = 325
CCM : $R_f$ = 0.34 (AcOEt 3:7 Ether de Pétrole)
RMN $^1H$ ($CD_2Cl_2$, 300 MHz): $\delta$ ppm = 8.07 (d, 2H, J=8.5Hz), 7.9 (d, 2H, J=8.5Hz), 7.56 (s, 1 H), 4.48 (t,1 H, J=5.7Hz), 2.98 (m, 2H), 2.8 (s, 3H), 1.59 (n, 1H, J=6.7Hz), 1.13 (sextuplet, 2H, J=7.22Hz), 0.86 (d, 6H, J=6.78Hz)

**Composé 17 : 4-(2-(cyanométhyl)thiazol-4-yl)-N-isopentylbenzènesulfonamide**

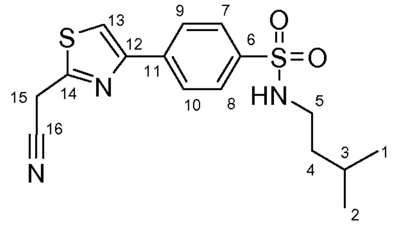

575mg (1 eq.) de 2-cyanothioacetamide sont ajoutés à une solution de 2g (1 eq.) de 4-(2-bromoacétyl)-N-isopentylbenzènesulfonamide (14) dans 200mL de THF sur tamis moléculaire. Le mélange réactionnel est agité au reflux du THF pendant 28h. Le THF est ensuite évaporé sous pression réduite puis le solide obtenu est repris dans l'acétate d'éthyle, lavé à l'eau (2x) puis à la saumure (1x). La phase organique est séchée sur sulfate de magnésium puis évaporée sous pression réduite. Le solide marron est recristallisé dans 5mL d'éthanol pour donner 946mg (47%) de poudre jaune pâle.

<u>LC-MS :</u> $t_R$ = 5.67min (méthode a) ; m/z : $[M+H]^+$ = 350
<u>RMN</u> $^1H$ ($CD_3OD$, 300MHz) : δ ppm = 8.15 (d,2H, $^3J$ = 8.70Hz, 9+10); 8.05 (s, 1 H, 13); 7.90 (d, 2H, $^3J$ = 8.70Hz, 7+8); 4.43 (s, 1 H, 15); 2.90 (t, 2H, $^3J$ = 7.20Hz, 5); 1.52-1.68 (m, 1 H, 3): 1.33 (q, 2H, $^3J$ = 7.10Hz, 4); 0.84 (d, 6H, $^3J$ = 6.65Hz, 1+2)
<u>RMN</u> $^{13}C$ ($CD_3OD$, 75MHz) : δ ppm = 159.4 (14); 153.7 (12); 139.9 (11); 137.5 (6); 127.2 (7+8); 126.5 (9+10); 117.0 (13); 115.9 (16); 41.0 (5); 38.1 (4); 25 (3); 21.2 (1+2); 20.9 (15)

## Scheme 2:

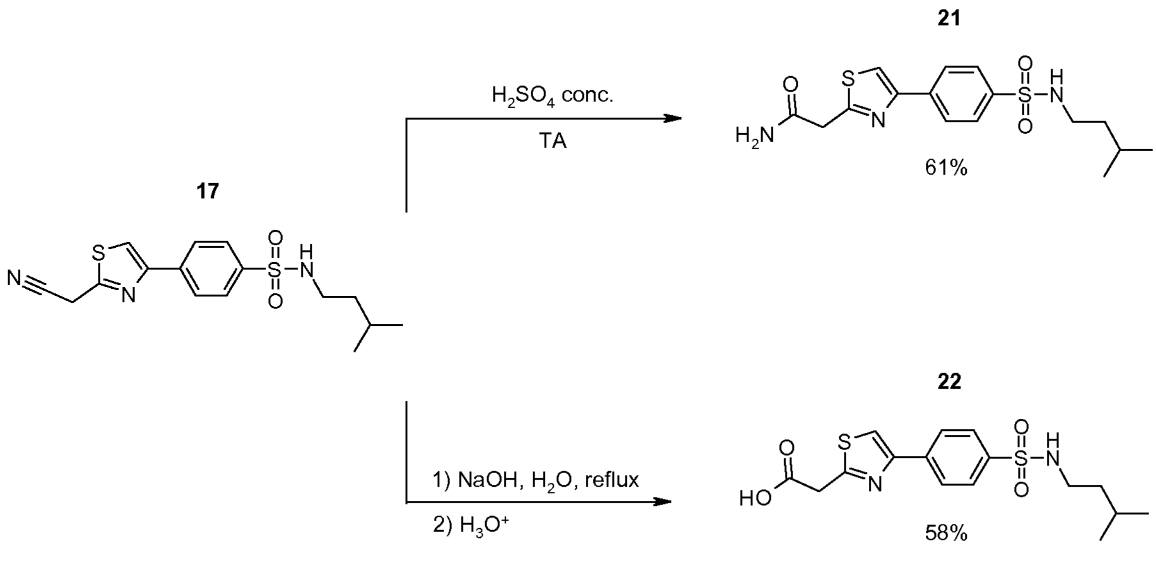


**Composé 21 : 2-(4-(4-(N-isopentylsulfamoyl)phényl)thiazol-2-yl)acétamide**

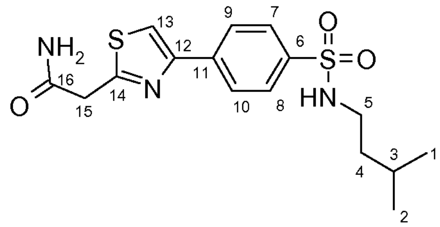

100mg de 4-(2-(cyanométhyl)thiazol-4-yl)-N-isopentylbenzènesulfonamide (17) sont solubilisés dans 100µL d'acide sulfurique à 96%. La solution est agitée à TA pendant 12h. Le milieu réactionnel est alors versé dans 100µL une solution d'ammoniaque à 28% à 0°C. De l'ammoniaque est ensuite ajouté jusqu'à atteindre un pH de 9. La solution est alors filtrée, le pH du filtrat est neutralisé avec de l'HCl 1N puis extrait à l'acétate d'éthyle. Les phases organiques sont rassemblées, séchées sur sulfate de magnésium puis évaporées sous pression réduite pour donner 64mg (61%) de poudre blanche.

LC-MS : $t_R$ = 5.20min (méthode a) ; m/z : $[M+H]^+$ = 368
RMN $^1H$ ($CD_3OD$, 300MHz) : δ ppm = 8.12 (d, 2H, $^3J$ = 8.61 Hz, 9+10); 7.97 (s, 1H, 13); 7.89 (d, 2H, $^3J$ = 8.61 Hz, 7+8); 4.05 (s, 1 H, 15); 2.90 (t, 2H, $^3J$ = 7.18Hz, 5); 1.62 (n, 1 H, $^3J$ = 6.40Hz, 3); 1.34 (q, 2H, $^3J$ = 7.08Hz, 4); 0.84 (d, 6H, $^3J$ = 6.62Hz, 1+2)
RMN $^{13}C$ ($CD_3OD$, 75MHz) : δ ppm = 173.2 (16); 165.9 (14); 154.4 (12); 141.2 (11); 139.6 (6); 128.7 (7+8); 127.9 (9+10); 118.1 (13); 42.5 (5); 39.7 (4); 26.6 (3); 22.8 (1+2)

**Composé 22 : acide 2-(4-(4-(N-isopentylsulfamoyl)phényl)thiazol-2-yl)acétique**

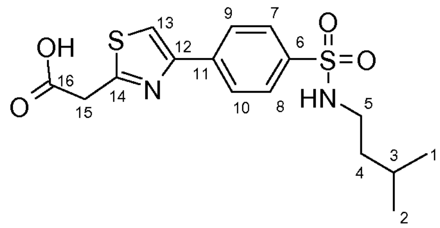

Une solution de 200mg (1 eq.) de 4-(2-(cyanométhyl)thiazol-4-yl)-N-isopentylbenzènesulfonamide **(17)** dans 2.5mL d'eau et 45.8mg (2eq.) de NaOH est chauffée au reflux pendant 15h. Le pH est alors ajusté à 4-5 à TA à l'aide d'une solution d'acide acétique glacial. Le produit est extrait à l'acétate d'éthyle. Les phases organiques sont rassemblées, lavées avec une solution aqueuse d'HCl de pH=4, séchées sur sulfate de magnésium puis évaporées sous pression réduite pour donner 122mg (58%) de poudre beige.

LC-MS : $t_R$ = 5.07min (méthode a) ; m/z : $[M+H]^+$ = 369
RMN $^1H$ ($CD_3OD$, 300MHz) : δ ppm = 8.11 (d, 2H, $^3J$ = 8.80Hz, 9+10); 7.97 (s, 1 H, 13); 7.89 (d, 2H, $^3J$ = 8.80Hz, 7+8); 2.90 (t, 2H, $^3J$ = 7.30Hz, 5); 1.61 (n, 1H, $^3J$ = 6.70Hz, 3); 1.34 (q, 2H, $^3J$ = 7.20Hz, 4); 0.84 (d, 6H, $^3J$ = 6.60Hz, 1+2)

**Scheme 3 :**

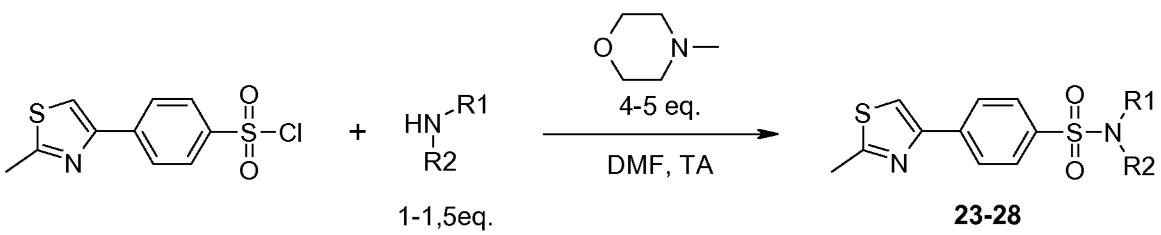


**23-28**

**Procédure générale (i)**

On ajoute successivement 99µL (5eq.) de N-méthylmorpholine et entre 1 et 1.5 eq. de l'amine correspondante à une solution de 50mg (1 eq.) de chlorure d'acide 4-(2-méthylthiazol-4-yl)benzènesulfonique dans 4mL de DMF anhydre. Le mélange est agité à TA pendant 2h. Le DMF est évaporé sous pression réduite. Le produit est repris dans l'acétate d'éthyle, lavé à l'eau (2x) puis à la saumure (1x). Les phases organiques sont séchées sur sulfate de magnésium puis évaporées sous pression réduite.

**Composé 23 : N-(3,3-diméthylbutyl)-4-(2-méthylthiazol-4-yl)benzènesulfonamide**

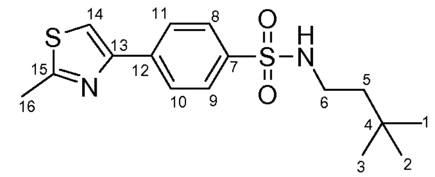

Procédure (i) avec 38.7µL (1.5 eq.) de 3,3-diméthylbutylamine.
26.5mg (44%) de poudre beige.

LC-MS : $t_R$ = 6.79min (méthode a) ; m/z : $[M+H]^+$ = 339
RMN $^1H$ (DMSO-$d_6$, 300MHz) : δ ppm = 8.09 (d, 2H, $^3J$ = 8.70Hz, 10+11); 7.89 (d, 2H, $^3J$ = 8.70Hz, 8+9); 7.84 (s, 1H, 14); 2.86-2.92 (m, 2H, 6); 2.77 (s, 3H, 16); 1.35-1.40 (m, 2H, 5); 0.85 (s, 9H, 1+2 +3)
RMN $^{13}C$ (DMSO-$d_6$, 75MHz) : δ ppm = 168.7 (15); 154.4 (13); 140.9 (7); 139.5 (12); 128.6 (8+9); 127.8 (10+11); 116.9 (14); 44.3 (5); 40.8 (6); 30.5 (4); 29.7 (1+2+3); 18.9 (16).

**Composé 24 : 4-(2-méthylthiazol-4-yl)-N-(2,2,2-trifluoropropyl)benzènesulfonamide**

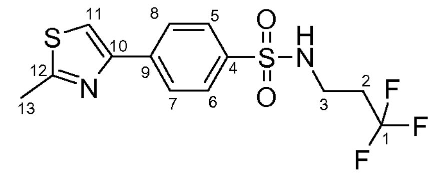

Procédure (i) avec 26.9mg (1eq.) de chlorhydrate de 2,2,2-trifluoropropylamine.
26.6mg (39%) de poudre beige.

LC-MS : $t_R$ = 5.80min (méthode a) ; m/z : $[M+H]^+$ = 351
RMN $^1H$ ($CD_3OD$, 300MHz) : δ ppm = 8.10 (d, 2H, $^3J$ = 8.80Hz, 7+8); 7.90 (d, 2H, $^3J$ = 8.80Hz, 5+6); 7.86 (s, 1H, 11); 3.12 (t, 2H, $^3J$ = 7.30Hz, 3); 2.77 (s, 3H, 13); .2.39 (qt, 2H, $^3J_{2-1}$ = 10.80Hz, $^3J_{2-3}$ = 7.30Hz, 2)
RMN $^{13}C$ ($CD_3OD$, 75MHz) : δ ppm = 168.7 (12); 154.3 (10); 140.5 (4); 139.8 (9); 128.6 (5+6); 127.9 (7+8); 127.6 (q, $^1J_{C-F}$ = 276.1 Hz, 1); 117.1 (11); 37.39 (d, $^3J_{C-F}$ =3.10Hz ; 3); 35.21 (q, $^2J_{C-F}$ = 27.9Hz, 2); 18.9 (13).

**Composé 25 : 4-(2-méthylthiazol-4-yl)-N-(2,2,3,3,3-pentafluoropropyl)benzènesulfonamide**

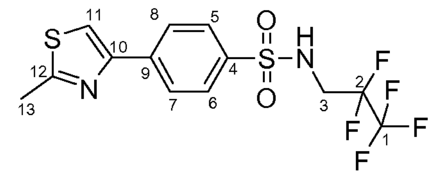

Procédure (i) avec 38.9µL (1 eq.) de 2,2,3,3,3-pentafluoropropylamine, 100mg de chlorure d'acide sulfonique, 8mL de DMF anhydre et 198µL de N-méthylmorpholine.
40.1 mg (29%) de poudre beige.

LC-MS : $t_R$ = 6.22min (méthode a) ; m/z : $[M+H]^+$ = 387
RMN $^1H$ ($CD_3OD$, 300MHz) : δ ppm = 8.10 (d, 2H, $^3J$ = 8.80Hz, 7+8); 7.91 (d, 2H, $^3J$ = 8.80Hz, 5+6); 7.86 (s, 1 H, 11); 3.70 (td, 2H, $^3J_{3-2}$ = 15.80Hz, $^4J_{3-1}$ = 0.8Hz, 3); 2.77 (s, 3H, 13).

**Composé 26 : 4-(2-méthylthiazol-4-yl)-N-(2,2,2-trifluoroéthyl)benzènesulfonamide**

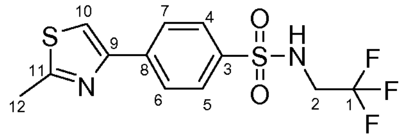

Procédure (i) avec 24.4mg (1 eq.) de chlorhydrate de 2,2,2-trifluoroéthylamine.
19.8mg (31%) de poudre beige.

LC-MS : $t_R$ = 5.53min (méthode a) ; m/z : $[M+H]^+$ = 337
RMN $^1H$ ($CD_3OD$, 300MHz) : δ ppm = 8.09 (d, 2H, $^3J$ = 8.80Hz, 6+7); 7.91 (d, 2H, $^3J$ = 8.80Hz, 4+5); 7.85 (s, 1H, 10); 3.68 (q, 2H, $^3J$ = 9.00Hz, 2); 2.80 (s, 3H, 12)
RMN $^{13}C$ ($CD_3OD$, 75MHz) : δ ppm = 168.7 (11); 154.3 (9); 141.2 (8); 139.8 (3); 125.6 (q, $^1J_{C-F}$ = 277Hz, 1); 117.1 (10); 45.06 (q, $^2J_{C-F}$ = 35.0Hz, 2); 12.9 (12).

**Composé 27 : 4-(2-méthylthiazol-4-yl)-N-(4,4,4-trifluorobutyl)benzènesulfonamide**

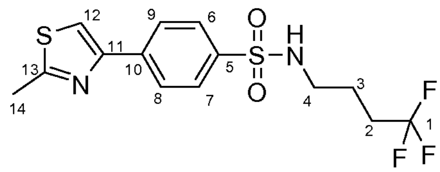

Procédure (i) avec 55.7mg (1.2eq.) de 4,4,4-trifluorobutylamine, 100mg de chlorure d'acide sulfonique, 5mL de DMF anhydre et 198µL de N-méthylmorpholine.
75.1 mg (54%) de poudre beige.

LC-MS : $t_R$ = 6.13min (méthode b) ; m/z : $[M+H]^+$ = 365
RMN $^1H$ ($CD_3OD$, 300MHz) : δ ppm = 8.02 (d, 2H, $^3J$ = 8.74Hz, 8+9); 7.89 (d, 2H, $^3J$ = 8.74Hz, 6+7); 7.46 (s, 1H, 12); 5.10 (t, 1H, $^3J$ = 6.39Hz, NH); 3.03 (q, 2H, $^3J$ = 6.64Hz, 4); 2.22-2.06 (m, 2H, 2); 1.76 (qn, 2H, $^3J$ = 7.33Hz, 3)
RMN $^{13}C$ ($CDCl_3$, 75MHz) : δ ppm = 166.8 (13); 153.2 (11); 138.8 (10); 138.5 (5); 127.6 (6+7); 127.0 (8+9); 126.9 (q, $^1J_{C-F}$ = 276Hz, 1); 115.2 (12); 42.0 (4); 30.9 (q, $^2J_{C-F}$ = 29.0Hz, 2); 22.6 (3); 19.4 (14).

**Composé 28: N-(2,2,3,3,4,4,4-héptafluorobutyl)-4-(2-méthylthiazol-4-yl)benzènesulfonamide**

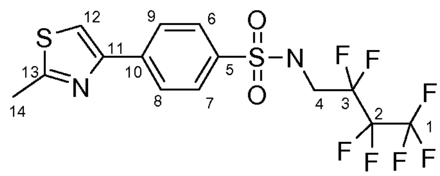

Procédure (i) avec 29.2µL (1.2eq.) de 2,2,3,3,4,4,4-héptafluorobutylamine. 34mg (44%) de poudre beige.

LC-MS : $t_R$ = 6.65min (méthode b) ; m/z : $[M+H]^+$ = 437
RMN $^1H$ ($CDCl_3$, 300MHz) : δ ppm = 8.02 (d, 2H, $^3J$ = 8.70Hz, 8+9); 7.88 (d, 2H, $^3J$ = 8.70Hz, 6+7); 7.49 (s, 1 H, 12); 5.50 (br s, 1 H, NH); 3.75 (td, 2H, $^3J_{4-3}$ = 15.30Hz, $^4J_{4-2}$ = 5.10Hz, 4); 2.80 (s, 3H, 14)
RMN $^{19}F$ découplé $^1H$ ($CDCl_3$, 282MHz) : δ ppm =-127.36/-127.51 (m, 2F, 3); -118.74 (qt, 2F, $^3J_{2-1}$ = 9.30Hz, $^3J_{2-3}$ = 5.60Hz, 2); -80.71 (t, 3F, $^3J$ = 9.30Hz, 1).

## Scheme 4:

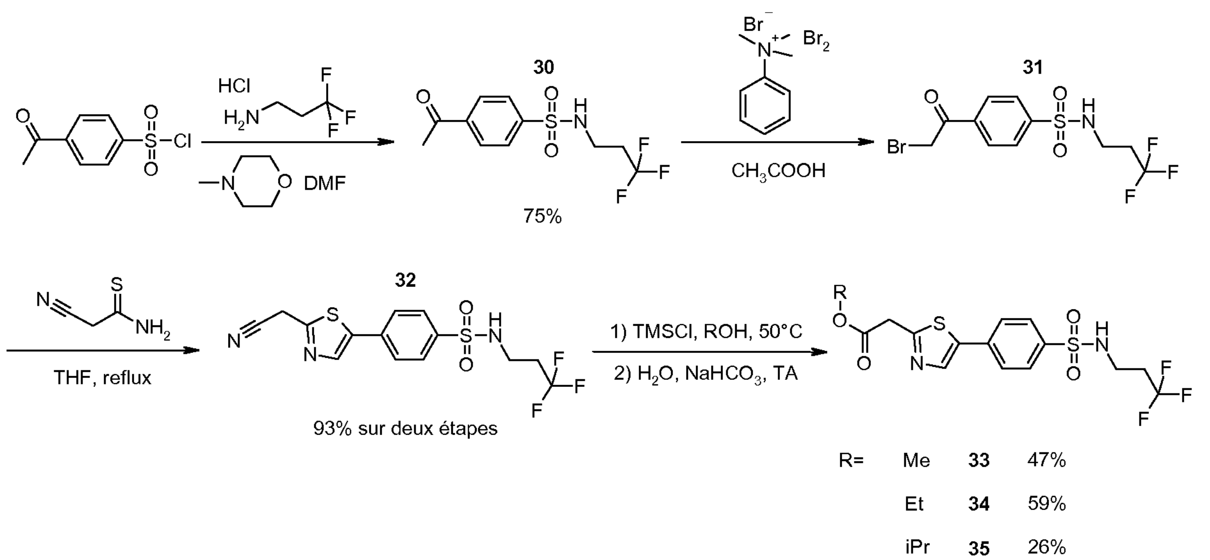


**Composé 30 : 4-acétyl-N-(3,3,3-trifluoropropyl)benzènesulfonamide**

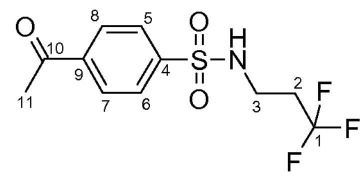

754µL (5eq.) de N-méthylmorpholine et 300mg (1 eq.) de chlorure d'acide 4-acétylbenzènesulfonique sont successivement ajoutés à une solution de 186mg (1.2eq.) de chlorhydrate de 3,3,3-trifluoropropylamine dans 10mL de DMF anhydre. Le mélange est agité pendant 2h à TA. Le DMF est évaporé sous pression réduite. Le produit est repris dans l'acétate d'éthyle puis lavé à l'aide d'une solution aqueuse d'HCl 1 N (2x) puis à la saumure (1x). Les phases organiques sont séchées sur sulfate de magnésium puis concentrées sous pression réduite pour donner 303mg (75%) de poudre jaune clair.

LC-MS: $t_R$ = 5.33min (méthode a) ; m/z : $[M+H]^+$ = 296
RMN $^1H$ (DMSO-$d_6$, 300MHz) : δ ppm = 8.15 (d, 2H, $^3J$ = 8.60Hz, 7+8); 8.09 (br s, 1 H, NH); 7.93 (d, 2H, $^3J$ = 8.60Hz, 5+6); 2.99 (t, 2H, $^3J$ = 6.90Hz, 3); 2.43 (qt, 2H, $^3J_{2-1}$ 11.22Hz, $^3J_{2-3}$ = 6.96Hz, 2)

**Composé 31 : 4-(2-bromoacétyl)-N-isopentylbenzènesulfonamide**

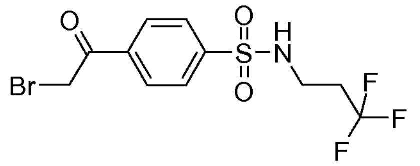

On ajoute goutte à goutte une solution de 303mg (1 eq.) de 4-acétyl-N-(3,3,3-trifluoropropyl)benzènesulfonamide **(30)** dans 35mL d'acide acétique glacial à une solution de 386mg (1 eq.) de tribromure de triméthylphénylammonium dans 30mL d'acide acétique glacial. Le mélange réactionnel est agité pendant 4h à TA. 0.1 eq. de réactif de bromation sont ajoutés pour consommer totalement la cétone non bromée résiduelle. L'acide acétique est évaporé sous pression réduite, le solide jaune obtenu est repris dans l'acétate d'éthyle puis lavé à l'eau (2x) et à la saumure (1x). La phase organique est séchée sur sulfate de magnésium puis évaporée sous pression réduite pour donner 358mg (93%) de solide beige.

LC-MS : $t_R$ = 5.85min (méthode a) ; m/z : $[M+H]^+$ = 372

**Composé 32: 4-(2-(cyanométhyl)thiazol-4-yl)-N-(3,3,3-trifluoropropyl)benzènesulfonamide**

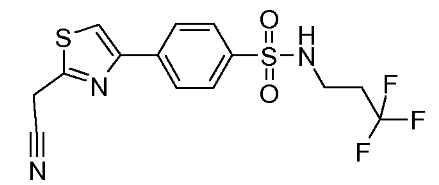

105.4mg (1.1eq.) de 2-cyanothioacétamide sont ajoutés à une solution de 358mg (1eq.) de 4-(2-bromoacétyl)-N-isopentylbenzènesulfonamide **(31)** dans 25mL de THF sur tamis moléculaire. Le mélange réactionnel est laissé au reflux du THF pendant 24h. 0.1eq supplémentaire de 2-cyanothioacétamide sont ajoutés, le milieu est agité 1h supplémentaire à 70°C. Le THF est ensuite évaporé sous pression réduite puis le solide obtenu est repris dans l'acétate d'éthyle et lavé à la saumure (2x). La phase organique est séchée sur sulfate de magnésium puis évaporée sous pression réduite. L'huile vert-olive obtenue est agitée 1 h à TA dans du méthanol en présence de $MgSO_4$ et de charbon actif. Le milieu est filtré pour donner un filtrat jaune trouble. Le méthanol est évaporé sous pression réduite. Le solide jaune est repris dans l'éthanol puis filtré de nouveau pour donner 270mg (75%) de solide jaune utilisé tel quel dans les étapes suivantes.

LC-MS : $t_R$ = 5.35min (méthode a) ; m/z : $[M+H]^+$ = 376

**Composé 33 : 2-(4-(4-(N-(3,3,3-trifluoropropyl)sulfamoyl)phényl)thiazol-2-yl)acétate de méthyle**

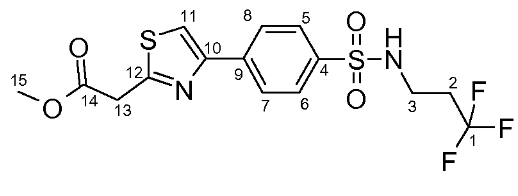

1.05mL (36eq.) de méthanol anhydre, 1.60mL (18eq.) de TMSCl et 270mg (1 eq.) de 4-(2-(cyanométhyl)thiazol-4-yl)-N-(3,3,3-trifluoropropyl)benzènesulfonamide (32) sont successivement ajoutés à TA dans un ballon sec et sous Argon. Le mélange est ensuite agité à 50°C pendant 15h puis laissé revenir à TA. 1 mL d'eau est ensuite ajouté et le pH est neutralisé à l'aide d'une solution aqueuse saturée en $NaHCO_3$. Le mélange est agité 10min à TA puis extrait à l'acétate d'éthyle. Les phases organiques sont lavées à la saumure (2x), séchées sur sulfate de magnésium et évaporées. Le solide orange obtenu est purifié sur colonne de silice prépaquée (EtOAc 1:9 Ether de pétrole → EtOAc 3:7 Ether de pétrole) pour donner 137mg (47%) de poudre beige pâle.

LC-MS : $t_R$ = 6.22min (méthode b) ; m/z : $[M+H]^+$ = 409
RMN $^1H$ ($CDCl_3$, 300MHz) : δ ppm = 8.06 (d, 2H, $^3J$ = 8.40Hz, 7+8); 7.91 (d, 2H, $^3J$ = 8.40Hz, 5+6); 7.64 (s, 11); 4.81 (t, 1H, $^3J$ = 6.50Hz, NH); 4.17 (s, 2H, 13); 3.81 (s, 3H, 15); 3.25 (q, 2H, $^3J$ = 6.70Hz, 3); 2.37 (qt, 2H, $^3J_{2-1}$ = 10.50Hz, $^3J_{2-3}$ = 6.80Hz, 2)
RMN $^{13}C$ ($CDCl_3$, 75MHz) : δ ppm = 169.4 (14); 162.3 (12); 153.0 (10); 138.7 (4 ou 9); 138.5 (4 ou 9); 127.6 (5+6); 127.0 (7+8); 125.9 (q, $^1J_{C-F}$ = 276.7Hz, 1); 116.6 (11); 52.7 (15); 38.6 (13); 36.7 (3); 34.5 (q, $^2J_{C-F}$ = 29.0Hz, 2).

**Composé 34 : 2-(4-(4-(N-(3,3,3-trifluoropropyl)sulfamoyl)phényl)thiazol-2-yl)acétate d'éthyle**

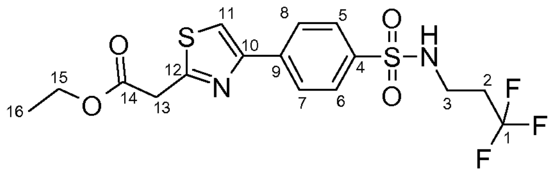

186μL (12eq.) d'éthanol absolu, 195μL de TMSCl (6eq.) et 100mg (1eq.) de 4-(2-(cyanométhyl)thiazol-4-yl)-N-(3,3,3-trifluoropropyl)benzènesulfonamide **(32)** sont successivement ajoutés à TA dans un ballon sec et sous Argon. Le mélange est ensuite agité à 30°C pendant 48h. On convertit ainsi le nitrile à 67% en ester désiré et 33% en acide carboxylique (anaylse HPLC, 215nm). 99μL (3eq.) de $SOCl_2$ sont alors ajoutés dans 466μL (30eq.) d'éthanol absolu. Le milieu réactionnel est agité à 40°C pendant 48h puis laissé revenir à TA. 1mL d'eau est ensuite ajouté et le pH est neutralisé à l'aide d'une solution aqueuse saturée en $NaHCO_3$. Le mélange est agité 10min à TA puis extrait à l'acétate d'éthyle. Les phases organiques sont lavées à la saumure, séchées sur sulfate de magnésium et évaporées. Le solide orange obtenu est purifié sur colonne de silice prépaquée (AcOEt 1:9 Ether de pétrole → AcOEt 3:7 Ether de pétrole) pour donner 66mg (59%) de poudre beige pâle.

LC-MS : $t_R$ = 6.22min (méthode b) ; m/z : $[M+H]^+$ = 423
RMN $^1H$ ($CDCl_3$, 300MHz) : δ ppm = 8.04 (d, 2H, $^3J$ = 8.70Hz, 7+8); 7.90 (d, 2H, $^3J$ = 8.70Hz, 5+6); 7.63 (s, 1 H, 11); 5.11 (t, 1H, $^3J$ = 6.50 Hz, N**H**); 4.27 (q, 2H, $^3J$ = 7.20Hz, 3); 4.14 (s, 2H, 13); 3.24 (q, 2H, $^3J$ = 6.80Hz, 15); 2.36 (qt, 2H, $^3J_{2-1}$ = 10.60Hz, $^3J_{2-3}$ = 6.90Hz, 2); 1.33 (t, 3H, $^3J$ = 7.10Hz, 16)
RMN $^{13}C$ ($CDCl_3$, 75MHz) :δ ppm = 169.0 (14); 162.6 (12); 153.0 (10); 138.8 (4); 128.6 (9); 127.7 (5+6); 127.1 (7+8); 126 (q, $^1J_{C-F}$ = 276Hz, 1); 116.7 (11); 61.9 (15); 39.0 (13); 36.7 (3); 34.6 (q, $^2J_{C-F}$ = 28.3Hz, 2); 14.2 (16)

**Composé 35 : 2-(4-(4-(N-(3,3,3-trifluoropropyl)sulfamoyl)phényl)thiazol-2-yl)acétate d'isopropyle**

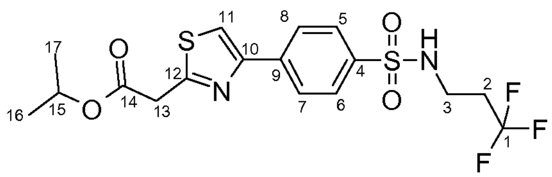

244μL (12eq.) d'isopropanol anhydre, 195μL (6eq.) de TMSCl et 100mg (1 eq.) de 4-(2-(cyanométhyl)thiazol-4-yl)-N-(3,3,3-trifluoropropyl)benzènesulfonamide (32) sont successivement ajoutés à TA dans un ballon sec et sous Argon. Le mélange est ensuite agité à 30°C pendant 48h. 197μL (6eq.) de $SOCl_2$ sont alors ajoutés dans 466μL (30eq.) d'isopropanol anhydre. Le milieu réactionnel est agité à 40°C pendant 48h puis laissé revenir à TA. 1mL d'eau est ensuite ajouté et le pH est neutralisé à l'aide d'une solution aqueuse saturée en $NaHCO_3$. Le mélange est agité 10min à TA puis extrait à l'acétate d'éthyle. Les phases organiques sont lavées à la saumure (2x), séchées sur sulfate de magnésium et évaporées. Le solide orange obtenu est purifié sur colonne de silice prépaquée (AcOEt 1:9 Ether de pétroles → AcOEt 3:7 Ether de pétrole). Le produit est purifié une deuxième fois sur HPLC pour donner 29mg (25%) de poudre beige pâle.

LC-MS : $t_R$ = 6.50min (méthode b) ; m/z : $[M+H]^+$ = 437
RMN $^1H$ ($CDCl_3$, 300MHz) : δ ppm = 8.04 (d, 2H, $^3J$ = 8.70Hz, 7+8); 7.90 (d, 2H, $^3J$ = 8.70Hz, 5+6); 7.63 (s, 1H,

11); 5.06-5.18 (m, 15+N**H**); 4.11 (s, 2H, 13); 3.23 (q, 2H, $^3J$ = 6.8 Hz, 3); 2.36 (tq, 2H, $^3J_{2-1}$ = 10.60Hz, $^3J_{2-3}$ = 6.90Hz, 2); 1.30 (d, 6H, $^3J$ = 6.30Hz, 16+17)

RMN $^{13}C$ ($CDCl_3$, 75MHz) : δ ppm = 168.6 (14); 162.8 (12); 152.9 (10); 138.8 (4 ou 9); 138.5 (4 ou 9); 127.7 (5+6); 127.1 (7+8); 125.9 (q, $^1J_{C-F}$ = 277.5Hz, 1); 116.7 (11); 69.7 (15); 39.4 (13); 36.7 (3); 34.5 (q, $^2J_{C-F}$ = 28.3Hz, 2); 21.8 (16+17)

## Scheme 5:

33

36

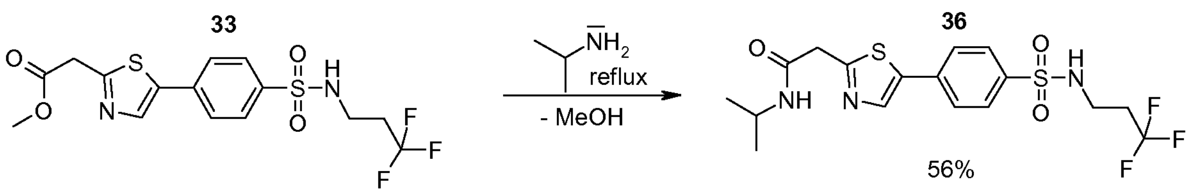


**Composé 36 : N-isopropyl-2-(4-(4-(N-(3,3,3-trifluoropropyl)sulfamoyl)phényl)thiazol-2-yl)acétamide**

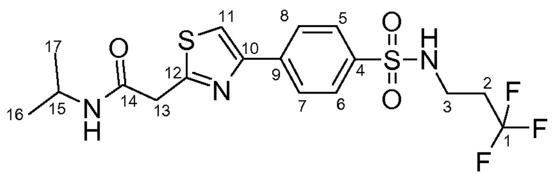

54mg de 2-(4-(4-(N-(3,3,3-trifluoropropyl)sulfamoyl)phényl)thiazol-2-yl)acétate de méthyle (33) et 1 mL d'isopropylamine sont introduits dans un ballon de 5mL. Le mélange est chauffé au reflux de l'isopropylamine pendant 5h. 0.5mL supplémentaires d'ispropylamine sont ajoutés et le mélange est chauffé au reflux 4h supplémentaires. L'isopropylamine est alors évaporée sous pression réduite. Le produit est repris dans l'acétate d'éthyle et lavé à la saumure (2x). La phase organique est séchée sur sulfate de magnésium puis évaporée sous pression réduite. Le solide orange obtenu est purifié sur colonne de silice prépaquée (DCM 98 : 2 MeOH) pour donner 32mg (56%) de poudre beige.

LC-MS : $t_R$ = 5.57min (méthode b) ; m/z : $[M+H]^+$ = 436

RMN $^1H$ (DMSO-$d_6$, 300MHz) : δ ppm = 8.22 (s, 1 H, 11); 8.14 (d, 2H, $^3J$ = 8.51 Hz, 7+8); 7.89 (d, 2H, $^3J$ = 8.51 Hz, 5+6); 4.07 (s, 2H, 13); 3.92-3.79 (m, 1 H, 15); 2.99 (t, 2H, $^3J$ = 6.91 Hz, 3); 2.43 (qt, 2H, $^3J_{2-1}$ = 11.21 Hz, $^3J_{2-3}$ = 6.96Hz, 2); 1.08 (d, 6H, $^3J$ = 6.58Hz)

## Scheme 6:

37

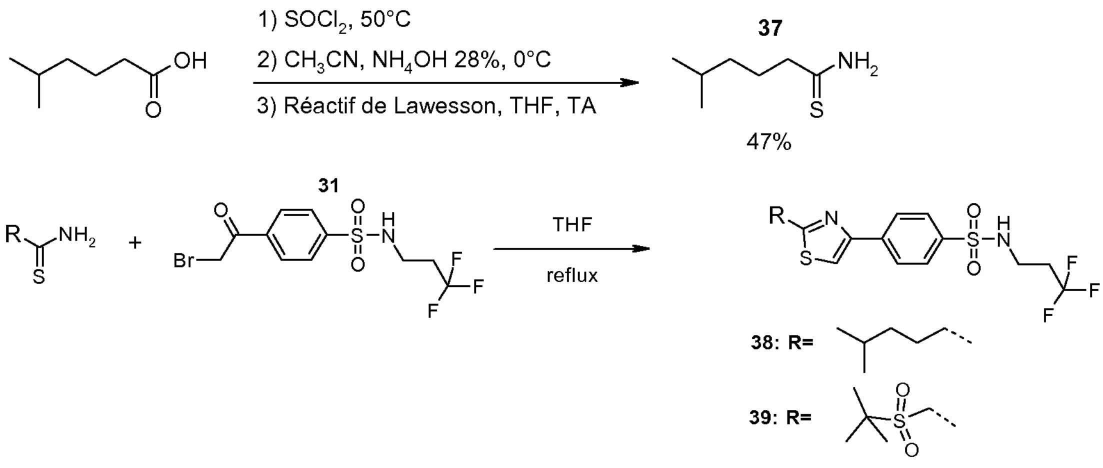


31

38: R=

39: R=

**Composé 37 : 5-méthylhexanethioamide**

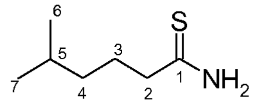

2mL d'acide 5-méthylhexanoïque et 5mL de chlorure de thionyle sont introduits dans un ballon de 25mL. Le mélange est chauffé à 50°C pendant 1h puis le chlorure de thionyle est évaporé sous pression réduite. Un mélange $CH_3CN$ (5mL) : (10mL) $NH_4OH$ (28%) est ensuite ajouté à 0°C. La solution est alors agitée pendant 15min à 0°C. Le produit est extrait à l'acétate d'éthyle. Les phases organiques sont rassemblées, séchées sur sulfate de magnésium puis évaporées sous pression réduite pour donner une poudre blanche.

2,9g (0.5eq.) de réactif de Lawesson et 50mL de THF anhydre sont alors ajoutés au produit. La solution est agitée à TA pendant 4h. Le THF est évaporé sous pression réduite et le produit est purifié sur colonne de silice prépaquée (Cyclohexane 85:15 AcOEt → Cyclohexane 80:20 AcOEt) pour donner 950mg (47%) d'huile transparente qui cristallise ensuite.

<u>LC-MS :</u> $t_R$ = 3.03min (méthode c) ; m/z : $[M+H]^+$ = 146
<u>RMN 1 H ($CDCl_3$, 300MHz) :</u> δ ppm = 8.35+7.46 (N**H**$_2$); 2.58 (t, 2H, $^3J$ = 7.60Hz, 2); 1.64-1.74 (m, 2H, 3 ou 4); 1.50 (sp, 1H, $^3J$ = 6.70Hz, 5); 1.12-1.21 (m, 2H, 3 ou 4); 0.82 (d, 6H, $^3J$ = 6.60Hz, 6+7)

**Composé 38 : 4-(2-(4-méthylpentyl)thiazol-4-yl)-N-(3,3,3-trifluoropropyl)benzènesulfonamide**

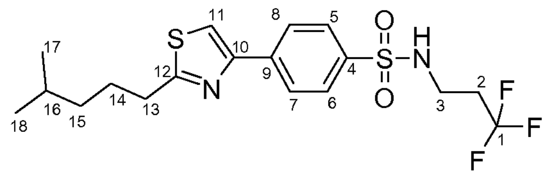

232mg (1 eq.) de 4-(2-bromoacétyl)-N-isopentylbenzènesulfonamide (31) sont ajoutés à une solution de 90mg (1eq.) de 5-méthylhexanethioamide (37) dans 20mL de THF anhydre. Le milieu réactionnel est chauffé au reflux pendant 3h. Le THF est ensuite évaporé sous pression réduite. Le produit est repris dans l'acétate d'éthyle, lavé à l'eau (2x) puis à la saumure (1x). La phase organique est séchée sur sulfate de magnésium puis évaporée sous pression réduite. L'huile jaune obtenue est purifiée sur colonne de silice prépaquée (Cyclohexane 9:1 AcOEt → Cyclohexane 8:2 AcOEt) pour donner 60mg (24%) de poudre beige.

<u>LC-MS :</u> $t_R$ = 7.60min (méthode d) ; m/z : $[M+H]^+$ = 421
<u>RMN $^1H$ ($CDCl_3$, 300MHz) :</u> δ ppm = 8.07 (d, 2H, $^3J$ = 8.60Hz, 5+6); 7.91 (d, 2H, $^3J$ = 8.60Hz, 7+8); 7.51 (s,1 H, 11); 4.89 (t, 1H, $^3J$ = 6.50Hz, N**H**); 3.26 (q, 2H, $^3J$ = 6.70Hz, 3); 3.06 (t, 2H, $^3J$ = 7.70Hz, 13); 2.37 (qt, 2H, $^3J_{2-1}$ = 10.70Hz $^3J_{2-3}$ = 6.70Hz, 2); 1.85 (q, 2H, $^3J$ = 7.80Hz, 14); 1.63 (n, 1H, $^3J$ = 6.70Hz, 16); 1.30-1.38 (m, 4H, 15+graisse); 0.92 (d, 6H, $^3J$ = 6.6Hz, 17+18)
<u>RMN $^{13}C$ ($CDCl_3$, 75MHz) :</u> δ ppm = 172.6 (12); 153.0 (10); 139.2 (9); 138.3 (4); 127.7 (7+8); 127.2 (5+6); 120.5-131.5 (q, $^1J_{C-F}$ = 277.2Hz, 1); 114.9 (11); 38.5 (15); 36.8 (3); 34.6 (q, $^2J_{C-F}$ = 28.4Hz, 2); 34.0 (13); 28.1 (14); 28.0 (16); 22.7 (17+18)

**Composé 39 : 4-(2-(tert-butylsulfonylméthyl)thiazol-4-yl)-N-(3,3,3-trifluoropropyl)-benzènesulfonamide**

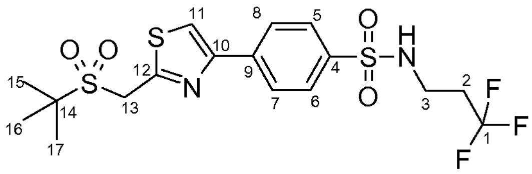

150mg (1 eq.) de 4-(2-bromoacétyl)-N-isopentylbenzènesulfonamide (31) sont ajoutés à une solution de 78.3mg (1eq.) de 2-(tert-butylsulfonyl)éthanethioamide dans 7mL de THF anhydre. Le milieu réactionnel est chauffé au reflux pendant 72h. Le THF est ensuite évaporé sous pression réduite. Le produit est repris dans l'acétate d'éthyle, lavé à l'eau puis à la saumure. La phase organique est séchée sur sulfate de magnésium puis évaporée sous pression réduite. Le produit obtenu est purifié par HPLC pour donner 119mg (63%) de poudre beige.

LC-MS : $t_R$ = 5.67min (méthode d) ; m/z : $[M+H]^+$ = 471
RMN $^1H$ (DMSO-$d_6$, 300MHz) : δ ppm = 8.44 (s, 1 H, 11); 8.19 (d, 2H, $^3J$ = 8.70Hz, 7+8); 7.94 (t, 1H, $^3J$ = 6.0Hz, N**H**); 7.90 (d, 2H, $^3J$ = 8.70Hz, 5+6); 5.09 (s, 2H, 13); 3.00 (q, 2H, $^3J$ = 6.60Hz, 3); 2.44 (qt, 2H, $^3J_{2-1}$ = 11.2Hz, $^3J_{2-3}$ = 7.0Hz, 2); 1.38 (s, 9H, 15+16+17) RMN $^{13}C$ (DMSO-$d_6$, 75Hz : δ ppm = 157.1 (12); 152.5 (10); 139.0 (4); 137.4 (9); 127.2 (5+6); 126.6 (7+8); 126.4 (q, $^1J_{C-F}$ = 277.0Hz, 1); 120.0 (11); 60.2 (14); 50.3 (13); 36.03(q, $^3J_{C-F}$ = 3.70Hz, 3); 66.3 (q, $^2J_{C-F}$ = 27.2Hz, 2); 22.9 (15+16+17)

## Scheme 7:

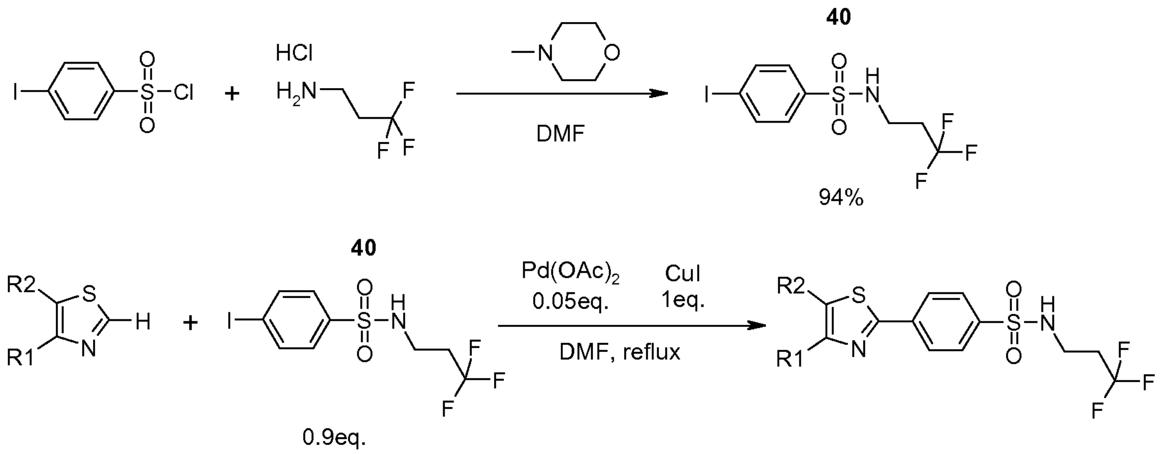


| | | |
|---|---|---|
| **41** | R1 = Me<br>R2 = H | 53% |
| **42** | R1R2 = $-C_4H_4-$ | 20% |

**Composé 40 : 4-iodo-N-(3,3,3-trifluoropropyl)benzènesulfonamide**

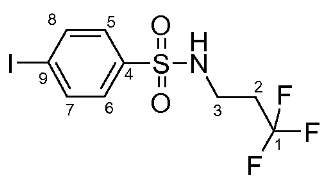

1.82mL (5eq.) de N-méthylmorpholine sont ajoutés à une solution de 593.2mg (1.2eq.) de chlorhydrate de 3,3,3-trifluoropropylamine dans 50mL de DMF. Le mélange est agité 5min à TA puis 1g (1eq.) de chlorure d'acide 4-iodobenzènesulfonique est ajouté. Le milieu réactionnel est agité à TA pendant 2h. Le DMF est évaporé sous pression réduite puis le produit est repris dans l'acétate d'éthyle, lavé à l'eau (2x) et à la saumure (1x). La phase organique est séchée sur sulfate de magnésium puis évaporée sous pression réduite pour donner 1.17g (94%) de solide jaune pâle.

LC-MS : $t_R$ = 6.22min (méthode b) ; m/z : $[M-H]^-$ = 378
RMN $^1H$ ($CD_2Cl_2$, 300MHz) : δ ppm = 7.96 (d, 2H, $^3J$ = 8.70Hz, 5+6 ou 7+8); 7.60 (d, 2H, $^3J$ = 8.70Hz, 5+6 ou 7+8); 4.81 (m, 1 H, NH); 3.25 (q, 2H, $^3J$ = 6.70Hz, 3); 2.43 (qt, 2H, $^3J_{2-1}$ = 10.60Hz, $^3J_{2-3}$ = 6.80Hz, 2)

**Composé 41: 4-(4-méthylthiazol-2-yl)-N-(3,3,3-trifluoropropyl)benzènesulfonamide**

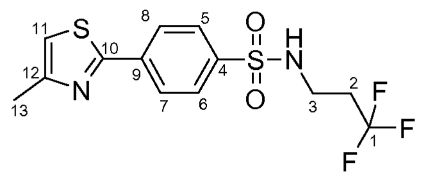

40.0μL (1 eq.) de 4-méthylthiazole, 4.9mg (0.05eq.) d'acétate de palladium, 83.7mg (1 eq.) d'iodure de cuivre et 150mg (0.9eq.) de 4-iodo-N-(3,3,3-trifluoropropyl)-benzènesulfonamide **(40)** sont placés successivement dans un schlenk sous un flux d'Argon. 1.5mL de DMF anhydre sont ajoutés *via* une canule sous Argon à température ambiante. Le schlenk est tiré trois fois sous vide et rempli d'Argon successivement. Le mélange réactionnel est agité à 140°C sous Argon pendant 48h. Le milieu réactionnel est dilué à TA par 2mL d'un mélange d'acétate d'éthyle et d'une solution aqueuse saturée en $NH_4Cl$ (1:1 en volume). Le milieu est alors filtré et le solide lavé à l'acétate d'éthyle. La phase organique est lavée à la saumure (2x), séchée sur sulfate de magnésium, filtrée sur célite puis évaporée sous pression réduite. Le produit est alors purifié sur colonne de silice prépaquée (AcOEt 1:9 Ether de Pétroles → AcOEt 2:8 Ether de Pétrole) pour donner 73mg (53%) de poudre blanche.

LC-MS : $t_R$ = 6.07min (méthode b) ; m/z : $[M+H]^+$ = 351
RMN $^1H$ ($CD_2Cl_2$, 300MHz) : δ ppm = 8.11 (d, 2H, $^3J$ = 8.70Hz, 7+8); 7.91 (d, 2H, $^3J$ = 8.70Hz, 5+6); 7.04 (s, 1 H, 11); 4.83 (m, 1 H, N**H**); 3.26 (q, $^3J$ = 6.70Hz, 3); 2.51 (s, 3H, 13); 2.39 (tq, 2H, $^3J_{2-1}$ = 10.60Hz, $^3J_{2-3}$ = 6.80Hz, 2)
RMN $^{13}C$ ($CD_2Cl_2$, 75MHz) : δ ppm = 164.8 (10); 154.4 (12); 141.0 (4); 137.0 (9); 128.0 (5+6); 127.2 (7+8); 126.85 (q, $^1J_{C-F}$ = 277.1 Hz, 1); 116.9 (11); 36.4 (q, $^3J_{C-F}$ = 3.8Hz, 3); 33.6 (q , $^2J_{C-F}$ = 27.4Hz, 2); 17.3 (13)

**Composé 42 : 4-(benzo[d]thiazol-2-yl)-N-(3,3,3-trifluoropropyl)benzènesulfonamide**

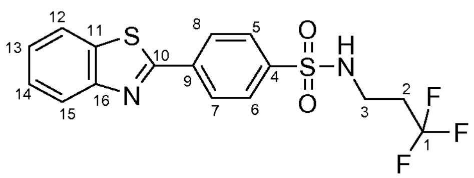

48.0μL (1 eq.) de 4-méthylthiazole, 4.9mg (0.05eq.) d'acétate de palladium, 83.7mg (1 eq.) d'iodure de cuivre et 150mg (0.9eq.) de 4-iodo-N-(3,3,3-trifluoropropyl)-benzènesulfonamide **(40)** sont placés successivement dans un schlenk sous un flux d'Argon. 1.5mL de DMF anhydre sont ajoutés *via* une canule sous Argon à température ambiante. Le schlenk est tiré trois fois sous vide et rempli d'Argon successivement. Le mélange réactionnel est agité à 140°C sous Argon pendant 48h. Le milieu réactionnel est dilué à TA par 2mL d'un mélange d'acétate d'éthyle et d'une solution aqueuse saturée en $NH_4Cl$ (1:1 en volume). Le milieu est alors filtré et le solide lavé à l'acétate d'éthyle. La phase organique est lavée à la saumure (2x), séchée sur sulfate de magnésium, filtrée sur célite puis évaporée sous pression réduite Le produit est alors purifié sur colonne de silice prépaquée (AcOEt 1:9 Ether de Pétroles → AcOEt 2:8 Ether de Pétrole). Le produit est ensuite recristallisé dans 2mL d'éthanol pour donner 31 mg (20%) de cristaux transparents.

LC-MS : $t_R$ = 6.73min (méthode b) ; m/z : $[M+H]^+$ = 387
RMN $^1H$ (DMSO-$d_6$, 300MHz): δ ppm = 8.34 (d, 2H, $^3J$ = 8.50Hz, 7+8); 8.21-8.23 (m ; 1 H, 12 ou 15); 8.09-8.14 (m, 2H, N**H** + 12 ou 15); 8.00 (d, 2H, $^3J$ = 8.50Hz, 5+6); 7.60 (td, 1H, $^3J$ = 7.40Hz, $^4J$ = 1.20Hz, 13 ou 14); 7.53 (td, 1H, $^3J$ = 7.40Hz, $^4J$ = 1.20Hz, 13 ou 14); 3.04 (t, 2H, $^3J$ = 6.90Hz, 3); 2.39 (qt, 2H, $^3J_{2-1}$ = 10.60Hz, $^3J_{2-3}$ = 6.80Hz, 2)
RMN $^{13}C$ ($CD_2Cl_2$, 75MHz) : δ ppm = 166.0 (10); 154.0 (16); 142.4 (9); 136.7 (16); 135.3 (11); 128.5 (5+6); 128.1 (7+8); 127.5 (13 ou 14); 126.6 (13 ou 14); 126.9 (q, $^1J_{C-F}$ = 273.4 Hz, 1); 123.8 (15 ou 12); 123.1 (15 ou 12); 36.5

(3); 33.7 (q, $^{2}J_{C-F}$ = 27.5Hz, 2)

**Scheme 8:**

43

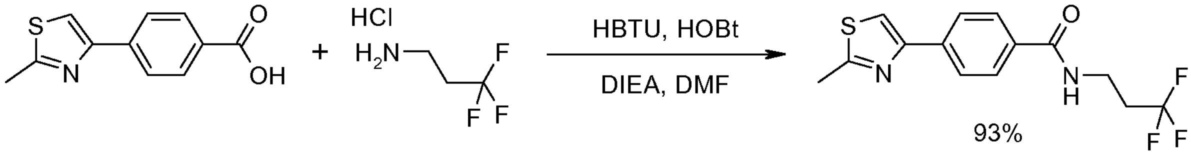


**Composé 43 : 4-(2-méthylthiazol-4-yl)-N-(3,3,3-trifluoropropyl)benzamide**

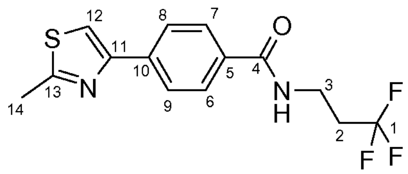

50mg (1 eq.) d'acide 4-(2-méthylthiazol-4-yl)benzoïque, 37mg (1.1 eq.) de chlorhydrate de 3,3,3-trifluoropropylamine (1.1eq), 103mg (1.2eq.) d'HBTU, 52mg (1.5eq.) d'HOBt, 118µL (3eq.) de DIEA et 2.5mL de DMF sont introduits dans cet ordre là dans un ballon de 10mL. La solution est agitée pendant 2h à TA. Le DMF est évaporé sous pression réduite. Le produit est repris dans l'acétate d'éthyle puis lavé successivement à l'aide d'une solution aqueuse saturée en $K_2CO_3$ (2x), une solution aqueuse d'HCl 1N (2x), d'eau (1x) et de saumure (1x). La phase organique est séchée sur sulfate de magnésium puis évaporée sous pression réduite pour donner 67mg (93%) de poudre beige.

LC-MS : $t_R$ = 3.10min (méthode c) ; m/z : $[M+H]^+$ = 315
RMN $^{1}H$ ($CD_3OD$, 300MHz) : δ ppm = 7.99 (d, 2H, $^{3}J$ = 8.50Hz, 8+9); 7.87 (d, 2H, $^{3}J$ = 8.50Hz, 6+7); 7.76 (s, 1 H, 12); 3.64 (t, 2H, $^{3}J$ = 7.00Hz, 3); 2.76 (s, 3H, 14); 2.54 (qt, 2H, $^{3}J_{2-1}$ = 10.60Hz, $^{3}J_{2-3}$ = 6.80Hz, 2)
RMN $^{13}C$ ($CD_3OD$, 75MHz) : δ ppm = 168.4 (4); 167.1 (13); 153.6 (11); 137.4 (10); 133.1 (5); 127.4 (6+7); 126.7 (q, $^{1}J_{C-F}$ = 277.8Hz, 1); 125.9 (8+9); 114.7 (12); 33.1 (3); 32.7 (q, $^{2}J_{C-F}$ = 27.5Hz, 2); 17.5 (14)

## Scheme 9 :

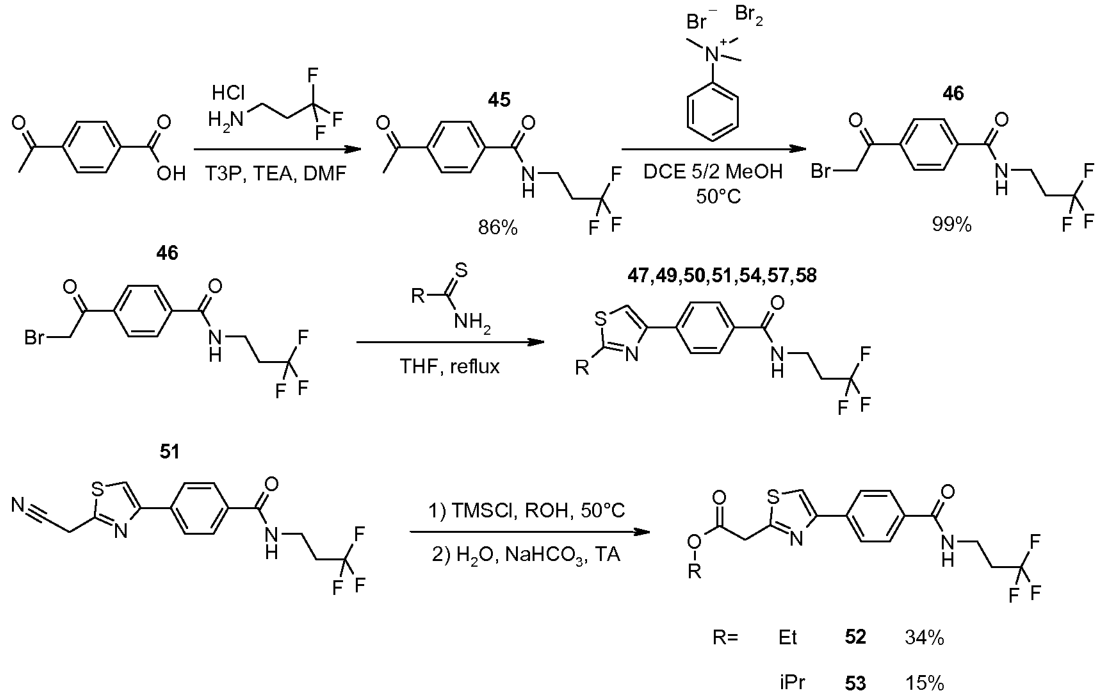


**Composé 45 : 4-acétyl-N-(3,3,3-trifluoropropyl)benzamide**

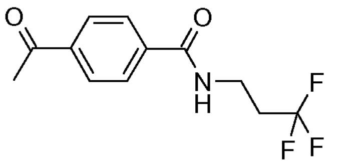

546.6mg (1 eq.) de chlorhydrate de 3,3,3-trifluoropropylamine sont ajoutés à une solution de 600mg (1eq.) d'acide 4-acétylbenzoïque, 1.66g (1.2eq.) d'HBTU, 98mg (0.2eq.) d'HOBt, et 2.53mL (4eq.) de DIEA dans 15mL de DMF. Le milieu réactionnel est alors agité pendant 2h à TA. Le DMF est ensuite évaporé sous pression réduite. L'huile orange obtenue est reprise dans l'acétate d'éthyle puis lavée à l'aide d'une solution aqueuse d'HCl 1N (2x), une solution aqueuse saturée en $Na_2CO_3$ (2x) puis à la saumure (1x). La phase organique est séchée sur sulfate de magnésium puis évaporée sous pression réduite. Le produit obtenu est purifié sur colonne de silice prépaquée (Cyclohexane 85:15 AcOEt→ 80:20 → 70: 30) pour donner 837 mg (88%) de poudre blanche.

LC-MS : $t_R$ = 2.70min (méthode c) ; m/z : $[M+H]^+$ = 260

**Composé 46 : 4-(2-bromoacétyl)-N-(3,3,3-trifluoropropyl)benzamide**

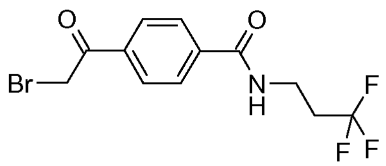

436.1 mg (1eq.) de tribromure de triméthylphénylammonium sont ajoutés à une solution de 300mg (1 eq.) de

4-acétyl-N-(3,3,3-trifluoropropyl)benzamide **(45)** dans un mélange de 15mL de DCE et 6mL de méthanol. La solution est agitée à 50°C. 43.6mg (0.1 eq) de réactif de bromation sont ajoutés après 1 h et 3h de réaction. Le DCE et le méthanol sont évaporés sous pression réduite. Le produit est repris dans l'acétate d'éthyle puis lavé à l'eau (2x) et à la saumure (1x). La phase organique est séchée sur sulfate de magnésium puis évaporée sous pression réduite pour donner 387mg (99%) de poudre beige.

LC-MS : $t_R$ = 2.93min (méthode c) ; m/z : $[M+H]^+$ = 340

**Composé 47 : 4-(2-(4-méthylpentyl)thiazol-4-yl)-N-(3,3,3-trifluoropropyl)benzamide**

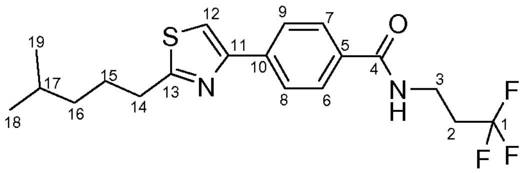

100m (1 eq.) de 4-(2-bromoacétyl)-N-(3,3,3-trifluoropropyl)benzamide **(46)** sont ajoutés à une solution de 43mg (1eq.) de 5-méthylhexanethioamide **(37)** dans 10mL de THF anhydre. Le milieu réactionnel est chauffé au reflux pendant 3h. Le THF est ensuite évaporé sous pression réduite. Le produit est repris dans l'acétate d'éthyle, lavé à l'eau (2x) puis à la saumure (1x). La phase organique est séchée sur sulfate de magnésium puis évaporée sous pression réduite. Le solide beige obtenu est purifié sur colonne de silice prépaquée (Cyclohexane 9:1 AcOEt) pour donner 25mg (22%) de poudre beige.

LC-MS : $t_R$ = 3.77min (méthode c) ; m/z : $[M+H]^+$ = 385
RMN $^1$H ($CD_2Cl_2$, 300MHz) : δ ppm = 7.99 (d, 2H, $^3$J=8.66Hz, 8+9); 7.84 (d, 2H, $^3$J=8.66Hz, 6+7); 7.51 (s, 1 H, 12); 6.83 (t, 1H, $^3$J=5.70Hz, N**H**); 3.73 (q, 2H, $^3$J=6.50Hz, 3); 3.06 (t, 2H, $^3$J=7.77Hz, 14); 2.53 (qt, 2H, $^3J_{2-1}$=10.92Hz $^3J_{2-3}$=6.78Hz, 2); 1.87 (qn, 2H, $^3$J=7.85Hz, 15); 1.66 (n, 1H, $^3$J=6.65Hz, 17); 1.36 (q, 2H, $^3$J=7.51Hz, 16); 0.95; (d, 6H, $^3$J=6.63Hz, 18+19)

**Composé 48: 3-méthylbutanethioamide**

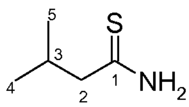

1.01g (0.5eq.) de réactif de Lawesson et 16mL de THF anhydre sont ajoutés à 505.8mg (1 eq.) d'isovaléramide. La solution est agitée à TA pendant 4h puis le THF est évaporé sous pression réduite. Le produit est repris dans l'éther diisopropylique puis filtré. Le filtrat est ensuite purifié sur colonne de silice prépaquée (DCM 100%) pour donner 396mg (70%) d'huile transparente qui cristallise ensuite.

LC-MS : $t_R$ = 1.92min (méthode c) ; m/z : $[M+H]^+$ = 118
RMN $^1$H ($CDCl_3$, 300MHz) : δ ppm = 7.65 (br s, 1 H, N**H**); 6.85 (br s, 1 H, N**H**); 2.52 (d, 2H, $^3$J = 7.28Hz, 2); 2.25 (n, 1H, $^3$J = 6.95Hz, 3); 1.00 (d, 6H, $^3$J = 6.59Hz, 4+5)

**Composé 49 : 4-(2-isobutylthiazol-4-yl)-N-(3,3,3-trifluoropropyl)benzamide**

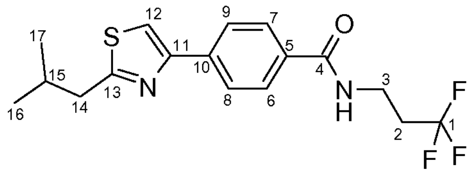

100m (1 eq.) de 4-(2-bromoacétyl)-N-(3,3,3-trifluoropropyl)benzamide **(46)** sont ajoutés à une solution de 34.7mg (1eq.) de 3-méthylbutanethioamide **(48)** dans 6mL de THF anhydre. Le milieu réactionnel est chauffé au reflux pendant une nuit. 3.5mg (0.1 eq) de thioamide sont ajoutés et la solution est agitée 1h supplémentaire au reflux. Le THF est ensuite évaporé sous pression réduite. Le produit est repris dans l'acétate d'éthyle, lavé à l'eau (2x) puis à la saumure (1x). La phase organique est séchée sur sulfate de magnésium puis évaporée sous pression réduite. Le résidu est purifié par HPLC préparative pour donner 78mg (74%) de poudre beige.

<u>LC-MS :</u> $t_R$ = 3.17min (méthode c) ; m/z : $[M+H]^+$ = 357
<u>RMN</u> $^1H$ ($CD_2Cl_2$, 300MHz) : δ ppm = 8.01 (d, 2H, $^3J$ = 8.60Hz, 8+9); 7.82 (d, 2H, $^3J$ = 8.60Hz, 6+7); 7.53 (s, 1 H, 12); 6.47 (br s, 1 H, N**H**); 3.74 (t, 2H, $^3J$ = 6.50Hz, 3); 2.95 (d, 2H, $^3J$ = 6.70Hz, 14); 2.53 (qt, 2H, $^3J_{2-1}$ = 10.80Hz, $^3J_{2-3}$ = 6.50Hz, 2); 2.19 (n, 1H, $^3J$ = 6.70Hz, 15); 1.05 (d, 6H, $^3J$ = 6.60Hz, 16+17)
<u>RMN</u> $^{13}C$ ($CD_2Cl_2$, 75MHz) : δ ppm = 171.0 (13); 167.4 (4); 154.0 (11); 138.2 (10); 133.6 (5); 127.8 (6+7); 127.1 (q, $^1J_{C-F}$ = 277.8Hz, 1); 126.7 (8+9); 114.2 (12); 42.8 (14); 34.0 (q, $^2J_{C-F}$ = 27.5Hz, 2); 33.8 (3); 30.2 (15); 22.4 (16+17)

**Composé 50 :4-(2-(tert-butylsulfonylméthyl)thiazol-4-yl)-N-(3,3,3-trifluoropropyl)-benzamide**

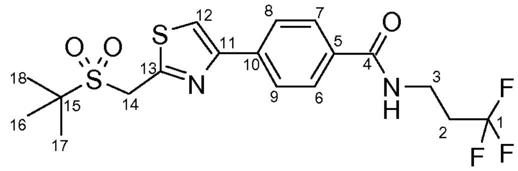

10mg (1 eq.) de 4-(2-bromoacétyl)-N-(3,3,3-trifluoropropyl)benzamide **(46)** sont ajoutés à une solution de 57.8mg (1eq.) de 2-(tert-butylsulfonyl)éthanethioamide dans 6mL de THF anhydre. Le milieu réactionnel est agité et chauffé au reflux pendant une nuit. 11.7mg (0.2eq.) supplémentaires de thioamide sont ajoutés et le mélange est agité 2h au reflux. Le THF est ensuite évaporé sous pression réduite. Le produit est repris dans l'acétate d'éthyle, lavé à l'eau (2x) puis à la saumure (1x). La phase organique est séchée puis évaporée sous pression réduite. Le résidu sur colonne de silice prépaquée (Cyclohexane 85 :15 Isopropanol → Cyclohexane 1:1 Isopropanol) pour donner 98mg (76%) de poudre blanche.

<u>LC-MS :</u> $t_R$ = 5.23min (méthode c) ; m/z : $[M+H]^+$ = 435
<u>RMN</u> $^1H$ (DMSO-$d_6$, 300MHz) : δ ppm = 8.76 (t, 1H, $^3J$ = 5.60Hz, N**H**); 8.37 (s, 1 H, 12); 8.08 (d, 2H, $^3J$ = 8.40Hz, 8+9); 7.93 (d, 2H, $^3J$ = 8.40Hz, 6+7); 5.09 (s, 2H, 14); 3.52 (q, 2H, $^3J$ = 6.50Hz, 3); 2.57 (qt, 2H, $^3J_{2-1}$ = 10.94Hz, $^3J_{2-3}$ = 6.62Hz, 2); 1.39 (s, 9H, 16+17+18)
<u>RMN</u> $^{13}C$ (DMSO-$d_6$, 75MHz : δ ppm = 166.3 (4); 157.2 (13); 153.7 (11); 136.8 (5); 133.9 (10); 128.3 (6+7); 127.5 (q, $^1J_{C-F}$ = 267Hz, 1); 126.3 (8+9); 119.3 (12); 60.6 (15); 50.8 (14); 33.3 (q, $^3J_{C-F}$ = 3.5Hz, 3); 32.9 (q, $^2J_{C-F}$ = 27.0Hz, 2); 23.4 (16+17+18)

**Composés 51 : 4-(2-(cyanométhyl)thiazol-4-yl)-N-(3,3,3-trifluoropropyl)benzamide**

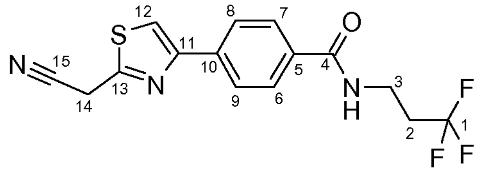

107mg (1.2eq.) de 2-cyanothioacétamide sont ajoutés à une solution de 300mg (1 eq.) de 4-(2-bromoacétyl)-N-(3,3,3-trifluoropropyl)benzamide **(46)** dans 25mL de THF. Le milieu est agité pendant 24h au reflux. Le THF est alors évaporé sous pression réduite. Le résidu est repris dans l'acétate d'éthyle puis lavé à la saumure (2x). La phase organique est séchée sur sulfate de magnésium puis évaporée sous pression réduite pour donner 302mg de solide orange. Le produit brut est utilisé ainsi dans la réaction suivante.

LC-MS : $t_R$ = 2.96min (méthode c) ; m/z : $[M-H]^-$ = 296

**Composé 52 :2-(4-(4-(3,3,3-trifluoropropylcarbamoyl)phényl)thiazol-2-yl)acétate d'éthyle**

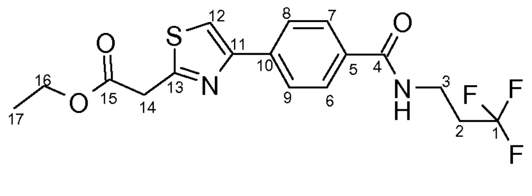

150mg (1 eq.) de 4-(2-bromoacétyl)-N-(3,3,3-trifluoropropyl)benzamide **(46)** sont ajoutés à une solution de 44.4mg (1eq.) de 2-cyanoéthanethioamide dans 10mL de THF. Le mélange est agité pendant 15h au reflux. Le THF est évaporé sous pression réduite et le résidu obtenu est utilisé tel quel. Sous Argon, 515µL (20eq.) d'éthanol absolu et 545µL (10eq.) de TMSCl sont successivement ajoutés à TA dans un ballon sec. Le mélange est ensuite agité à 40°C pendant 2h. 200µL de $SOCl_2$ sont alors ajoutés dans 1 mL d'éthanol absolu. Le milieu réactionnel est agité à 40°C pendant 2h puis laissé revenir à TA. 1 mL d'eau est ensuite ajouté et le pH est neutralisé à l'aide d'une solution aqueuse saturée en $NaHCO_3$. Le mélange est agité 15min à TA puis extrait à l'acétate d'éthyle. Les phases organiques sont lavées à la saumure (2x), séchées sur sulfate de magnésium et évaporées. Le solide orange obtenu est recristallisé dans 2mL d'éthanol absolu pour donner 58mg (34%) de cristaux marron clair.

LC-MS : $t_R$ = 2.77min (méthode c) ; m/z : $[M+H]^+$ = 387
RMN $^1H$ ($CD_2Cl_2$, 300MHz) : δ ppm = 8.01 (d, 2H, $^3J$ = 8.50Hz, 8+9); 7.83 (d, 2H, $^3J$ = 8.50Hz, 6+7); 7.65 (s, 1 H, 12); 6.47 (br s, 1 H, N**H**); 4.26 (q, $^3J$ = 7.10Hz, 16); 4.14 (s, 2H, 14); 3.73 (q, 2H, $^3J$ = 6.40Hz, 3); 2.53 (qt, 2H, $^3J_{2-1}$ = 10.9Hz, $^3J_{2-3}$ = 6.60Hz, 2); 1.32 (t, 3H, $^3J$ = 7.10Hz, 17)

**Composé 53 :2-(4-(4-(3,3,3-trifluoropropylcarbamoyl)phényl)thiazol-2-yl)acétate d'isopropyle**

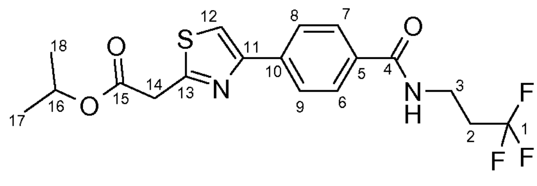

Sous argon, 3.23mL (48eq.) d'isopropanol, 2.66mL (24eq.) de TMSCl et 300mg (1 eq.) de 4-(2-(cyanométhyl)thiazol-4-yl)-N-(3,3,3-trifluoropropyl)benzamide (**51**) sont successivement ajoutés à TA dans un ballon sec. La solution est agitée pendant 10min à TA puis 16µL (1eq.) d'eau sont ajoutés. Le mélange est ensuite agité à 40°C pendant une nuit. Le pH est neutralisé à l'aide d'une solution aqueuse saturée en $NaHCO_3$. Le mélange est agité 15 min à TA puis extrait à l'acétate d'éthyle. Les phases organiques sont lavées à la saumure (2x), séchées sur sulfate de magnésium et évaporées. Le résidu obtenu est alors purifié sur colonne de silice prépaquée (AcOEt 3:7 Cyclohexane) pour donner 51 mg (15%) de solide blanc.

LC-MS : $t_R$ = 5.87min (méthode d) ; m/z : $[M+H]^+$ = 387
RMN $^1H$ ($CDCl_3$, 300MHz) : δ ppm = 7.99 (d, 2H, $^3J$ = 8.60Hz, 8+9); 7.83 (d, 2H, $^3J$ = 8.60Hz, 6+7); 7.58 (s, 1H, 12); 6.44 (m, 1H, N**H**); 5.14 (sp, 1H, $^3J$ = 6.30Hz, 16); 4.13(s, 2H, 14); 3.77 (q, 2H, $^3J$ = 6.30Hz, 3); 2.52 (tq, 2H, $^3J_{2-1}$ = 10.8Hz, $^3J_{2-3}$ = 6.30Hz, 2); 1.32 (d, 6H, $^3J$ = 6.20Hz, 17+18)
RMN $^{13}C$ ($CD_2Cl_2$ 75MHz : δ ppm = 168.9 (4); 167.4 (13); 163.0 (15); 154.0 (11); 137.9 (6); 133.9 (5); 127.9 (6+7);

127.3 (q, $^1J_{C-F}$ = 277.0Hz, 1); 126.8 (8+9); 116.1 (12); 69.8 (16); 39.8 (14); 34.1 (q, $^2J_{C-F}$ = 27.5Hz, 2); 33.9 (3); 22.0 (17+18)

RMN $^{19}F$ ($CDCl_3$, 282.4MHz) : δ ppm = -64.95 (t, $^3J$ = 10.8Hz)

**Composé 57 : 4-(2-(pyridin-4-yl)thiazol-4-yl)-N-(3,3,3-trifluoropropyl)benzamide**

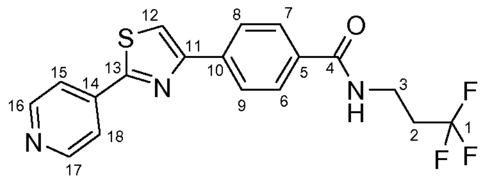

32.7mg (1eq.) de thioisonicotinamide sont ajoutés à une solution de 100mg (1eq.) de 4-(2-bromoacétyl)-N-(3,3,3-trifluoropropyl)benzamide **(46)** dans 6mL de THF. Le mélange est agité pendant une nuit au reflux. Le précipité formé est filtré puis lavé à l'éther diéthylique. Le solide orange obtenu est ensuite repris à l'acétate d'éthyle dans une ampoule à décanter, lavé avec une solution saturée de $K_2CO_3$ puis à l'eau (2x) et à la saumure (1x). La phase organique est séchée sur sulfate de magnésium puis évaporée sous pression réduite. Le résidu est purifié sur colonne de silice prépaquée (Cyclohexane 8:2 Isopropanol) pour donner 59mg (66%) de solide jaune.

LC-MS : $t_R$ = 2.32min (méthode c) ; m/z : $[M+H]^+$ = 378

RMN $^1H$ ($CD_3OD$, 300MHz) : δ ppm = 8.70 (m, 2H, 16+17); 8.20 (s, 1 H, 12); 8.18 (d, 2H, $^3J$ = 8.80Hz, 8+9); 8.08 (m, 2H, 15+18); 7.94 (d, 2H, $^3J$ = 8.80Hz, 6+7); 3.67 (t, 2H, $^3J$ = 7.00Hz, 3); 2.57 (qt, 2H, $^3J_{2-1}$ = 11.0Hz, $^3J_{2-3}$ = 7.00Hz, 2)

RMN $^{13}C$ ($CD_3OD$, 75MHz) : δ ppm = 168.5 (4); 164.7 (13); 155.9 (11); 149.9 (16+17); 141.2 (14); 137.1 (5); 133.7 (10); 127.5 (6+7); 126.6 (q, $^1J_{C-F}$ = 278.1Hz, 1); 126.2 (8+9); 120.6 (15+48); 117.2 (12); 33.1 (q, $^3J_{C-F}$ = 3.3Hz, 3); 32.7 (q, $^2J_{C-F}$ = 27.8Hz)

**Composé 58: 4-(2-(2-éthylpyridin-4-yl)thiazol-4-yl)-N-(3,3,3-trifluoropropyl)benzamide**

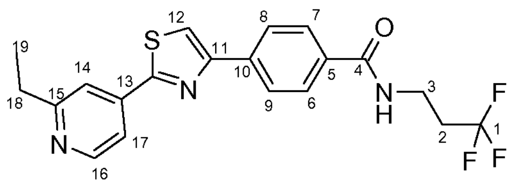

45mg (1.1eq.) d'éthionamide sont ajoutés à une solution de 100mg (1eq.) de 4-(2-bromoacétyl)-N-(3,3,3-trifluoropropyl)benzamide **(46)** dans 10mL de THF. Le mélange est agité à reflux pendant 24h. Le précipité formé est filtré puis lavé par une solution d'éther diéthylique. Le solide jaune obtenu est ensuite repris à l'acétate d'éthyle dans une ampoule à décanter, lavé avec une solution saturée de $K_2CO_3$ puis à l'eau (2x) et à la saumure (1x). La phase organique est séchée sur sulfate de magnésium puis évaporée sous pression réduite pour donner 75mg (63%) de solide jaune.

LCMS : $t_R$ = 3.12min (méthode c) ; (m/z) : $[M+H]^+$ = 406

RMN $^1H$ ($CDCl_3$, 300MHz) : δ ppm = 8.66 (d, 1H, $^3J$ = 5.30Hz, 16); 8.09 (d, 2H, $^3J$ = 8.50Hz, 8+9); 7.86 (d, 2H, $^3J$ = 8.5 Hz, 6+7); 7.78 (s, 1H, 12); 7.69 (m, 2H, 15+17); 6.45 (m, 1H, N**H**); 3.77 (q, 2H, $^3J$ = 6.30Hz, 3); 2.94 (q, 2H, $^3J$ = 7.60Hz, 19); 2.52 (qt, 2H, $^3J_{2-1}$ = 10.80Hz, $^3J_{2-3}$ = 6.40Hz, 2); 1.40 (t, 3H, $^3J$ = 7.60Hz, 20)

RMN $^{13}C$ ($CD_2Cl_2$, 75MHz) : δ ppm = 167.5 (4); 166.5 (13); 165.4 (18); 156.1 (16); 150.6 (16); 140.9 (14); 137.6 (10); 134.4 (5); 128.1 (q, $^1J_{C-F}$ = 276.9Hz, 1); 128.0 (6+7); 127.0 (8+9); 119.1 (17); 118.2 (15); 116.4 (12); 34.26 (q, $^2J_{C,F}$ = 27.7Hz, 2); 34.1 (3); 32.0 (19); 14.2 (20)

**Scheme 9:**

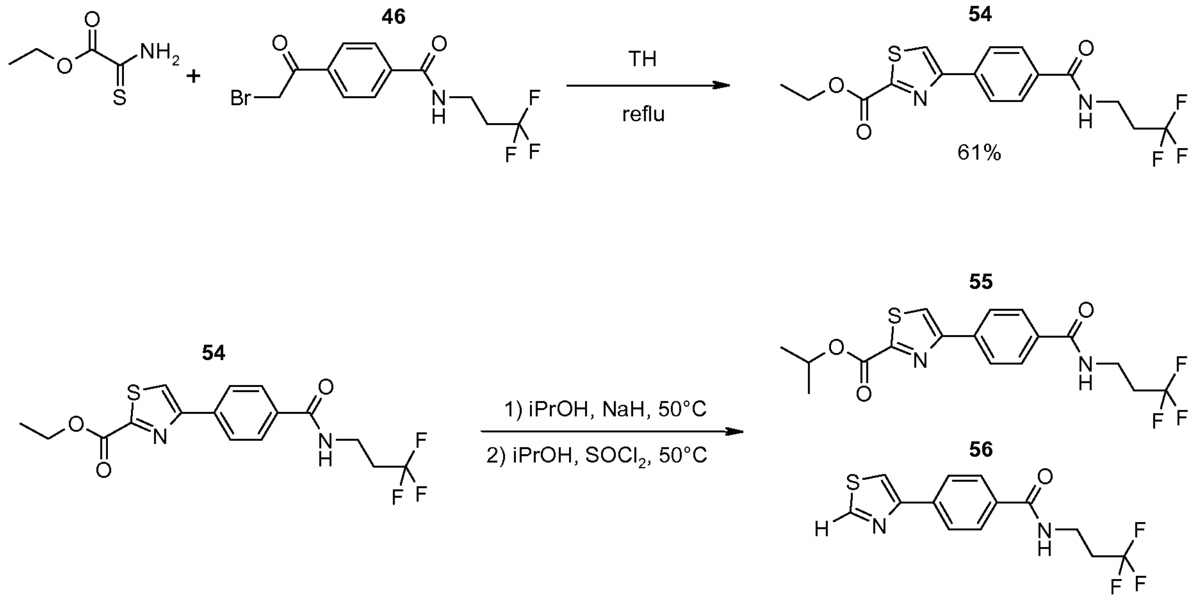


**Composé 54 : 4-(4-(3,3,3-trifluoropropylcarbamoyl)phényl)thiazole-2-carboxylate d'éthyle**

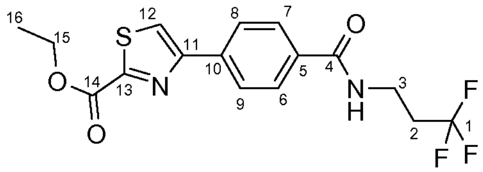

150mg (1eq.) de 4-(2-bromoacétyl)-N-(3,3,3-trifluoropropyl)benzamide **(46),** 71 mg (1.2eq.) de thiooxamate d'éthyle et 15mL de THF sont introduits dans un ballon de 50mL. Le mélange est chauffé à reflux pendant 24h. 11.5mg (0.2eq) de thioamide sont ajoutés et la solution est à nouveau agitée pendant 24h au reflux. 350mg (3eq.) de résine PS-Tos-$NHNH_2$ sont ajoutés et le milieu réactionnel est agité à TA pendant une nuit. La solution est ensuite filtrée puis le THF est évaporé sous pression réduite. Le résidu est repris dans l'acétate d'éthyle puis lavé à l'aide d'une solution aqueuse saturée en $K_2CO_3$ (2x) et à la saumure (1x). La phase organique est séchée sur sulfate de magnésium puis concentrée sous pression réduite. Le résidu obtenu est purifié sur colonne de silice prépaquée (Cyclohexane 8:2 AcOEt $\rightarrow$ Cyclohexane 7:3 AcOEt) pour donner 100.1 mg (61%) de poudre beige.

LC-MS : $t_R$ = 3.22min (méthode c) ; m/z : $[M+H]^+$ = 373
RMN $^1H$ ($CDCl_3$, 300MHz) : $\delta$ ppm = 8.05 (d, 2H, $^3J$ = 8.60Hz) ; 7.81-7.85 (m, 3H, 6+7+12); 6.44 (m, 1H, $^3J$ = 5.90Hz, N**H**); 4.53 (q, 2H, $^3J$ = 7.10Hz, 15); 3.75 (q, $^3J$ = 6.30Hz, 3); 2.50 (qt, 2H, $^3J_{2-1}$ = 10.80Hz, $^3J_{2-3}$ = 6.60Hz, 2); 1.47 (t, 3H, $^3J$ = 7.10Hz, 16)

**Composés 55: 4-(4-(3,3,3-trifluoropropylcarbamoyl)phényl)thiazole-2-carboxylate d'isopropyle et 56 : 4-(thiazol-4-yl)-N-(3,3,3-trifluoropropyl)benzamide**

**55**

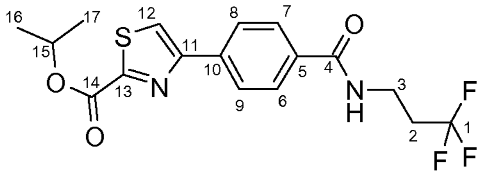

**56**

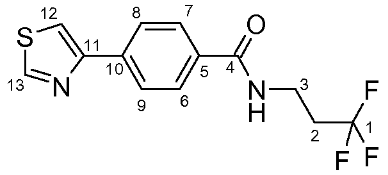

100mg (1eq.) de 4-(2-bromoacétyl)-N-(3,3,3-trifluoropropyl)benzamide **(46),** 40mg (1.1eq.) de thiooxamate d'éthyle et 10mL de THF sont introduits dans un ballon de 25mL. Le mélange est agité pendant 24h au reflux. Le THF est évaporé sous pression réduite. Le produit est alors solubilisé dans 2.5mL d'isopropanol.

Dans un ballon refroidi à 0°C, 20.4mg (3eq.) de sodium sont dissous sous Argon dans 2.5mL d'isopropanol. Le mélange est chauffé à 30°C jusqu'à dissolution complète du sodium. A cette solution est ajoutée goutte à goutte la solution dans l'isopropanol précédemment réalisée. Le mélange est ensuite agité pendant une nuit au reflux. 500$\mu$L de $SOCl_2$ sont alors ajoutés. La solution est agitée pendant une nuit au 50°C. Le milieu réactionnel est évaporé sous pression réduite et les deux composés **55** et **56** formés en proportions identiques sont séparés par HPLC préparative pour donner 24mg de **55** et 21.7mg de **56.**

**Composé 55 :**

LC-MS : $t_R$ = 4.90min (méthode d) ; m/z : $[M+H]^+$ = 387

RMN $^1$H ($CDCl_3$, 300MHz) : $\delta$ ppm = 8.07 (d, 2H, $^3J$ = 8.50Hz); 7.84 (s, 1 H, 12); 7.85 (d, 2H, $^3J$ = 8.50Hz, 6+7); 6.42 (m, 1H, N**H**); 5.37 (sp, 1H, $^3J$ = 6.30Hz, 15); 3.77 (q, 2H, $^3J$ = 6.30Hz, 3); 2.52 (qt, 2H, $^3J_{2-1}$ = 10.70Hz, $^3J_{2-3}$ = 6.40Hz, 2); 1.46 (d, 6H, $^3J$ = 6.30Hz, 16+17)

RMN $^{13}$C ($CD_2Cl_2$ 75MHz) : $\delta$ ppm = 166.7 (4); 159.2 (13 ou 14); 159.0 (13 ou 14); 156.0 (11); 136.6 (10); 134.1 (5); 127.5 (6+7); 126.7 (q, $^1J_{C-F}$ = 276.8Hz, 1); 126.6 (8+9); 120.2 (12); 70.8 (15); 33.6 (q, $^2J_{C-F}$ = 27.5Hz, 2); 33.5 (q, $^3J_{C-F}$ = 3.6Hz, 3); 21.5 (16+17)

**Composé 56 :**

LC-MS : $t_R$ = 5.82min (méthode d) ; m/z : $[M+H]^+$ = 301

RMN $^1$H ($CDCl_3$, 300MHz) : $\delta$ ppm = 8.92 (d, 1H, $^4J$ = 1.80Hz, 13); 8.03 (d, 2H, $^3J$ = 8.40Hz, 8+9); 7.85 (d, 2H, $^3J$ = 8.40Hz, 6+7); 7.64 (d, 1H, $^4J$ = 1.80Hz, 12); 3.77 (q, 2H, $^3J$ = 6.30Hz, 3); 2.51 (qt, 2H, $^3J_{2-1}$ = 10.80Hz, $^3J_{2-3}$ = 6.40Hz, 2)

RMN $^{13}$C ($CD_2Cl_2$, 75MHz) : $\delta$ ppm = 166.8 (4); 155.0 (11); 153.3 (13); 137.3 (10); 133.5 (5); 127.4 (6+7); 126.5 (8+9); 114.4 (12); 33.6 (q, $^2J_{C-F}$ = 27.3Hz, 2); 33.4 (3)

## Scheme 10

**43** **62**

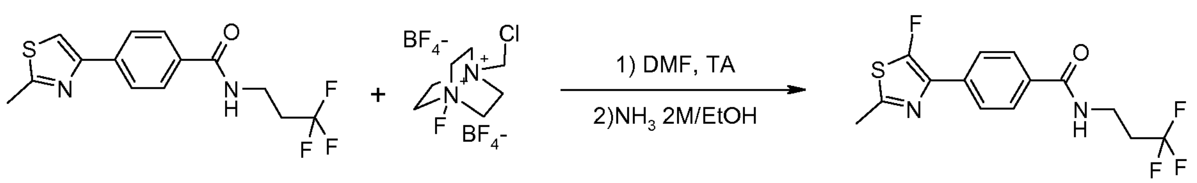


16%

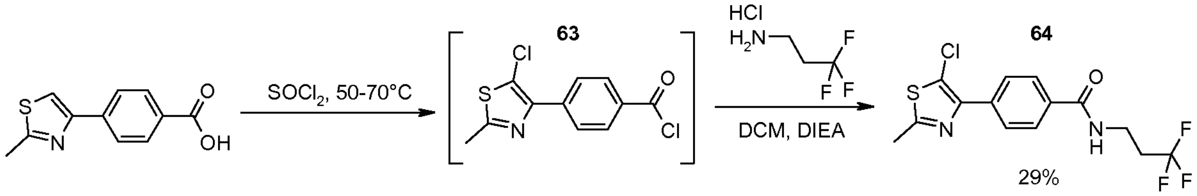

**Composé 62 : 4-(5-fluoro-2-méthylthiazol-4-yl)-N-(3,3,3-trifluoropropyl)benzamide**

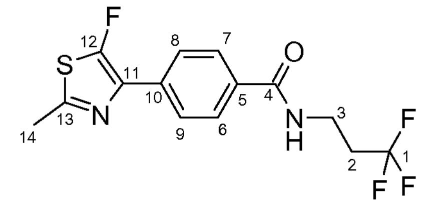

101 mg (1 eq.) de Selectfluor sont ajoutés à une solution de 90mg de 4-(2-méthylthiazol-4-yl)-N-(3,3,3-trifluoropropyl)benzamide **(43)** dans 1.1 mL de DMF anhydre. La solution est agitée pendant 15h à 5°C. L'analyse LCMS du milieu réactionnel montre une très faible conversion du produit de départ. 0.5eq de Selectfluor sont alors ajoutés à 0°C et la solution est agitée pendant une nuit à 15°C. 1 eq. supplémentaire de Selectfluor est alors ajouté à 0°C puis la solution est agitée pendant 24h à TA. Une conversion > 70% est ainsi obtenue. 1 mL d'une solution d'ammoniaque 2M dans l'éthanol ainsi que 0.5mL d'eau sont ajoutés à 0°C et la solution est agitée à TA pendant une nuit. Le solvant est alors évaporé sous pression réduite. L'huile jaune obtenue est reprise dans l'acétate d'éthyle puis lavée à l'eau (2x) et à la saumure (1x). La phase organique est séchée sur sulfate de magnésium puis concentrée sous pression réduite. Le solide jaune obtenu est purifié par HPLC préparative pour donner 15mg (16%) de poudre blanche.

LC-MS : $t_R$ = 2.93min (méthode c), 5.32min (méthode d) ; m/z : $[M+H]^+$ = 333
RMN $^1H$ ($CD_2Cl_2$, 300MHz) : δ ppm = 7.97 (d, 2H, $^3J$ = 8.30Hz, 8+9); 7.81 (d, 2H, $^3J$ = 8.30Hz, 6+7); 6.52 (br s, 1H, N**H**); 3.70 (q, 2H, $^3J$ = 6.50Hz, 3); 3.63 (d, 3H, $^5J$ = 2.30Hz, 14); 2.50 (qt, $^3J_{2-1}$ = 10.90Hz, $^3J_{2-3}$ = 6.70Hz, 2)
RMN $^{13}C$ ($CD_2Cl_2$ 75MHz : δ ppm = 167.4 (4); 158.2 (d, $^1J_{C-F}$=302.1Hz, 12); 154.5 (d, $^3J_{C-F}$=10.XHz, 13); 135.7 (d, $^3J_{C-F}$=5.8Hz, 10); 133.6 (5); 133.5 (d, $^2J_{C-F}$=12.7Hz, 11); 127.8 (6+7); 127.3 (d, $^4J_{C-F}$=6.1Hz, 8+9); 127.2 (q, $^1J_{C-F}$=275.4Hz , 1); 34.2 (q, $^2J_{C-F}$=27.0Hz, 2), 34.0 (3); 20.7 (14)

**Composé 64 : 4-(5-chloro-2-méthylthiazol-4-yl)-N-(3,3,3-trifluoropropyl)benzamide**

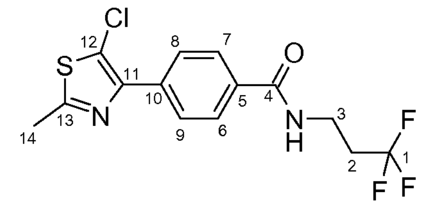

50mg (1 eq.) d'acide 4-(2-méthylthiazol-4-yl)benzoïque sont ajoutés à une solution de 5mL de chlorure de thionyle. Le mélange est agité à 50°C pendant 2h. 2mL de chlorure de thionyle sont alors ajoutés et la solution est agitée 2h supplémentaires à 50°C. Un troisième ajout de 2mL de $SOCl_2$ est ensuite effectué et la solution est agitée 2h supplémentaires à 50°C. Le milieu réactionnel est alors évaporé sous pression réduite et le résidu obtenu (composé **63)** est repris dans le dichlorométhane. 34mg (1 eq.) de chlorhydrate de 3,3,3-trifluoropropylamine et 158μL (4eq.) de DIEA sont ajoutés au milieu réactionnel et la solution est agitée pendant une nuit à TA. Le dichlorométhane est alors évaporé sous pression réduite puis le résidu est repris dans l'acétate d'éthyle. La phase organique est lavée à la saumure (2x), séchée sur sulfate de magnésium puis évaporée sous pression réduite. L'huile beige obtenue est alors purifiée une première fois sur colonne de silice prépaquée (Cyclohexane 85:15 iPrOH). La poudre blanche récupérée n'est pas pure. Le produit est ensuite purifié sur HPLC préparative pour donner 23.5mg (29%) de poudre blanche.

LCMS : $t_R$ = 3.37min (méthode c) ; m/z : $[M+H]^+$ = 349, $[M-H]^-$ = 347
RMN $^1H$ ($CD_2Cl_2$, 300MHz) : δ ppm = 8.08 (d, 2H, $^3J$ = 8.40Hz, 8+9); 7.84 (d, 2H, $^3J$ = 8.40Hz, 6+7); 6.50 (m, 1H, N**H**); 3.74 (q, 2H, $^3J$ =6.40Hz, 3); 2.70 (s, 3H, 14); 2.53 (qt, 2H, $^3J_{2-1}$ = 10.90Hz, $^3J_{2-3}$ = 6.40Hz, 2)
RMN $^{13}C$ ($CD_2Cl_2$, 75MHz) : δ ppm = 167.4 (4); 163.6 (13); 148.2 (11); 136.7 (10); 134.1 (5); 128.8 (6+7); 127.4 (8+9); 127.3 (q, $^1J_{C,F}$ = 277Hz, 1); 121.1 (12); 34.1 (q, $^2J_{C,F}$ = 27.6Hz, 2); 34.0 (q, $^3J_{C,F}$ = 3.1 Hz, 3); 20.1 (14)

## Scheme 11:

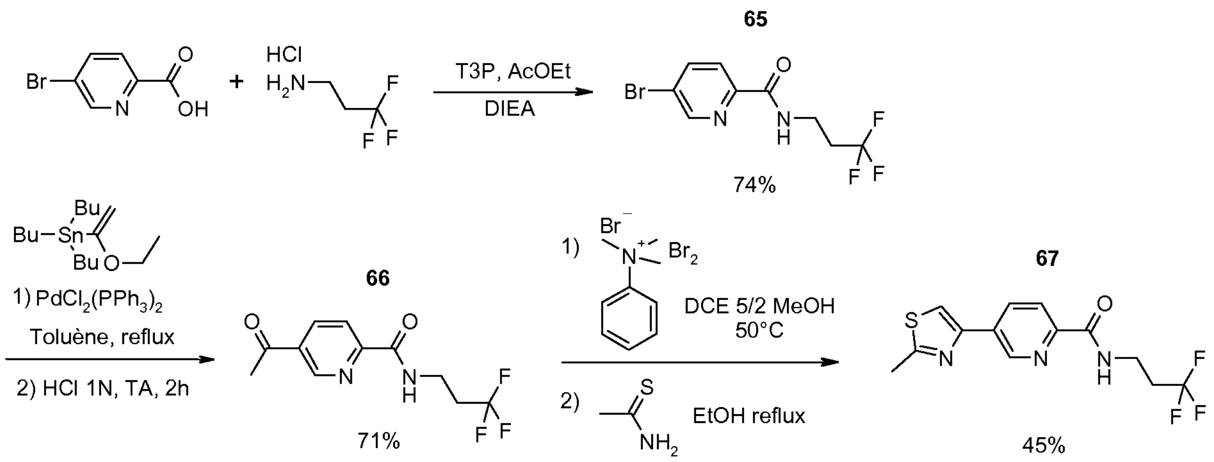


**Composé 65: 5-bromo-N-(3,3,3-trifluoropropyl)picolinamide**

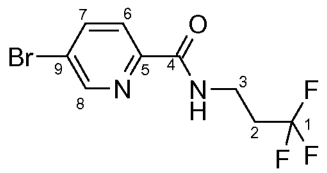

296mg (1 eq.) de chlorhydrate 3,3,3-trifluoropropylamine sont ajoutés à une solution de 400mg (1eq.) d'acide 5-bromopicolinique, 1.40mL (1.2eq.) de solution de T3P à 50% dans l'AcOEt et 1.03mL (3eq.) de DIEA dans 10mL d'AcOEt. Le mélange est agité pendant 3h à TA. On ajoute alors de l'eau et de l'acétate d'éthyle. La phase organique est lavée à l'eau (2x) puis à la saumure (1x), séchée sur sulfate de magnésium et évaporée sous pression réduite pour donner 433mg (74%) de solide blanc.

LC-MS : $t_R$ = 2.58min (méthode c) ; m/z : $[M+H]^+$ = 297
RMN $^1H$ ($CD_3OD$, 300MHz) : $\delta$ ppm = 8.73 (dd, 1H, $^4J_{7-8}$ = 2.25Hz, $^5J_{8-6}$ = 0.52Hz, 8); 8.16 (dd, 1H, $^3J_{7-6}$ = 8.39Hz, $^4J_{7-8}$= 2.27Hz, 7); 8.01 (dd, 1H, $^3J_{6-7}$= 8.33Hz, $^5J_{6-8}$ = 0.59Hz, 6); 3.67 (q, 2H, $^3J$ = 6.79Hz, 3); 2.53 (qt, 2H, $^3J_{2-1}$ = 10.99Hz, $^3J_{2-3}$ = 7.11 Hz, 2)

**Composé 66 : 5-acétyl-N-(3,3,3-trifluoropropyl)picolinamide**

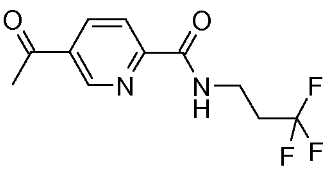

400mg (1 eq.) de 5-bromo-N-(3,3,3-trifluoropropyl)picolinamide **(65)** sont ajoutés à une solution de 95mg (0.1eq.) de $PdCl_2(PPh_3)_2$ dans 3.5mL de toluène dans un tube de type Schlenk sous argon. Le mélange est agité pendant 5min à TA puis 545µL (1.2eq.) de tributyl(1-ethoxyvinyl)stannane sont ajoutés toujours sous argon. Le milieu réactionnel est alors agité pendant une nuit à 90°C. La solution est refroidie à TA puis 1 mL d'une solution aqueuse d'acide chlorhydrique 1 N sont ajoutés. Le mélange est alors agité pendant 1 h à TA. Le milieu réactionnel est neutralisé à l'aide d'une solution aqueuse saturée en $NaHCO_3$ puis extrait à l'acétate d'éthyle. Les phases organiques sont rassemblées, lavées à la saumure (2x), séchées sur sulfate de magnésium et filtrées sur célite. Le résidu jaune obtenu est

purifié sur colonne de silice prépaquée (Cyclohexane 95:5 AcOEt → 9:1 → 85:15 → 75:25 → 7:3) pour donner 250mg (71%) de solide blanc.

LC-MS : $t_R$ = 1.57min (méthode c) ; m/z : $[M+H]^+$ = 261

**Composé 67 : 5-(2-méthylthiazol-4-yl)-N-(3,3,3-trifluoropropyl)picolinamide**

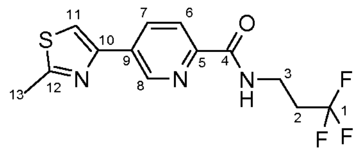

332mg (1 eq.) de tribromure de triméthylphénylammonium sont ajoutés à une solution de 230mg (1 eq.) de 5-acétyl-N-(3,3,3-trifluoropropyl)picolinamide **(66)** dans un mélange DCE (12.5mL) / MeOH (5mL). Le mélange est agité pendant 24h à 70°C. Le mélange de solvant est alors évaporé sous pression réduite. Le résidu est repris dans l'acétate d'éthyle et lavé à l'eau puis à la saumure. La phase organique est séchée sur sulfate de magnésium et évaporée sous pression réduite pour donner un solide jaune qui est ensuite solubilisé dans 2mL d'EtOH absolu. 101 mg (1.5eq.) de thioacétamide sont alors ajoutés et le milieu réactionnel est agité pendant 4h à reflux. L'EtOH est évaporé sous pression réduite et le résidu est repris dans l'acétate d'éthyle. La phase organique est lavée à l'eau (2x) et à la saumure (1x), séchée sur sulfate de magnésium puis évaporée sous pression réduite. Le résidu est purifié par HPLC préparative pour donner 125mg (45%) de poudre blanche.

LC-MS : $t_R$ = 2.53min (méthode c) ; m/z : $[M+H]^+$ = 316
RMN $^1H$ ($CD_3OD$, 300MHz) : δ ppm = 9.15 (dd, 1H, $^4J_{8-7}$ = 2.20Hz, $^5J_{8-6}$ = 0.75Hz, 8); 8.40 (dd, 1H, $^3J_{7-6}$ = 8.18Hz, $^4J_{7-8}$ = 2.23Hz, 7); 8.12 (dd, 1H, $^3J_{6-7}$ = 8.18Hz, $^5J_{6-8}$ = 0.74Hz, 6); 7.94 (s, 1 H, 11); 3.69 (t, 2H, $^3J$ = 7.11 Hz, 3); 2.78 (s, 3H, 13); 2.55 (qt, $^3J_{2-1}$ = 10.97Hz, $^3J_{2-3}$ = 7.22Hz, 2)
RMN $^{13}C$ ($CD_3OD$, 75MHz) : δ ppm = 167.7 (12); 165.2 (4); 150.7 (10); 148.2 (5); 146.2 (8); 134.3 (7); 132.7 (9); 126.5 (q, $^1J_{C-F}$ = 275.9Hz, 1); 121.7 (6); 116.2 (11); 32.8 (q, $^2J_{C-F}$ = 27.8Hz, 2); 32.6 (q, $^3J_{C-F}$ = 3.7Hz, 3); 17.5 (13)

## Scheme 12 :

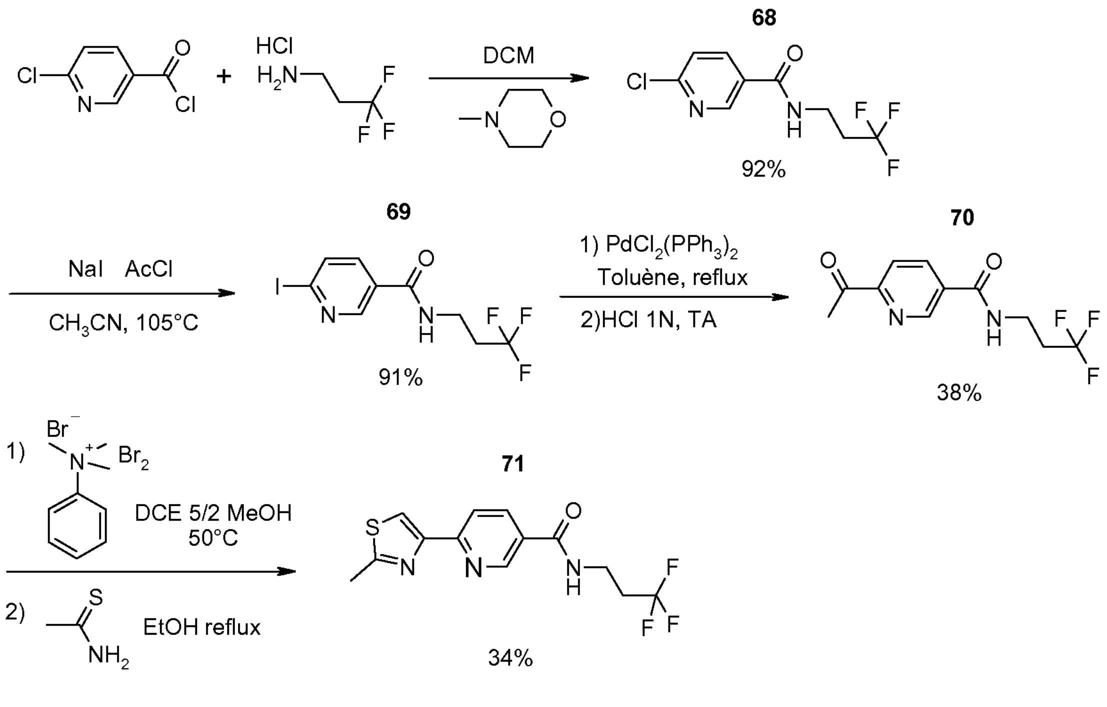

**Composé 68 : 6-chloro-N-(3,3,3-trifluoropropyl)nicotinamide**

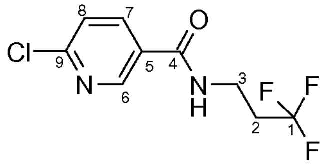

700mg (1 eq.) de chlorure d'acide 6-chloro-nicotinique sont ajoutés à une solution de 595mg (1 eq.) de chlorhydrate de 3,3,3-trifluoropropylamine et 1.75mL (4eq.) de N-méthylmorpholine dans 40mL de DCM anhydre. Le milieu réactionnel est agité pendant 1h à TA. Un équivalent supplémentaire (700mg) de chlorure d'acide est ajouté et la solution est agitée pendant une nuit à TA. Le DCM est évaporé sous pression réduite. Le solide obtenu est repris dans un mélange Cylohexane 1:1 Acétate d'éthyle puis filtré sur silice. Le produit est ensuite purifié sur colonne de silice prépaquée (Cyclohexane 7:3 AcOEt) pour donner 918mg (92%) de poudre blanche.

LC-MS : $t_R$ = 2.32min (méthode c) ; m/z : $[M+H]^+$ = 253, $[M-H]^-$ = 251

**Composé 69 : 6-iodo-N-(3,3,3-trifluoropropyl)nicotinamide**

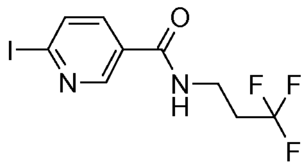

28μL (0.2eq.) de chlorure d'acide acétique sont ajoutés sous argon à une solution de 500mg (1 eq.) de 6-chloro-N-(3,3,3-trifluoropropyl)nicotinamide (68), 1.48g (5eq.) de NaI dans 15mL d'acétonitrile dans un tube à carrousel. La solution est agitée à 105°C pendant une nuit. L'acétonitrile est évaporée sous pression réduite, le produit est alors repris dans l'acétate d'éthyle, filtré, lavé avec une solution aqueuse de $Na_2SO_3$ à 10% puis avec une solution aqueuse de $Na_2CO_3$ à 5% puis à la saumure. La phase organique est alors séchée sur sulfate de magnésium puis évaporée sous pression réduite pour donner 619mg (91%) de poudre blanche.

LC-MS : $t_R$ = 2.33min (méthode c) ; m/z : $[M+H]^+$ = 345, $[M-H]^-$ = 343

**Composé 70 : 6-acétyl-N-(3,3,3-trifluoropropyl)nicotinamide**

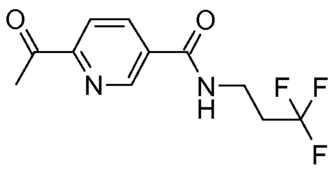

300mg (1 eq.) de 6-iodo-N-(3,3,3-trifluoropropyl)nicotinamide **(69)** sont ajoutés à une solution de 61mg (0.1eq.) de $PdCl_2(PPh_3)_2$ dans 2.6mL de toluène. Le milieu réactionnel est agité 5min à TA puis 353μL (1 .2eq.) de tributyl(1-ethoxyvinyl)stannane sont ajoutés sous argon. Le milieu réactionnel est alors agité sous argon pendant une nuit à 110°C. Le milieu réactionnel est refroidi à TA, puis 8mL d'une solution aqueuse d'HCl 1 N sont ajoutés et la solution est agitée pendant 2h à TA. Le milieu réactionnel est neutralisé à l'aide d'une solution aqueuse saturée en $NaHCO_3$. Le milieu est extrait à l'acétate d'éthyle. Les phases organiques sont rassemblées, lavées à la saumure (2x), séchées sur sulfate de magnésium et filtrées sur célite. Le résidu marron obtenu est solubilisé dans 5mL d'acétonitrile puis filtré. Le filtrat est

alors purifié par HPLC préparative pour donner 89mg (35%) de poudre blanche.

LC-MS : $t_R$ = 4.10min (méthode d) ; m/z : $[M+H]^+$ = 261, $[M-H]^-$ = 259

**Composé 71 : 6-(2-méthylthiazol-4-yl)-N-(3,3,3-trifluoropropyl)nicotinamide**

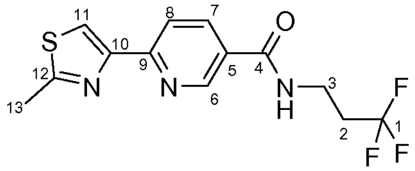

129mg (1 eq.) de tribromure de triméthylphénylammonium sont ajoutés à une solution de 89mg (1 eq.) de 6-acétyl-N-(3,3,3-trifluoropropyl)nicotinamide **(70)** dans 5.9mL d'un mélange DCE 5:2 MeOH. Le milieu réactionnel est agité à 50°C pendant 2h. L'analyse LCMS du milieu montre un taux de conversion quasi nul, la réaction est alors chauffée à 70°C pendant 72h. L'analyse LCMS du milieu montre une conversion non complète du milieu réactionnel et la formation de deux produits majoritaires : la cétone mono-bromée et la cétone mono-chlorée, ainsi qu'un peu de cétone di-chlorée. La réaction est néanmoins stoppée, et le mélange de solvant est évaporé sous pression réduite. Le résidu est repris dans 1 mL d'éthanol absolu. 26 mg (1 eq.) de thioacétamide sont alors ajoutés et la solution est agitée pendant 1h à reflux. 13mg (0,5eq.) supplémentaires sont ajoutés et le milieu réactionnel est agité pendant 3h au reflux puis refroidi à TA. 6mL d'éther diéthylique sont ajoutés et la solution est refroidie au réfrigérateur pendant une nuit. Le précipité formé est filtré puis lavé à l'éther diéthylique. Le précipité est repris dans l'acétate d'éthyle puis lavé avec une solution aqueuse saturée en $NaHCO_3$ (2x) puis à la saumure (1x). La phase organique est ensuite séchée sur sulfate de magnésium puis évaporée sous pression réduite afin de donner 39mg (34%) de poudre blanche.

LC-MS : $t_R$ = 2.28min (méthode c) ; m/z : $[M+H]^+$ = 316, $[M-H]^-$ = 314
RMN $^1H$ ($CD_3OD$, 300MHz) : $\delta$ ppm = 8.97 (dd, 1 H, $^4J_{6-7}$ = 2.29Hz, $^5J_{6-8}$ = 0.84Hz, 6); 8.24 (dd, 1H, $^3J_{7-8}$ = 8.27Hz, $^4J_{7-6}$ = 2.30Hz, 7); 8.13 (dd, 1H, $^3J_{8-7}$ = 8.30Hz, $^5J_{8-6}$ = 0.81 Hz, 8); 8.11 (s, 1 H, 11); 3.65 (t, 2H, $^3J$ = 7.00Hz, 3); 2.76 (s, 3H, 13); 2.55 (qt, 2H, $^3J_{2-1}$ = 10.84Hz, $^3J_{2-3}$ = 7.13Hz, 2)
RMN $^{13}C$ ($CD_3OD$, 75MHz) : $\delta$ ppm = 167.3 (12); 166.4 (4); 154.4 (9); 153.4 (10); 148.1 (6); 136.1 (7); 128.5 (5); 126.6 (q, $^1J_{C-F}$ = 276.3Hz, 1); 120.3 (8); 118.8 (11); 33.1 (q, $^3J_{C-F}$ = 9.74Hz, 3); 32.6 (q, $^2J_{C-F}$ = 27.7Hz, 2); 17.6 (13)

**Scheme 13 :**

**a/**

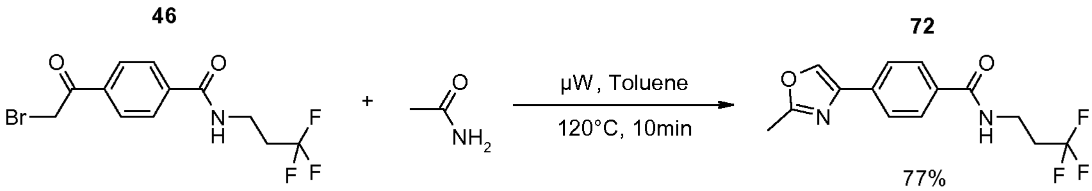


**b/**

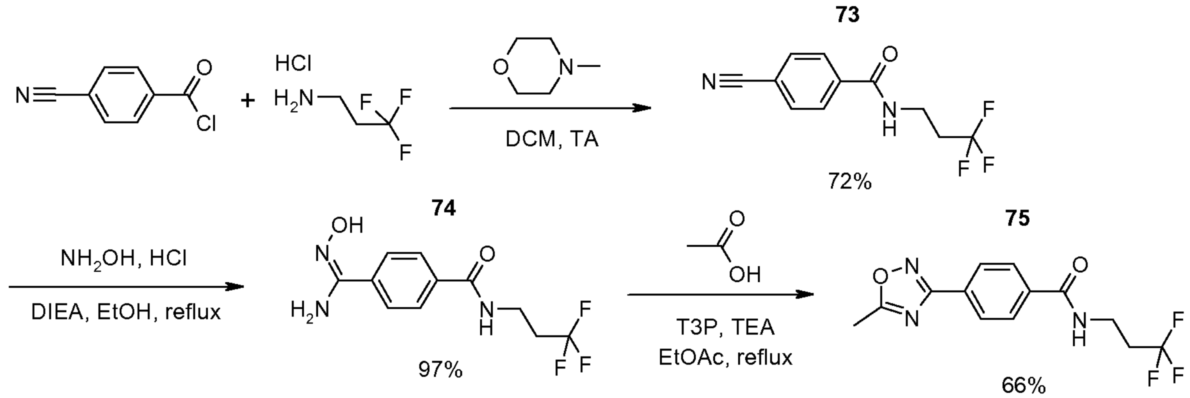


**c/**

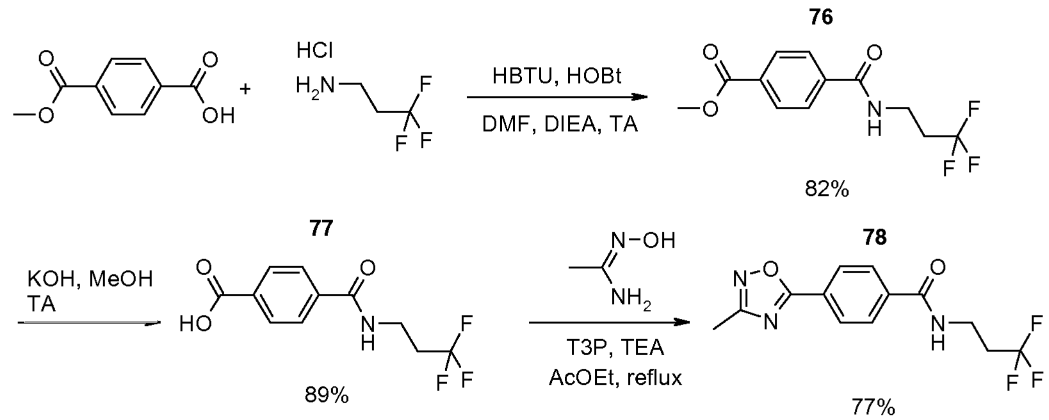


**d/**

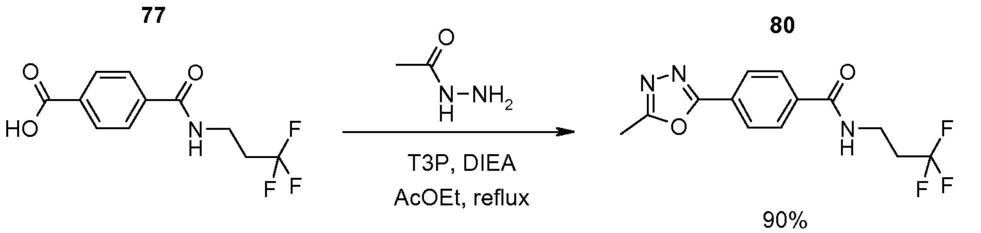

**e/**

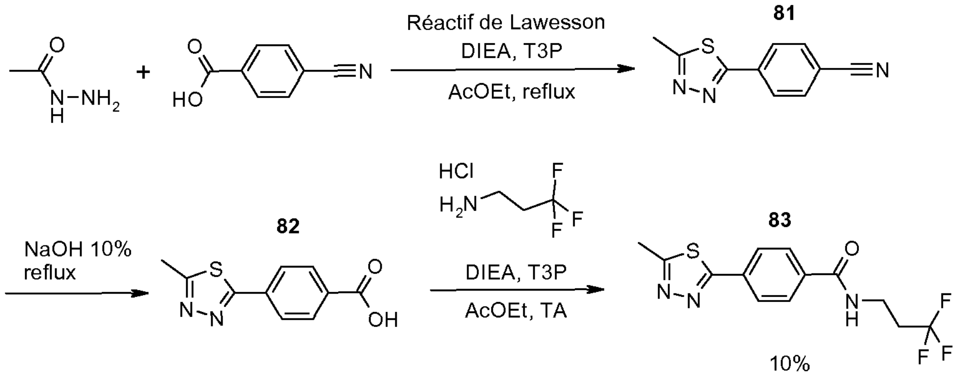
Réactif de Lawesson
DIEA, T3P
AcOEt, reflux
81
NaOH 10%
reflux
82
HCl
DIEA, T3P
AcOEt, TA
83
10%

**f/**

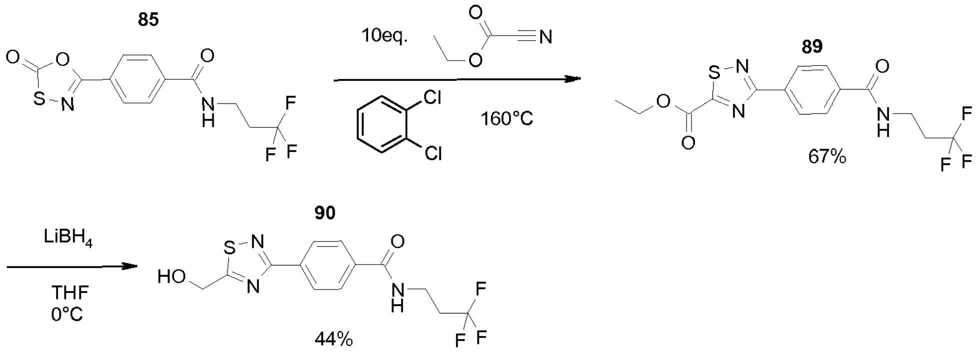
85
10eq.
160°C
89
67%
LiBH$_4$
THF
0°C
90
44%

**g/**

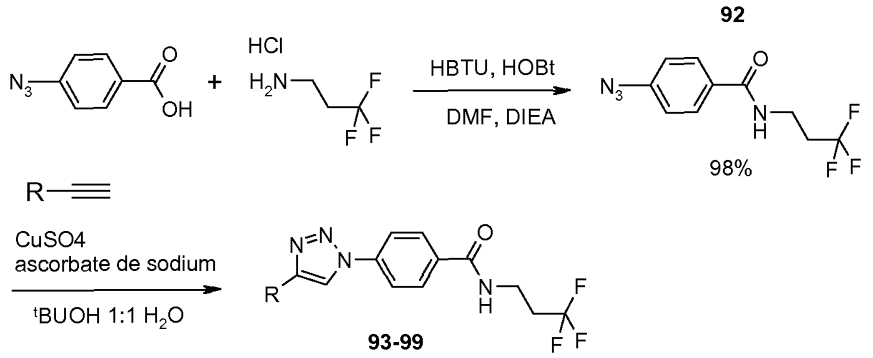
HCl
HBTU, HOBt
DMF, DIEA
92
98%
CuSO4
ascorbate de sodium
$^t$BUOH 1:1 H$_2$O
93-99

**h/**

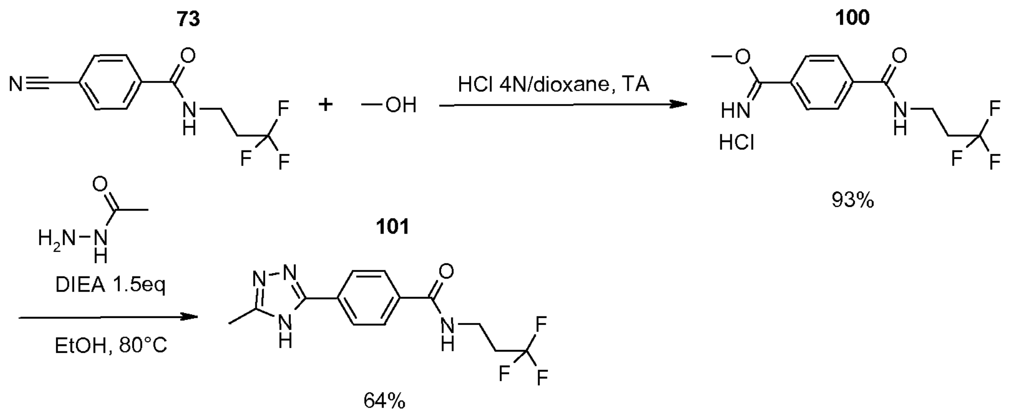
73
HCl 4N/dioxane, TA
100
HCl
93%
DIEA 1.5eq
EtOH, 80°C
101
64%

**Composé 72 : 4-(2-méthyloxazol-4-yl)-N-(3,3,3-trifluoropropyl)benzamide**

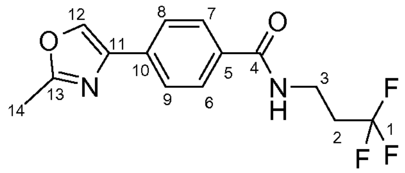

100mg (1 eq.) de 4-acétyl-N-(3,3,3-trifluoropropyl)benzamide (**45**) sont ajoutés à une solution de 21 mg (1.2eq.) d'acétamide dans 300μL de toluène. Le milieu réactionnel est agité en réacteur micro-ondes pendant 10min à 110°C ($P_{max}$= 300W). 240mg (3eq.) de résine Tos-$NHNH_2$ sont ajoutés. La solution est agitée une nuit à TA puis filtrée. La solution est reprise dans l'acétate d'éthyle puis lavée à l'eau (2x) et à la saumure (1x). La phase organique est séchée sur sulfate de magnésium puis évaporée sous pression réduite afin de donner 68mg (77%) de poudre beige.

LC-MS : $t_R$ = 2.30min (méthode c) ; m/z : $[M+H]^+$ = 299, $[M-H]^-$ = 297
RMN $^1H$ ($CD_2Cl_2$, 300MHz) : δ ppm = 7.97 (s, 12); 7.84-7.77 (m, 4H, 6+7+8+9); 3.70 (q, 2H, $^3J$ = 6.50Hz, 3); 2.60-2.44 (m, 3H, 14+2)
RMN $^{13}C$ ($CD_2Cl_2$, 75MHz) : δ ppm = 167.6 (4); 162.8 (13); 140.0 (11); 134.9 (5 ou 10); 134.8 (5 ou 10); 133.6 (12); 127.8 (6+7 ou 8+9); 127.1 (q, $^1J_{C-F}$ = 277.0Hz, 1); 125.7 (6+7 ou 8+9); 33.8 (q, $^2J_{C-F}$ = 27.1 Hz, 2); 33.8 (q, $^3J_{C-F}$ = 3.7Hz, 3); 14.0 (3)

**Composé 73 : 4-cyano-N-(3,3,3-trifluoropropyl)benzamide**

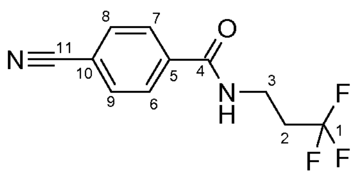

305mg (1 eq.) de chlorhydrate de 3,3,3-trifluoropropylamine sont ajoutés à une solution de 300mg (1eq.) d'acide 4-cyanobenzoïque, 1.46mL (1.2eq.) de solution de T3P à 50% dans l'acétate d'éthyle et 1.4mL (4eq.) de DIEA dans 3mL d'acétate d'éthyle. Le milieu réactionnel est agité une nuit à TA. La solution est alors lavée à l'eau (2x) puis à la saumure (1x). La phase organique est séchée sur sulfate de magnésium, évaporée sous pression réduite pour donner 424mg (86%) de solide blanc.

LC-MS : $t_R$ = 2.31 min (méthode c) ; m/z : $[M-H]^-$ = 241
RMN $^1H$ ($CDCl_3$, 300MHz) : δ ppm = 7.86 (d, 2H, $^3J$ = 8.47Hz, 6+7); 7.75 (d, 2H, $^3J$ = 8.47Hz, 8+9); 6.50 (br, s, 0.5H, N**H**); 3.74 (q, 2H, $^3J$ = 6.30Hz; 3); 2.49 (qt, 2H, $^3J_{2-1}$ = 10.69Hz, $^3J_{2-3}$ = 6.38Hz; 2)

**Composé 74 : 4-(N'-hydroxycarbamimidoyl)-N-(3,3,3-trifluoropropyl)benzamide**

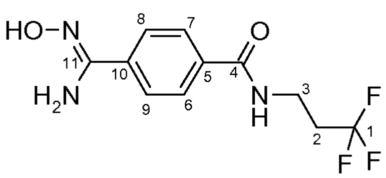

300mg (1 eq.) de 4-cyano-N-(3,3,3-trifluoro-propyl)-benzamide **(73)** sont ajoutés à une solution de 129mg (1.5eq.) de chlorhydrate d'hydroxylamine et 343μL (1.6eq.) de DIEA dans 2.5mL d'éthanol. La solution est agitée à

reflux pendant 2h. Le solvant est alors évaporé sous pression réduite. Le résidu est repris dans l'acétate d'éthyle puis lavé à l'eau (2x) et à la saumure (1x). La phase organique est séchée sur sulfate de magnésium et évaporée sous pression réduite pour donner 330mg (97%) de poudre blanche.

LC-MS : $t_R$ = 3.74min (méthode d) ; m/z : $[M-H]^-$ = 274
RMN $^1H$ (DMSO-$d_6$, 300MHz) : $\delta$ ppm = 9.79 (s, 1 H, O**H**); 8.70 (t, 1H, $^3J$ = 5.52Hz, N**H**-amide); 7.83 (d, 2H, $^3J$ = 8.55Hz, 6+7 ou 8+9); 7.77 (d, 2H, $^3J$ = 8.48Hz, 7+6 ou 8+9); 5.89 (s, 2H, N$H_2$); 3.50 (q, 2H, $^3J$ = 6.49Hz, 3); 2.55 (qt, 2H, $^3J_{2-1}$ = 11.42Hz, $^3J_{2-3}$ = 6.94Hz, 2).

**Composé 75 : 4-(5-méthyl-1,2,4-oxadiazol-3-yl)-N-(3,3,3-trifluoropropyl)benzamide**

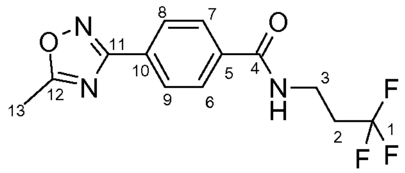

330mg (1 eq.) de 4-(N'-hydroxycarbamimidoyl)-N-(3,3,3-trifluoropropyl)benzamide **(74)** sont ajoutés à une solution de 78µL (1.1 eq.) d'acide acétique, 1.84mL (2.5eq.) de solution de T3P à 50% dans l'acétate d'éthyle et 643µL (3eq.) de DIEA dans 9mL d'acétate d'éthyle. La solution est agitée pendant une nuit au reflux. La conversion est incomplète. Le milieu réactionnel est alors évaporé sous pression réduite. 1mL d'acétate d'éthyle, 368µL (0.5eq) de T3P et 107µL (0.5eq.) de DIEA sont ajoutés. La solution est agitée pendant 48h au reflux. 2mL d'eau sont alors ajoutés et la réaction est agitée pendant 15min à TA. La phase organique est ensuite lavée à l'eau (2x) puis à la saumure (1x), séchée sur sulfate de magnésium et évaporée sous pression réduite. Le résidu obtenu est purifié sur colonne de silice prépaquée (Cyclohexane 9:1 Isopropanol) pour donner 215mg (66%) de solide blanc.

LC-MS : $t_R$ = 2.37min (méthode c) ; m/z : $[M+H]^+$ = 300, $[M-H]^-$ = 298
RMN $^1H$ (DMSO-$d_6$, 300MHz) : $\delta$ ppm = 8.86 (t, 1H, $^3J$ = 5.54Hz, N**H**); 8.09 (d, 2H, $^3J$ = 8.46Hz, 8+9); 7.99 (d, 2H, $^3J$ = 8.55Hz, 6+7); 3.52 (q, 2H, $^3J$ = 6.42Hz, 3); 2.68-2.50 (m, 5H, 13+2)
RMN $^{13}C$ (DMSO-$d_6$, 75MHz) : $\delta$ ppm = 177.8 (12); 167.1 (11); 165.6 (4); 136.6 (5); 128.8 (10); 128.1 (6+7); 127.0 (8+9); 126.9 (q, $^1J_{C-F}$ = 277.12Hz, 1); 32.9 (3); 32.4 (q, $^2J_{C-F}$ = 27.1 Hz, 2)

**Composé 76 : 4-(3,3,3-trifluoropropylcarbamoyl)benzoate de méthyle**

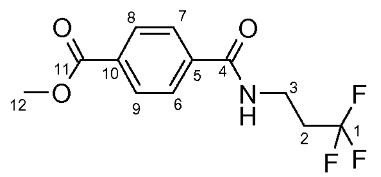

1,66g (1 eq.) de chlorhydrate de 3,3,3-trifluoropropylamine sont ajoutés à une solution de 2g (1eq.) de monométhyltéréphtalate, 5.04g (1.2eq.) de HBTU, 0.3g (0.2eq.) d'HOBt et 7.7mL (4eq.) de DIEA dans 45.5mL de DMF sur tamis moléculaire. Le milieu réactionnel est agité à TA pendant 1 h. Le DMF est alors évaporé sous pression réduite. Le résidu est repris dans l'acétate d'éthyle puis lavé avec une solution saturée en $NaHCO_3$ (2x), une solution d'acide chlorhydrique 1N (2x) à l'eau (1x) et à la saumure (1x). La phase organique est alors séchée sur sulfate de magnésium puis évaporé sous pression réduite. Le résidu obtenu est purifié sur colonne de silice prépaquée (Cyclohexane 9:1 AcOEt → Cyclohexane 85:15 AcOEt) pour donner 1.25g (41%) de poudre blanche. Les fractions impures sont de nouveau purifiées sur colonne de silice prépaquée (Cyclohexane 9:1 AcOEt) pour donner 1.27g (41%) de poudre blanche. Cela conduit à un rendement de 82%.

LCMS : $t_R$ = 2.44min (méthode c) ; m/z : $[M-H]^-$ = 274
RMN$^1H$ ($CD_2Cl_2$, 300MHz) : $\delta$ ppm : 8.10 (d, 2H, $^3J$ = 8.63Hz, 8+9 ou 6+7); 7.83 (d, 2H, $^3J$ = 8.55Hz, 7+6 ou 8+9);

6.60 (br s, 1 H, **N**H); 3.95 (s, 3H, 12); 3.74 (q, 2H, $^3J$ = 6.45Hz, 3); 2.53 (qt, 2H, $^3J_{2-1}$ = 10.93Hz, $^3J_{2-3}$ = 6.63Hz, 2)

**Composé 77 : acide 4-(3,3,3-trifluoropropylcarbamoyl)benzoïque**

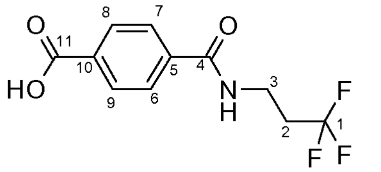

300mg (1 eq.) de 4-(3,3,3-trifluoropropylcarbamoyl)benzoate de méthyle (**76**) sont ajoutés à une solution de 244.6mg (4eq.) d'hydroxyde de potassium dans 2.66mL de MeOH. Le mélange est agité au reflux pendant 2h. Le milieu réactionnel est ensuite évaporé sous pression réduite. Le solide blanc obtenu est repris dans un mélange Eau : AcOEt. La phase aqueuse est lavée à l'acétate d'éthyle puis acidifiée à l'aide d'une solution aqueuse d'acide chlorhydrique 1 N jusqu'à pH=2. Le précipité blanc formé est alors solubilisé dans l'acétate d'éthyle. La phase organique est lavée à l'eau (2x) puis à la saumure (1x), séchée sur sulfate de magnésium puis concentrée sous pression réduite pour donner 254mg (89%) de poudre blanche.

LC-MS : $t_R$ = 4.28min (méthode d) ; m/z : $[M-H]^-$ = 260
RMN $^1H$ (DMSO-$d_6$, 300MHz) : $\delta$ ppm = 13.21 (br s, 1 H, COO**H**); 8.84 (t, 1H, $^3J$ = 5.56Hz, NH); 8.02 (d, 2H, $^3J$=8.25Hz, 6+7 ou 8+9); 7.91 (d, 2H, $^3J$=8.25Hz, 6+7 ou 8+9); 3.50 (q, 2H, $^3J$ = 6.30Hz, 3); 2.55 (qt, 2H, $^3J_{2-1}$ = 11.44Hz, $^3J_{2-3}$ = 6.96Hz, 2)

**Composé 78 : 4-(3-méthyl-1,2,4-oxadiazol-5-yl)-N-(3,3,3-trifluoropropyl)benzamide**

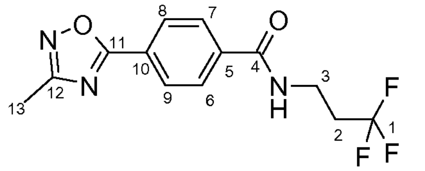

100mg (1eq.) d'acide 4-(3,3,3-trifluoropropylcarbamoyl)benzoïque (**77**) sont ajoutés à une solution de 28.4mg (1eq.) d'acétamidoxime, 570µL (2.5eq.) de solution de T3P à 50% dans l'acétate d'éthyle, 199µL (3eq.) de DIEA dans 191µL d'acétate d'éthyle. Le mélange est agité au reflux pendant 12h. L'analyse CCM du milieu révèle une conversion incomplète. 228µL (1 eq.) de solution de T3P et 66µL (1 eq.) de DIEA sont alors ajoutés. La réaction est agitée 24h supplémentaires au reflux. 200µL d'eau sont ajoutés au milieu réactionnel à TA et la solution est agitée pendant 10min à TA. Le produit est purifié par HPLC préparative pour donner 88mg (77%) de poudre blanche.

LC-MS : $t_R$ = 3.00min (méthode c) ; m/z : $[M-H]^-$ = 298
RMN $^1H$ ($CD_2Cl_2$, 300MHz) : $\delta$ ppm = 8.21 (d, 2H, $^3J$ = 8.42Hz, 8+9); 7.91 (d, 2H, $^3J$ = 8.42Hz, 6+7); 6.54 (br s, H, N**H**); 3.75 (q, 2H, $^3J$ = 6.41 Hz, 3); 2.61-2.46 (m, 5H, 13+2) RMN $^{13}C$ ($CD_2Cl_2$, 75MHz) : $\delta$ ppm = 174.8 (4); 168.5 (12); 166.6 (4); 138.1 (5); 128.6 (8+9); 128.0 (6+7); 127.4 (10); 127.0 (q, $^1J_{C-F}$ = 276.8Hz, 1); 34.0 (3); 33.9 (q, $^2J_{C-F}$ = 27.6Hz, 2); 11.9 (13)

**Composé 80 : 4-(5-méthyl-1,3,4-oxadiazol-2-yl)-N-(3,3,3-trifluoropropyl)benzamide**

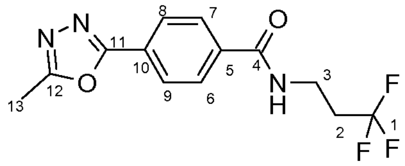

100mg (1 eq.) d'acide 4-(3,3,3-trifluoropropylcarbamoyl)benzoïque **(77)** sont ajoutés à une solution de 31.2mg (1.1eq.) d'acétylhydrazine, 570μL (2.5eq.) de solution de T3P à 50% dans l'acétate d'éthyle et 199μL (3eq.) de DIEA dans 196μL d'acétate d'éthyle. La solution est agitée pendant 24h au reflux. L'analyse LCMS du milieu réactionnel montre la formation de 50% du produit souhaité et de 50% de l'intermédiaire non cyclisé. 66μL (1eq.) de DIEA et 228μL de solution de T3P à 50% dans l'acétate d'éthyle sont alors ajoutés et la solution est agitée pendant 48h au reflux. La solution est laissée revenir à TA puis 1mL d'eau est ajouté et le mélange est agité pendant 15min à TA. La phase organique est alors lavée à l'eau (2x) et à la saumure (1x), séchée sur sulfate de magnésium et évaporée sous pression réduite pour donner 102mg (90%) de poudre blanche.

LC-MS : $t_R$ = 4.68min (méthode d) ; m/z : $[M-H]^-$ = 298
RMN $^1H$ ($CD_2Cl_2$, 300MHz) : δ ppm = 8.07 (d, 2H, $^3J$ = 8.44Hz, 8+9); 7.88 (d, 2H, $^3J$ = 8.44Hz, 6+7); 6.73 (br s, 1H, N**H**); 3.72 (q, 2H, $^3J$ = 6.46Hz, 3); 2.60 (s, 3H, 13); 2.50 (qt, 2H, $^3J_{2-1}$ = 10.85Hz, $^3J_{2-3}$ = 6.64Hz, 2)
RMN $^{13}C$ ($CD_2Cl_2$, 75MHz) : δ ppm = 166.8 (4); 164.7 (12); 164.5 (11); 137.2 (5); 128.1 (6+7); 127.2 (10); 127.2 (8+9); 127.0 (q, $^1J_{C-F}$ = 275.9Hz, 1); 34.1 (q, $^3J_{C-F}$ = 2.8Hz, 3); 33.9 (q, $^2J_{C-F}$ = 27.7Hz, 2); 11.3 (13)

**Composé 81 : 4-(5-méthyl-1,3,4-thiadiazol-2-yl)benzonitrile**

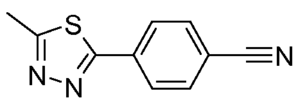

482mg (1.5eq.) de réactif de Lawesson sont ajoutés à TA à une solution de 100mg (1eq.) d'acide 4-cyanobenzoïque, 51 mg (1 eq.) d'acétylhydrazine, 606μL (1.5eq.) de solution de T3P à 50% dans l'acétate d'éthyle et 295μL (2.5eq.) de DIEA dans 1.5mL d'acétate d'éthyle. La solution est alors chauffée pendant une nuit au reflux. Le milieu réactionnel est refroidi à TA puis 5mL d'eau sont ajoutés. La solution est agitée pendant 15min à TA. La phase organique est lavée avec une solution aqueuse saturée en $NaHCO_3$ (2x) puis à la saumure (1x), séchée sur sulfate de magnésium et évaporée sous pression réduite. L'huile jaune obtenue est utilisée tel quel dans la réaction suivante.

LC-MS : $t_R$ = 2.10min (méthode c) ; m/z : $[M+H]^+$ = 202

**Composé 82 : acide 4-(5-méthyl-1,3,4-thiadiazol-2-yl)benzoïque**

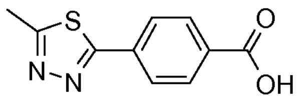

137mg (1eq.) de 4-(5-méthyl-1,3,4-thiadiazol-2-yl)benzonitrile **(81)** sont ajoutés à 5mL d'une solution aqueuse de soude à 10%. Le mélange est agité pendant 1h au reflux puis refroidi à TA et enfin lavé à l'acétate d'éthyle. La phase aqueuse est alors acidifiée à l'aide d'une solution d'HCl 1 N jusqu'à pH=2 puis extraite à l'acétate d'éthyle. Les phases organiques sont rassemblées, séchées sur sulfate de magnésium puis évaporées sous pression réduite. Le produit est utilisé tel quel dans l'étape suivante.

LC-MS : $t_R$ = 1.65min (méthode c) ; m/z : $[M-H]^-$ = 219

**Composé 83 : 4-(5-méthyl-1,3,4-thiadiazol-2-yl)-N-(3,3,3-trifluoropropyl)benzamide**

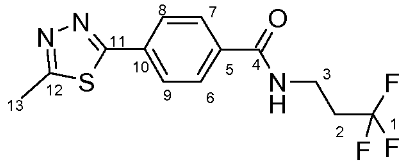

101 mg (1 eq.) de chlorhydrate de 3,3,3-trifluoropropylamine sont ajoutés à une solution de 150mg (1eq.) d'acide 4-(5-methyl-1,3,4-thiadiazol-2-yl)benzoïque **(82),** 485μL (1.2eq.) de solution de T3P à 50% dans l'acétate d'éthyle et 353μL (3eq.) de DIEA dans 3mL d'acétate d'éthyle. La solution est agitée pendant une nuit à TA. 81μL (0.2eq.) de solution de T3P sont ajoutés et le mélange est agité pendant 4h à TA. 2mL d'eau sont ajoutés et le milieu réactionnel est agité pendant 15min à TA. La phase organique est lavée à l'eau (2x) puis à la saumure (1x), séchée sur sulfate de magnésium puis évaporée sous pression réduite. Le résidu obtenu est purifié sur colonne de silice prépaquée (DCM → DCM 99:1 MeOH → DCM 98:2 MeOH) pour donner 22mg (10%) de poudre blanche.

LC-MS : $t_R$ = 2.17min (méthode c) ; m/z : $[M+H]^+$ = 316, $[M-H]^-$ = 314
RMN $^1H$ ($CD_2Cl_2$, 300MHz) : δ ppm = 8.04 (d, 2H, $^3J$ = 8.71 Hz, 8+9); 7.95 (d, 2H, $^3J$ = 8.71Hz, 6+7); 3.65 (t, 2H, $^3J$ = 6.99Hz, 3); 2.83 (s, 3H, 13); 2.55 (qt, $^3J_{2-1}$ = 10.88Hz, $^3J_{2-3}$ = 7.10Hz, 2)
RMN $^{13}C$ ($CD_2Cl_2$, 300MHz) : δ ppm = 169.7 (11); 169.1 (4); 168.4 (12); 137.7 (5 ou 10); 134.0 (5 ou 10); 129.3 (6+7); 128.9 (8+9); 128.0 (q, $^1J_{C-F}$ = 276.3Hz, 1); 34.5 (3); 34.0 (q, $^2J_{C-f}$ = 27.8Hz, 2)

**Composé 84 : N1-(3,3,3-trifluoropropyl)téréphthalamide**

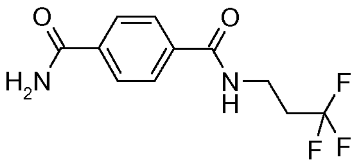

2mL de solution 7N d'ammoniaque dans le méthanol sont ajoutés à une solution de 200mg de 4-(3,3,3-trifluoropropylcarbamoyl)benzoate de méthyle **(76)** dans 2mL de méthanol. La solution est agitée pendant 36h au reflux. Les analyses LCMS et CCM du milieu réactionnel ne montrent quasiment aucune conversion de l'ester de départ. 500μL d'eau et 163mg (4eq.) de KOH sont alors ajoutés et le milieu réactionnel est agité pendant une nuit à reflux. La solution est acidifiée jusqu'à pH=4 à l'aide d'une solution d'acide chlorhydrique 1 N. Le précipité blanc formé est filtré et séché une nuit au dessiccateur (148mg).

Le produit est ensuite solubilisé dans 10mL de mélange DCE : $SOCl_2$ (1:1). La solution est agitée pendant 4h à 50°C. La solution est concentrée sous pression réduite puis reprise à 0°C dans 15mL de mélange $CH_3CN$ : $NH_4OH$ 28% (2:1). La solution est agitée à TA une nuit. Le milieu réactionnel est extrait à l'acétate d'éthyle. Les phases organiques sont regroupées puis lavées à l'eau (2x) et à la saumure (1x), séchées sur sulfate de magnésium et évaporées sous pression réduite pour donner 115mg (61%) de solide blanc.

LC-MS : $t_R$ = 1.77min (méthode c) ; m/z : $[M-H]^-$ = 259

**Composé 85 : 4-(2-oxo-1,3,4-oxathiazol-5-yl)-N-(3,3,3-trifluoropropyl)benzamide**

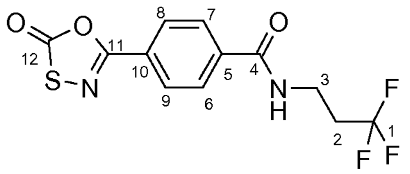

75μL (2eq.) de chlorure de (chlorothio)formyle sont ajoutés à une solution de 115mg (1eq.) de N1-(3,3,3-trifluoropropyl)téréphthalamide (**84**) dans 1,1mL de toluène. Le milieu réactionnel est agité pendant 24h à 90°C. 38μL (1eq.) de chlorure de (chlorothio)formyle sont alors ajoutés et la réaction est agitée à 90°C pendant 24h supplémentaires. Le toluène est évaporé sous pression réduite. Le résidu est repris dans du dichlorométhane puis filtré pour donner 95mg (68%) de solide blanc.

LC-MS : $t_R$ = 2.57min (méthode c) ; m/z : $[M-H]^-$ = 317
RMN $^1H$ (DMSO-$d_6$, 300MHz) : δ ppm = 8.90 (t, 1H, $^3J$ = 5.67Hz, N**H**); 8.05-8.00 (m, 4H, 6+7+8+9); 3.52 (q, 2H, $^3J$ = 6.44Hz, 3); 2.57 (qt, 2H, $^3J_{2-1}$ = 11.34Hz, $^3J_{2-3}$ = 6.70Hz, 2)

**Composé 89 : 3-(4-(3,3,3-trifluoropropylcarbamoyl)phényl)-1,2,4-thiadiazole-5-carboxylate d'éthyle**

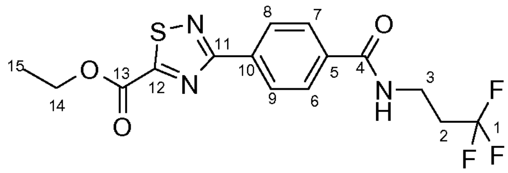

383mL (10eq.) de cyanoformiate d'éthyle sont ajoutés à une solution de 100mg (1 eq.) de 4-(2-oxo-1,3,4-oxathiazol-5-yl)-N-(3,3,3-trifluoropropyl)benzamide **(85)** dans 400μL de 1,2-dichlorobenzène. Le mélange est agité pendant 30min à 160°C dans un réacteur micro-ondes. La solution est refroidie à TA puis 6mL d'acétonitrile sont alors ajoutés. Le précipité formé est filtré pour donner 302mg (85%) de poudre blanche.

LC-MS : $t_R$ = 2.77min (méthode c) ; m/z : $[M+H]^+$ = 374
RMN $^1H$ ($CD_3OD$, 300MHz) : δ ppm = 8.44 (d, 2H, $^3J$ = 8.56Hz, 8+9); 7.97 (d, 2H, $^3J$ = 8.56Hz, 6+7); 4.54 (q, 2H, $^3J$ = 7.16Hz, 14); 3.66 (t, 2H, $^3J$ = 7.04Hz, 3); 2.55 (qt, 2H, $^3J_{2-1}$ = 10.83Hz, $^3J_{2-3}$ = 7.04Hz, 2); 1.47 (t, 3H, $^3J$ = 7.13Hz, 15)

**Composé 90 : 4-(5-(hydroxyméthyl)-1,2,4-thiadiazol-3-yl)-N-(3,3,3-trifluoropropyl)-benzamide**

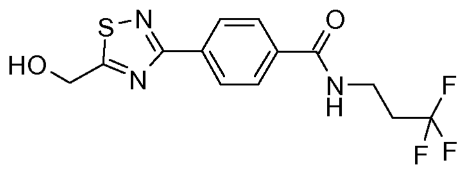

550μL d'une solution 2M de $LiBH_4$ dans le THF sont ajoutés à 0°C et sous argon à une solution de 140 mg de 3-(4-(3,3,3-trifluoropropylcarbamoyl)phényl)-1,2,4-thiadiazole-5-carboxylate d'éthyle (**89**) dans 3.5mL de THF anhydre. La solution est agitée pendant 30min à 0°C. 0.5mL d'une solution aqueuse saturée de $NH_4Cl$ sont alors ajoutés pour neutraliser l'excès d'hydrure. Le milieu est concentré sous pression réduite. Le résidu obtenu est alors purifié sur colonne de silice prépaquée (Toluène 97:3 MeOH Toluène 95:5 MeOH) pour donner 108mg (44%) de poudre blanche.

LC-MS : $t_R$ = 2.18min (méthode c) ; m/z : $[M+H]^+$ = 332, $[M-H]^-$ = 330

**Composé 92 : 4-azido-N-(3,3,3-trifluoropropyl)benzamide**

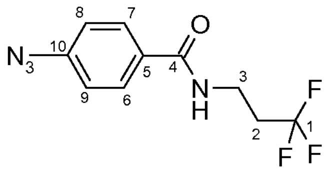

1.05g (1 eq.) de chlorhydrate de 3,3,3-trifluoropropylamine sont ajoutés à une solution de 1.14g (1eq .) d'acide 4-azidobenzoïque, 536mg (0.5eq) d'HOBt 3.19g (1.2eq) d'HBTU et 4.85mL (4eq.) de DIEA dans 5mL de DMF. La solution est agitée une nuit à TA puis évaporée sous pression réduite. Le résidu est dissous dans l'acétate d'éthyle et lavé avec une solution aqueuse saturée en $NaHCO_3$ (2x), une solution aqueuse d'HCl 1N (1x) et à la saumure (1x). La phase organique est séchée sur sulfate de magnésium puis évaporée sous pression réduite pour donner 1.77g (98%) d'un solide marron clair.

LC-MS : $t_R$ = 2.65min (méthode c) ; m/z : $[M-H]^-$ = 257
RMN $^1H$ ($CD_3OD$, 300MHz) : $\delta$ ppm = 7.86 (d, 2H, $^3J$ = 8.91Hz, 6+7 ou 8+9); 7.15 (d, 2H, $^3J$ = 8.92Hz, 6+7 ou 8+9); 3.61 (t, 2H, $^3J$ = 6.97Hz, 3); 2.51 (qt, $^3J_{2-1}$ = 10.99Hz, $^3J_{2-3}$ = 7.05Hz, 2)

**Procédure (ii) : procédure générale de formation des cycles triazoles par "click chemistry"**

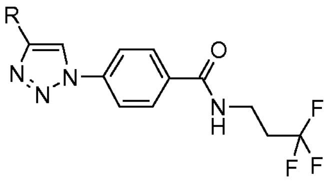

1eq. d'alcyne est ajouté à une solution de 115mg (1eq.) de 4-azido-N-(3,3,3-trifluoropropyl)benzamide (**92**), 8mg (0.1eq.) de $CuSO_4$, 99.1mg (1eq.) d'ascorbate de sodium dans 6mL d'un mélange t-BuOH/$H_2O$ (1/1). La solution est agitée une nuit à TA.

**Composé 93 : 4-(4-isopentyl-1H-1,2,3-triazol-1-yl)-N-(3,3,3-trifluoropropyl)benzamide**

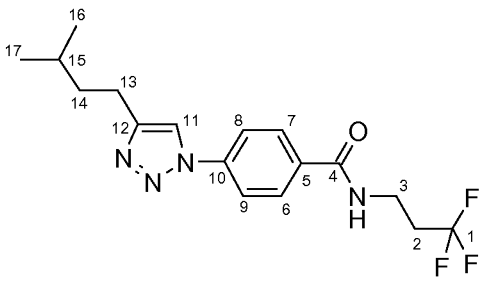

Procédure (ii) avec 65.7µL de 5-méthylhexyne. Le milieu est filtré après réaction. Le produit encore présent dans la phase aqueuse est extrait à l'acétate d'éthyle. Le produit est ensuite séché au dessiccateur pour donner 93mg (53%) de solide beige.

LC-MS : $t_R$ = 2.93min (méthode c) ; m/z : $[M+H]^+$ = 355
RMN $^1H$ (DMSO-$d_6$, 300MHz) : $\delta$ ppm = 8.84 (br s, 1 H, N**H**); 8.69 (s, 1 H, 11); 8.03 (br m, 4H, 6+7+8+9); 3.53 (q, 2H, $^3J$ = 6.34Hz, 3); 2.73 (t, 2H, $^3J$ = 7.42Hz, 13); 2.58 (qt, $^3J_{2-1}$ = 11.40Hz, $^3J_{2-3}$ = 7.11 Hz, 2); 1.67-1.54 (m, 3H,

14+15); 0.94 (d, 6H, $^3J$ = 6.13Hz, 16+17)

**Composé 94 : 4-(4-isobutyl-1H-1,2,3-triazol-1-yl)-N-(3,3,3-trifluoropropyl)benzamide**

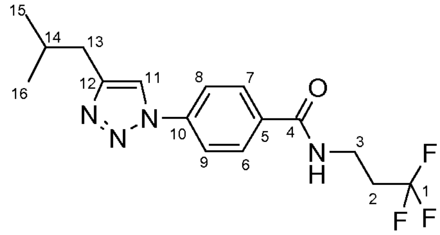

Procédure (ii) avec 58.9µL de 4-méthylpentyne. Après une nuit, la conversion n'est pas totale. 1eq. d'alcyne, 0.5eq. d'ascorbate de sodium et 0.05eq. de $CuSO_4$ sont ajoutés au milieu réactionnel. La solution est agitée pendant 4h à TA. Le produit est extrait à l'acétate d'éthyle. Les phases organiques sont rassemblées, séchées sur sulfate de magnésium puis évaporées sous pression réduite pour donner 128mg (75%) d'une poudre marron clair.

LC-MS : $t_R$ = 2.77min (méthode c) ; m/z : $[M+H]^+$ = 341
RMN $^1H$ (DMSO-$d_6$, 300MHz) : δ ppm = 8.42 (s, 1 H, 11); 8.05-7.97 (m, 4H, 6+7+8+9); 3.65 (t, 2H, $^3J$ = 6.98Hz, 3); 2.67 (d, 2H, $^3J$ = 6.85Hz, 13); 2.55 (qt, $^3J_{2-1}$ = 10.93Hz, $^3J_{2-3}$ = 6.93Hz, 2); 2.03 (n, 1H, $^3J$ = 6.75Hz, 14); 0.99 (d, 6H, $^3J$ = 6.63Hz, 15+16)

**Composé 95 : 4-(4-cyclopropyl-1H-1,2,3-triazol-1-yl)-N-(3,3,3-trifluoropropyl)-benzamide**

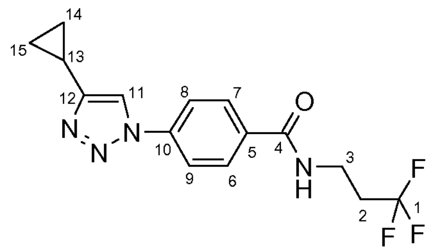

Procédure (ii) avec 42.4µL d'éthynylcyclopropane. Une partie du produit est récupérée par filtration du précipité formé. Le produit restant dans le filtrat est extrait à l'acétate d'éthyle. Les phases organiques sont rassemblées, séchées sur sulfate de magnésium puis évaporées sous pression réduite pour donner 73mg (45%) d'une poudre blanche.

LC-MS : $t_R$ = 2.52min (méthode c) ; m/z : $[M+H]^+$ = 325
RMN $^1H$ (DMSO-$d_6$, 300MHz) : δ ppm = 8.84 (br s, 1 H, N**H**); 8.64 (s, 1 H, 11); 8.05-7.97 (m, 4H, 6+7+8+9); 3.53 (q, 2H, $^3J$ = 6.44Hz, 3); 2.58 (qt, 2H, $^3J_{2-1}$ = 11.51Hz, $^3J_{2-3}$ = 6.95Hz, 2); 2.09-2.00 (m, 1 H, 13); 1.02-0.96+0.84-0.79 (m, 4H, 14+15)

**Composé 96 : 4-(4-(hydroxyméthyl)-1H-1,2,3-triazol-1-yl)-N-(3,3,3-trifluoropropyl)-benzamide**

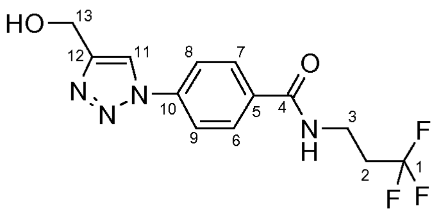

Procédure (ii) avec 29.2µL d'alcool propargylique. Le produit est extrait à l'acétate d'éthyle. Les phases organiques sont regroupées, séchées sur sulfate de magnésium puis évaporées sous pression réduite pour donner 52mg (33%) d'une poudre beige.

LC-MS : $t_R$ = 2.03min (méthode c) ; m/z : $[M+H]^+$ = 315
RMN $^1H$ ($CD_3OD$, 300MHz) : δ ppm = 8.57 (s, 1 H, 11); 8.07-7.99 (m, 4H, 6+7+8+9); 4.79 (s, 2H, 13); 3.67 (t, 2H, $^3J$ = 6.97Hz, 3); 2.57 (qt, 2H, $^3J_{2-1}$ = 10.91Hz, $^3J_{2-3}$ = 6.98Hz, 2) RMN $^{13}C$ ($CD_3OD$, 75MHz) : δ ppm = 168.9 (4); 150.3 (12); 140.6 (5 ou 10); 135.5 (5 ou 10); 130.1 (6+7); 128.0 (q, $^1J_{C-F}$ = 276.1 Hz, 1); 122.3 (11); 121.2 (8+9); 56.4 (13); 34.6 (3); 34.0 (q, $^2J_{C-F}$ = 27.7 Hz, 2)

**Composé 97 : 4-(4-(2-hydroxyéthyl)-1H-1,2,3-triazol-1-yl)-N-(3,3,3-trifluoropropyl)-benzamide**

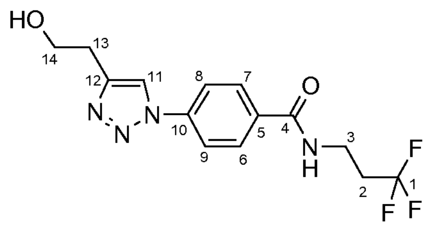

Procédure (ii) avec 37.9µL de 3-butyn-1-ol. Aucun précipité n'est observé. Le produit est extrait à l'acétate d'éthyle. Les phases organiques sont rassemblées, séchées sur sulfate de magnésium puis évaporées sous pression réduite pour donner 135mg (82%) de solide beige.

LC-MS : $t_R$ = 2.07min (méthode c) ; m/z : $[M+H]^+$ = 329
RMN $^1H$ ($CD_3OD$, 300MHz) : δ ppm = 8.44 (s, 1 H, 11); 8.04-7.96 (m, 4H, 6+7+8+9); 3.89 (t, 2H, $^3J$ = 6.62Hz, 14); 3.65 (t, 2H, $^3J$ = 6.98Hz, 3); 3.00 (t, 2H, $^3J$ = 6.51 Hz, 13); 2.44 (qt, 2H, $^3J_{2-1}$ = 10.85Hz, $^3J_{2-3}$ = 7.09Hz, 2)
RMN $^{13}C$ ($CD_3OD$, 75MHz) : δ ppm = 168.9 (4); 147.3 (12); 140.7 (5 ou 10); 135.4 (5 ou 10); 130.1 (6+7); 128.0 (q, $^1J_{C-F}$ = 275.8Hz, 1); 122.2 (11); 121.1 (8+9); 61.9 (14); 34.6 (3); 34.0 (q, $^2J_{C-F}$ = 28.1 Hz, 2); 29.9 (13)

**Composé 98 : 4-(4-(2-hydroxypropan-2-yl)-1H-1,2,3-triazol-1-yl)-N-(3,3,3-trifluoro-propyl)benzamide**

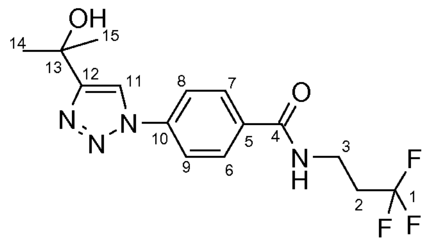

Procédure (ii) avec 48.9µL de 2-méthyl-3-butyn-2ol. Une partie du produit est récupérée par filtration du précipité formé. Le produit restant dans le filtrat est extrait à l'acétate d'éthyle. Les phases organiques sont rassemblées, séchées sur sulfate de magnésium puis évaporées sous pression réduite. Le produit est séché au dessiccateur pour donner 80mg (53%) de poudre blanche.

LC-MS : $t_R$ = 2.18min (méthode c) ; m/z : $[M-H]^-$ = 341
RMN $^1H$ (DMSO-$d_6$, 300MHz) : δ ppm = 8.84 (t, 1H, $^3J$ = 5.18Hz, N**H**); 8.71 (s, 1 H, 11); 8.08-8.01 (m, 4H, 6+7+8+9); 5.29 (s, 1 H, O**H**); 3.53 (q, 2H, $^3J$ = 6.40Hz, 3); 2.58 (qt, 2H, $^3J_{2-1}$ = 11.53Hz, $^3J_{2-3}$ = 6.72Hz, 2)

**Composé 99 : 1-(4-(3,3,3-trifluoropropylcarbamoyl)phényl)-1H-1,2,3-triazole-4-carboxylate de méthyle**

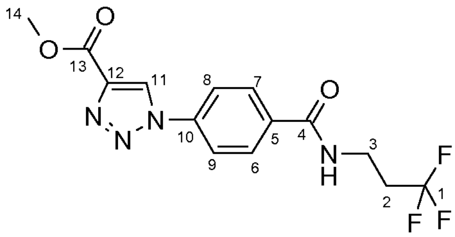

Procédure (ii) avec 41.4μL de propiolate de méthyle. Après une nuit, la conversion n'est pas totale. 0.5eq. d'alcyne, 0.5eq. d'ascorbate de sodium et 0.05eq. de $CuSO_4$ sont ajoutés au milieu réactionnel. La solution est agitée pendant 4h à TA. Le produit est extrait à l'acétate d'éthyle. Les phases organiques sont rassemblées, séchées sur sulfate de magnésium puis évaporées sous pression réduite pour donner 156mg (91%) d'une poudre beige.

LC-MS : $t_R$ = 2.37min (méthode c) ; m/z : $[M+H]^+$ = 343
RMN $^1H$ (DMSO-$d_6$, 300MHz) : δ ppm = 9.62 (s, 1 H, 11); 8.89 (t, 1H, $^3J$ = 5.56Hz, N**H**); 8.14 (d, 2H, $^3J$ = 8.88Hz, 8+9); 8.06 (d, 2H, $^3J$ = 8.88Hz, 6+7); 3.91 (s, 3H, 14); 3.54 (q, $^3J$ = 6.46Hz, 3); 2.59 (qt, 2H, $^3J_{2-1}$ = 11.44Hz, $^3J_{2-3}$ = 7.06Hz, 2)
RMN $^{13}C$ (DMSO-$d_6$, 75MHz) : δ ppm = 165.1 (13); 160.5 (4); 139.7 (12); 138.0 (5 ou 10); 134.5 (5 ou 10); 128.9 (6+7); 127.5 (11); 126.9 (q, $^1J_{C-F}$ = 277.1Hz, 1); 120.2 (8+9); 52.1 (14); 32.9 (3); 32.4 (q, $^2J_{C-F}$ = 26.6Hz, 2)

**Composé 100 : chlorhydrate de 4-(3,3,3-trifluoropropylcarbamoyl)benzimidate de méthyle**

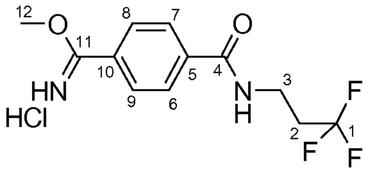

15mL d'une solution d'acide chlorhydrique 4N dans le dioxane sont ajoutés sous argon à 0°C à une solution de 300mg de 4-cyano-N-(3,3,3-trifluoropropyl)benzamide **(73)** dans 1 mL de MeOH. Le mélange est agité pendant une nuit à TA. 20mL d'éther diéthylique sont ajoutés et la solution est refroidie au réfrigérateur pendant une heure. Le précipité est alors filtré, lavé à l'éther diéthylique et séché au dessiccateur pour donner 359mg (93%) de poudre blanche.

LC-MS : $t_R$ = 1.85min (méthode c) ; m/z : $[M+H]^+$ = 275, $[M-H]^-$ = 273

**Composé 101 : 4-(5-méthyl-4H-1,2,4-triazol-3-yl)-N-(3,3,3-trifluoropropyl)benzamide**

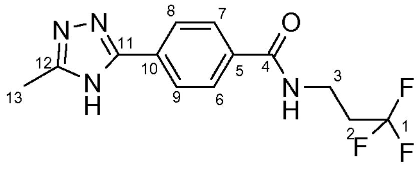

169μL (1.5eq.) de DIEA sont ajoutés à une solution de 200mg de chlorhydrate de 4-(3,3,3-trifluoropropylcarbamoyl)benzimidate de méthyle **(100)** dans 5mL d'éthanol absolu. Le milieu réactionnel est agité pendant 15min à TA. L'acétylhydrazine est alors ajoutée et la solution est agitée pendant 60h au reflux. L'éthanol est évaporé sous pression réduite. Le solide rose obtenu est purifié par HPLC préparative puis lyophilisé pour donner 123mg (64%) de poudre blanche.

LC-MS : $t_R$ = 1.92min (méthode c) ; m/z : $[M+H]^+$ = 299, $[M-H]^-$ =297
RMN $^1H$ ($CD_3OD$, 300MHz) : 8.08 (d, 2H, $^3J$ = 8.59Hz, 8+9); 7.90 (d, 2H, $^3J$ = 8.68Hz, 6+7); 3.64 (t, 2H, $^3J$ = 7.02Hz,

3); 2.62-2.46 (m, 5H, 13+2)

## Scheme 14:

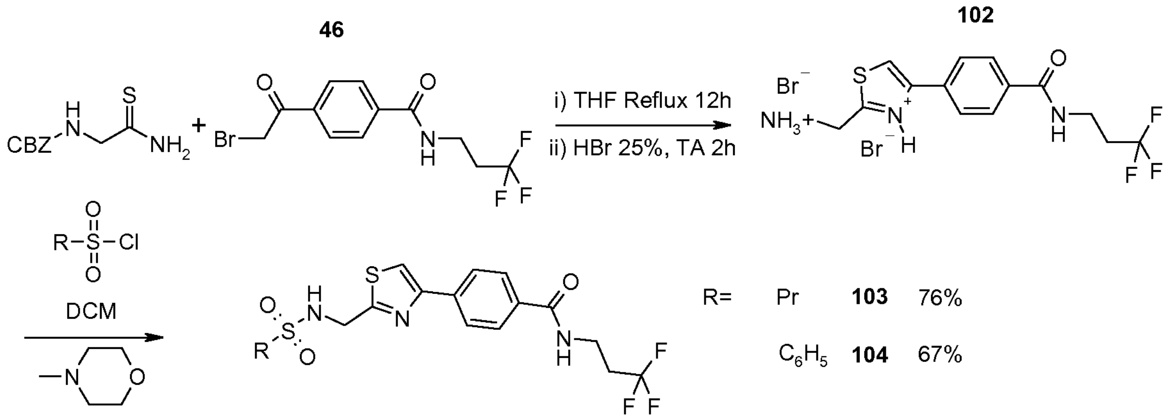


**Composé 102 : di-bromhydrate de (4-(4-(3,3,3-trifluoropropylcarbamoyl)phényl)-thiazol-2-yl)méthanaminium**

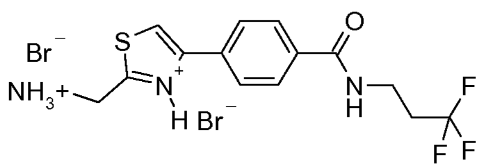

73mg (1.1eq.) de N-benzyloxycarbonylglycine thioamide sont ajoutés à une solution de 100mg (1eq.) de 4-(2-bromoacétyl)-N-(3,3,3-trifluoropropyl)benzamide **(46)** dans 5mL de THF anhydre. La solution est agitée pendant 3h à 70°C. Le THF est alors évaporé sous pression réduite et le solide obtenu est utilisé tel quel. 1 mL d'une solution d'HBr à 25% dans l'acide acétique est ajouté et le milieu réactionnel est agité pendant 1h à TA. 8mL d'éther diéthylique sont ensuite ajoutés à TA pour former un précipité qui est refroidi dans un bain d'eau glacée. Le précipité est alors filtré, lavé à l'éther diéthylique froid puis séché au dessiccateur pour donner 157mg de solide marron pâle. Le produit est séparé en deux pour la synthèse des composés **103** et **104.**

LC-MS : $t_R$ = 1.85min (méthode c) ; m/z : $[M+H]^+$ = 330

**Composé 103 : 4-(2-(propylsulfonamidométhyl)thiazol-4-yl)-N-(3,3,3-trifluoropropyl)-benzamide**

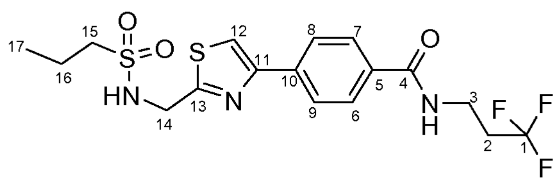

20μL (1.2eq.) de chlorure d'acide propansulfonique sont ajoutés à une solution de 75mg (1eq.) de composé **102** et 82μL de N-méthylmorpholine dans 1.5mL de DCM anhydre. Le milieu réactionnel est agité pendant 2h à TA. Le DCM est évaporé sous pression réduite puis le résidu est repris dans l'acétate d'éthyle puis lavé à l'eau (2x) et à la saumure (1x). La phase organique est séchée sur sulfate de magnésium puis évaporée sous pression réduite pour donner 54mg (76%) de poudre beige.

LC-MS : $t_R$ = 2.60min (méthode c) ; m/z : $[M-H]^-$ = 434
RMN $^1H$ ($CD_3OD$, 300MHz) : δ ppm : 8.05 (d, 2H, $^3J$ = 8.43Hz, 8+9); 7.96 (s, 1H, 12); 7.89 (d, 2H, $^3J$ = 8.49Hz,

6+7); 4.63 (s, 2H, 14); 3.66, (t, 2H, $^3J$ = 6.99Hz, 3); 3.15-3.09 (m, 2H, 15); 2.56 (qt, 2H, $^3J_{2-1}$ = 10.89Hz, $^3J_{2-3}$ = 7.11Hz, 2); 1.84 (sx, 2H, $^3J$ = 7.60Hz, 16); 1.04 (t, 3H, $^3J$ = 7.46Hz, 17).

**Composé 104 : 4-(2-(phénylsulfonamidométhyl)thiazol-4-yl)-N-(3,3,3-trifluoro-propyl)benzamide**

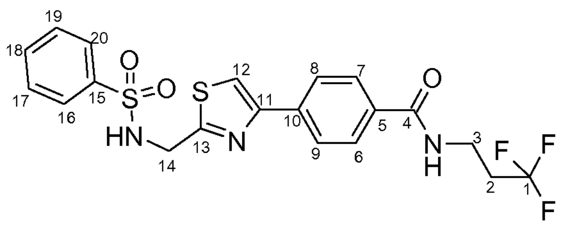

23μL (1.2eq.) de chlorure d'acide benzènesulfonique sont ajoutés à une solution de 75mg (1eq.) de composé **102** et 82μL de N-méthylmorpholine dans 1.5mL de DCM anhydre. Le milieu réactionnel est agité pendant 2h à TA. Le DCM est évaporé sous pression réduite puis le résidu est repris dans l'acétate d'éthyle puis lavé à l'eau (2x) et à la saumure (1x). La phase organique est séchée sur sulfate de magnésium puis évaporée sous pression réduite pour donner 51 mg (67%) de poudre beige.

LC-MS : $t_R$ = 2.73min (méthode c) ; m/z : $[M-H]^-$ = 468
RMN $^1H$ ($CD_3OD$, 300MHz) : δ ppm = 7.96 (d, $^3J$ = 8.50Hz, 8+9); 7.87 (m, 5H, 6+7+12+16+20); 5.57 (m, 3H, 17+18+19); 4.47 (s, 2H, 14); 3.65 (t, 2H, $^3J$ = 7.00Hz, 3); 2.55 (qt, 2H, $^3J_{2-1}$ = 11.0Hz, $^3J_{2-3}$ = 7.10Hz, 2)

## Scheme 15:

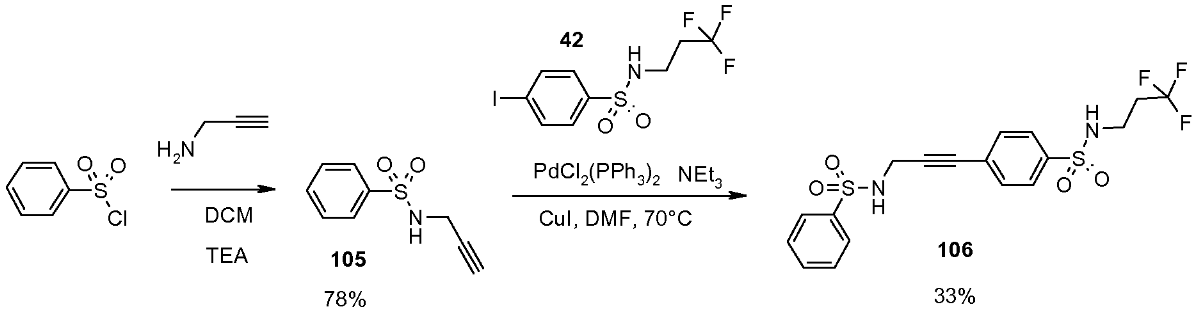


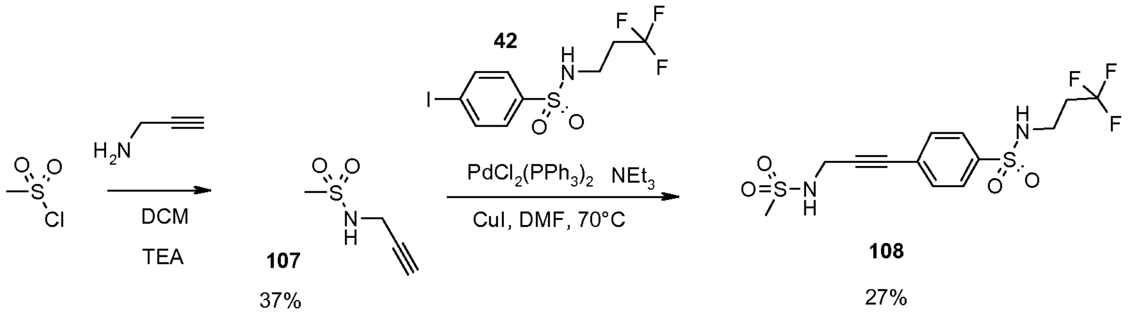


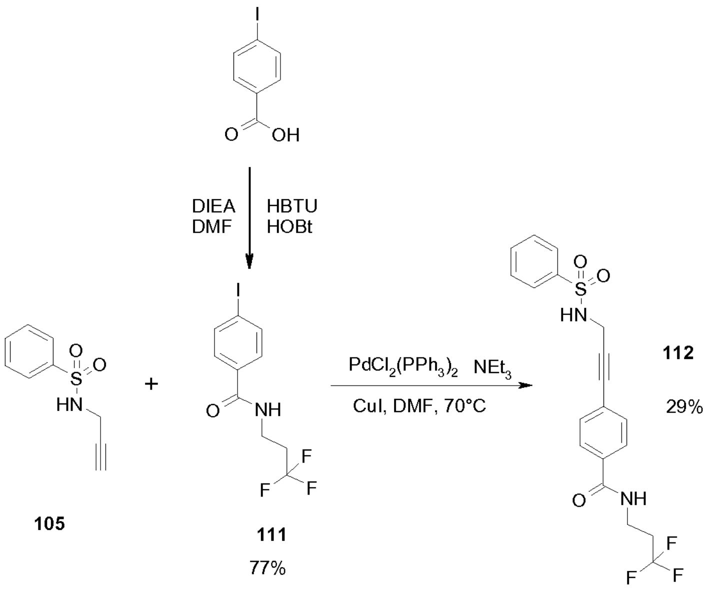


**Composé 105 : N-(prop-2-ynyl)benzènesulfonamide**

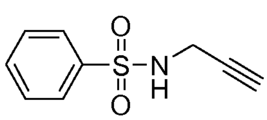

200µL (1 eq.) de chlorure d'acide benzènesulfonique, 129µL (1.2eq.) de propargylamine, 8mL de DCM et 1.08mL (4eq.) de DIEA sont agités à TA pendant 2h. Le DCM est évaporé sous pression réduite puis le résidu est repris dans l'acétate d'éthyle, lavé à l'eau (2x) et à la saumure (1x). La phase organique est séchée sur sulfate de magnésium puis évaporée sous pression réduite. Le résidu est purifié par HPLC préparative pour donner 238mg (78%) d'une huile transparente.

LC-MS : $t_R$ = 2.42min (méthode c) ; m/z : $[M-H]^-$ = 194
CCM : $R_f$ = 0.14 (AcOEt 2:8 Cyclohexane)

**Composé 106 : 4-(3-(phénylsulfonamido)prop-1-ynyl)-N-(3,3,3-trifluoropropyl)-benzènesulfonamide**

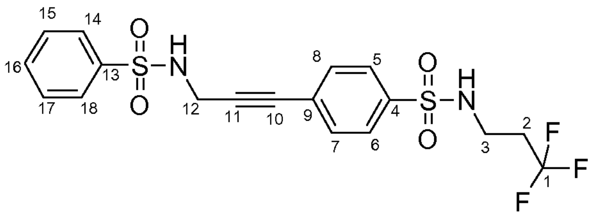

50mg (1eq.) de N-(prop-2-ynyl)benzènesulfonamide (**105**), 87mg (0.9eq.) de 4-iodo-N-(3,3,3-trifluoropropyl)benzènesulfonamide (**42**), 8.3mg (0.05eq.) de bis(triphényl-phosphine)palladium(II) chloride, 40.2mg (0.9eq.) d'iodure de cuivre, et 120μL (0.5mL/mmol) triéthylamine sont placés successivement dans un schlenk sous un flux d'Argon. Le DMF anhydre est ajouté *via* une canule sous Argon à température ambiante. Le mélange réactionnel est agité à 70°C sous Argon pendant 48h.

Le milieu réactionnel est alors dilué à TA par ajout d'un mélange AcOEt:$NH_4Cl_{sat}$. (1:1). Le précipité formé est filtré et lavé à l'acétate d'éthyle. La phase organique est lavée avec une solution aqueuse saturée en $NH_4Cl$ (2x) puis à la saumure (1x), séchée sur sulfate de magnésium, filtrée sur célite puis évaporée sous pression réduite. Le solide orange obtenu est solubilisé dans le minimum d'acétonitrile puis le produit est précipité par ajout d'éther diisoprpylique et filtré pour donner 38mg (33%) de poudre beige.

LC-MS : $t_R$ = 3.13min (méthode c) ; m/z : $[M+H]^+$ = 447
RMN $^1H$ ($CD_3OD$, 300MHz) : δ ppm = 7.92 (m, 2H, 14+18 ou 15+17); 7.74 (d, 2H, $^3J$ = 8.40Hz, 5+6); 7.50-7.57 (m, 3H,16+(14+18 ou 15+17)); 7.30 (d, 2H, $^3J$ = 8.40Hz, 7+8); 4.07 (s, 2H, 12); 3.08 (t, 2H, $^3J$ = 7.10Hz, 3); 2.36 (qt, 2H, $^3J_{2-1}$ = 10.60Hz, $^3J_{2-3}$ = 6.80Hz, 2)
RMN $^{13}C$ ($CD_3OD$, 75MHz) : δ ppm = 142.3 (4); 141.2 (13); 133.7 (16); 133.2 (7+8); 130.1 (14+18ou15+17); 128.4 (14+18ou15+17); 128.3 (9); 127.9 (5+6); 127.5 (q, $^1J$ = 276.7Hz, 1); 88.7 (11); 83.3 (10); 37.4 (3); 35.2 (q, $^2J$ = 28.2Hz, 2); 33.8 (12)

**Composé 107 : N-(prop-2-ynyl)méthanesulfonamide**

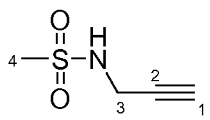

Une solution de 288μL (1 eq.) de chlorure d'acide méthanesulfonique dans 3mL d'éther diéthylique est ajoutée à une solution de 500μL (2eq.) de propargylamine dans 6mL d'éther diéthylique refroidie à 0°C. Le mélange est agité pendant 30min. Le précipité formé est filtré et lavé deux fois à l'éther. Le filtrat est évaporé sous pression réduite pour donner 178mg (37%) d'un solide blanc.

LC-MS : $t_R$ = 1.92 min (méthode c) ; m/z : $[M+H]^+$ = 134RMN $^1H$ ($CDCl_3$, 300MHz) : δ ppm = 4.69 (m, 1 H, N**H**) ; 4.00 (dd, 2H, $^3J_{3-NH}$ = 6.20Hz, $^4J_{3-1}$ = 2.50Hz, 2); 3.10 (s, 3H, 4); 2.41 (t, 1H, $^3J$ = 2.50Hz, 1)

**Composé 108 : 4-(3-(méthylsulfonamido)prop-1-ynyl)-N-(3,3,3-trifluoropropyl)-benzènesulfonamide**

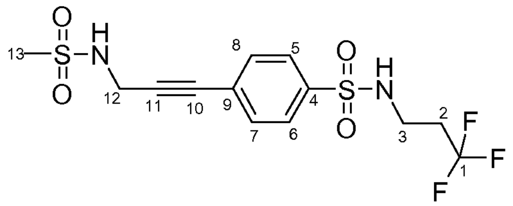

136mg (1.2eq.) d'acétate de tétrabutylammonium, 1 mg (0.002eq.) de Pd EnCat[30], 142mg (1 eq.) de **4**-iodo-N-(3,3,3-trifluoropropyl)benzènesulfonamide (**42**) et 50mg (1 eq.) de N-(prop-2-ynyl)méthanesulfonamide (**107**) sont ajoutés à 500μL de DMF. La solution est agitée pendant 24h à 80°C. Un mélange AcOEt:$NH_4Cl_{sat.}$ 1:1 (2mL) est ajouté au milieu réactionnel. Le mélange est agité 10min puis filtré pour récupérer les billes de palladium. Le produit est récupéré dans la phase organique par extraction Eau:AcOEt. La phase organique est alors lavée à la saumure (1x), séchée sur sulfate de magnésium, filtré sur célite et évaporée sous pression réduite. Le produit purifié sur colonne de silice prépaquée (Cyclohexane 95:5 Isopropanol → Cyclohexane 90:10 Isopropanol). On récupère 39mg (27%) d'un solide beige.

LC-MS : $t_R$ = 2.78min (méthode c) ; m/z : $[M+H]^+$ = 385
RMN $^1$H ($CD_3OD$, 300MHz) : δ ppm = 7.84 (d, 2H, $^3J$=8.59Hz, 5+6 ou 7+8); 7.64 (d, 2H, $^3J$=8.59Hz, 5+6 ou 7+8); 4.17 (s, 2H, 12); 3.12-3.08 (m, 5H, 13+3); 2.38 (qt, 2H, $^3J_{2-1}$=10.78Hz, $^3J_{2-3}$=7.28Hz, 2)

**Composé 111 : 4-iodo-N-(3,3,3-trifluoropropyl)benzamide**

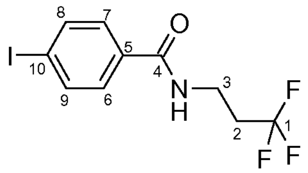

300mg (1eq.) d'acide 4-acétylbenzoïque, 181mg (1eq.) de chlorhydrate de 3,3,3-trifluoropropylamine, 33mg (0.2eq.) d'HOBT, 551mg (1.1eq.) d'HBTU et 10mL de DMF sont sucessivement ajoutés dans un ballon de 25mL. 840μL (4eq.) de DIEA sont ensuite ajoutés et le mélange est agité 2h à TA. Le DMF est évaporé sous pression réduite. Le résidu est repris dans l'acétate d'éthyle puis lavé à la saumure (2x). La phase organique est alors évaporée sous pression réduite puis le produit est purifié sur colonne de silice prépaquée (Cyclohexane 8:2 AcOEt) pour donner 327mg (77%) de poudre blanche.

LC-MS : $t_R$ = 3.25 min (méthode c) ; m/z : $[M+H]^+$ = 344RMN $^1$H ($CDCl_3$, 300MHz) : δ ppm = 7.80 (d, 2H, $^3J$ = 8.50Hz) ; 7.47 (d, $^3J$ = 8.50Hz); 6.34 (m, 1 H, N**H**); 3.72 (q, 2H, $^3J$ = 6.30Hz, 3); 2.47 (tq, 2H, $^3J_{2-1}$ = 10.70Hz, $^3J_{2-3}$ = 6.40Hz, 2)
RMN$^{19}$F ($CDCl_3$, 282.4MHz) : δppm = -64.92 (t, $^3J$ = 10.8Hz)

**Composé 112: 4-(3-(phénylsulfonamido)prop-1-ynyl)-N-(3,3,3-trifluoropropyl)-benzamide**

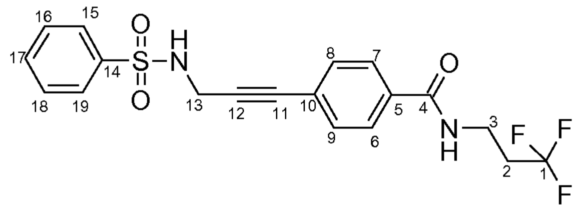

50mg (1 eq.) de N-(prop-2-ynyl)benzènesulfonamide (**105**), 83.5mg (0.95eq.) de 4-iodo-N-(3,3,3-trifluoropropyl)benzamide (**111**), 9mg (0.05eq.) de bis(triphénylphosphine)-palladium(II) chloride, 48mg (1eq.) d'iodure de cuivre, et 128μL (0.5mL/mmol) de triéthylamine sont placés successivement dans un Schlenk sous un flux d'argon. 1mL DMF

anhydre est ajouté *via* une canule sous argon à TA. Le mélange réactionnel est agité à 70°C sous Argon pendant 48h.

Le milieu réactionnel est dilué à TA par ajout d'un mélange 1:1 AcOEt:$NH_4Cl_{sat.}$. Le précipité alors formé est filtré et lavé à l'acétate d'éthyle. On effectue une extraction avec un mélange AcOEt:$NH_4Cl_{sat.}$. La phase organique est lavée à la saumure (1x), séchée sur sulfate de magnésium, filtrée sur célite puis évaporée sous pression réduite. L'huile marron obtenue est purifiée par colonne de silice prépaquée (DCM 95:5 AcOEt) pour donner 30mg (29%) d'un solide marron clair.

LC-MS : $t_R$ = 5.43min (méthode d) ; m/z : $[M+H]^+$ = 411
RMN $^1H$ ($CD_3OD$, 300MHz) : $\delta$ ppm = 7.94 (dd, 2H, $^3J$ = 7.40Hz, $^4J$ = 1.60Hz, 15+19); 7.71 (d, 2H, $^3J$ = 8.40Hz, 6+7 ou 8+9); 7.55 (m, 3H, 16+17+18); 7.21 (d, 2H, $^3J$ = 8.40Hz, 6+7 ou 8+9); 4.07 (s, 2H, 13); 3.62 (t, 2H, $^3J$ = 7.00Hz, 3); 2.52 (qt, 2H, $^3J_{2-1}$ = 10.90Hz, $^3J_{2-3}$ = 7.00Hz, 2);

***Evaluation** de **l'activité des composés***

**Le test de TSA (Thermal Shift Assay)**

La dénaturation thermique des protéines est un phénomène étudié depuis longtemps, au moyen de nombreuses techniques, basées notamment sur la calorimétrie (DSC : Differential Scanning Calorimetry) ou la fluorescence (DSF : Differential Scanning Fluorimetry ou encore Fluorescence Thermal Shift Assay).

Le test de Thermal Shift Assay utilise un marqueur fluorescent, dans notre cas le Sypro orange™. L'intensité de fluorescence de ce composé est amplifiée lorsqu'il se retrouve dans un environnement hydrophobe. Ainsi, dans des conditions normales, en solution aqueuse et en présence de la protéine native, le marqueur fluoresce peu, les zones hydrophobes de la protéine n'étant pas accessibles. Lorsque la protéine se dénature sous l'effet de la chaleur, le marqueur va pourvoir interagir avec ses régions hydrophobes, entraînant une augmentation de la fluorescence. On peut ainsi déterminer la température de fusion de la protéine, notée $T_m$, correspondant à un état où la protéine est dénaturée à 50%.

Cette technique permet de mesurer le pouvoir stabilisant ou déstabilisant sur la protéine d'un ligand spécifique. Pour cela, on effectue l'expérience avec la protéine seule puis en incubant la protéine avec un ligand potentiel. On obtient alors deux valeurs de températures de fusion différentes qui permettent de définir un écart de température $\Delta T_m$ exprimé en °C. Si $\Delta T_m > 0$, le ligand stabilise la protéine, si $\Delta T_m < 0$, le ligand déstabilise la protéine. Plus $|\Delta T_m|$ est grand, plus le pouvoir stabilisant ou déstabilisant du ligand est important. Or, le pouvoir stabilisant étant relié à l'affinité du ligand pour la protéine, plus $\Delta T_m$ est élevé et plus le ligand a d'affinité pour cette protéine.

Ce test de fluorescence TSA nous permet donc de quantifier l'affinité de nos ligands pour la protéine EthR et de les classer selon leur valeur de $\Delta T_m$ croissant.

**Le test de Résonance Plasmonique de Surface (RPS)** (décrit dans l'article *"Ethionamide Boosters Combining Bioisosteric Replacement and Structure-Based Drug Design to Solve Pharmacokinetic Issues in a series of potent 1,2,4-Oxadiazole EthR Inhibitors" publié en 2012 dans la revue Journal of Medecinal Chemistry)*

Après avoir mesuré l'affinité des ligands pour la protéine, il est nécessaire de vérifier que cela conduit bien à l'inactivation du répresseur transcriptionnel, l'empêchant ainsi de se lier à son opéron.Pour cela, un test fonctionnel basé sur le principe de la résonance plasmonique de surface est utilisé. La résonance plasmonique de surface (RPS) permet la visualisation en temps réel de phénomènes d'association ou de dissociation entre un partenaire immobilisé sur un biosenseur et un second partenaire injecté en flux continu sur cette surface. Dans le cas présent, la technique de RPS permet de visualiser l'interaction entre la protéine EthR (analyte injecté en flux continu) et le promoteur du gène *ethA* (partenaire immobilisé sur la surface). Deux cellules sont utilisées pour le test : une cellule de mesure où est accroché l'ADN dit relevant, c'est-à-dire correspondant au promoteur du gène *ethA* et une cellule de contrôle où se trouve un brin d'ADN non relevant (donc différent) de même longueur, permettant de soustraire les interactions protéine/ADN non spécifiques. Dans un premier temps, la protéine EthR est injectée seule, jusqu'à saturation, ce qui permet de mesurer un signal $SI_{EthR}$ correspondant à la quantité de protéine EthR venue se lier à l'ADN. Dans un second temps, l'expérience est réitérée en présence d'un ligand potentiel à différentes concentrations. Si le composé se lie à EthR, l'empêchant ainsi de venir se fixer sur l'ADN, une diminution de la réponse du signal ($SI_{EthR+lig}$) dose-dépendante est observée.

En utilisant l'équation ci-dessous, on peut calculer pour chaque concentration un pourcentage d'inhibition du ligand étudié.

$$\%Inhibition = 100 \times \frac{SI_{EthR} - SI_{EthR+Lig}}{SI_{EthR}}$$

En représentant la variation du pourcentage d'inhibition en fonction de la concentration en ligand, on en déduit une valeur d'$IC_{50}$.

**Le test cellulaire de potentialisation de l'éthionamide** (décrit dans l'article *"Ethionamide Boosters Combining Bioisosteric Replacement and Structure-Based Drug Design to Solve Pharmacokinetic Issues in a series of potent 1,2,4-Oxadiazole EthR Inhibitors" publié en 2012 dans la revue Journal of Medecinal Chemistry)*

Le test précédent valide la capacité des composés à inhiber l'interaction EthR/ADN en se liant à la protéine EthR. Le troisième test utilisé permet de vérifier que ces composés sont capables de potentialiser l'activité bactéricide de l'éthionamide sur *M. tuberculosis* seul ou sur des macrophages infectés par *M. tuberculosis.* Ce test est un test de « High Content Screening » (HCS) ou criblage à contenu dense. Les tests HCS sont réalisés sur des cultures cellulaires qui permettent d'étudier certains caractères phénotypiques d'un microorganisme (bactérie par exemple) dans un environnement donné. Les changements phénotypiques observés peuvent aller de l'augmentation (ou la diminution) de la production de certaines protéines marquées à la modification de la morphologie du microorganisme étudié.
Ce test a pour but de déterminer la concentration en ligand nécessaire pour potentialiser dix fois l'activité de l'éthionamide (ETH).
Pour mesurer la concentration de ligand nécessaire pour potentialiser dix fois l'activité de l'ETH, on se place à une concentration constante en éthionamide (0.1μg/mL ce qui correspond au $1/10^{ème}$ de sa $CMI_{99}$). En faisant varier la concentration en ligand on peut déterminer la concentration nécessaire pour inhiber 50% de la pousse bactérienne, c'est-à-dire la concentration nécessaire pour potentialiser dix fois l'activité de l'éthionamide. Cette concentration sera notée $EC_{50}$.

**Mesure de la solubilité**

40μL d'une solution à 10mM dans le DMSO de l'échantillon est ajoutée à 1,96mL de MeOH ou de PBS à pH 7.4. Les échantillons sont alors agités pendant 24h à TA, centrifugés pendant 5min puis filtrés sur des filtres de taille 0.45μm. 20μL de chaque solution est alors ajoutée à 180μL de MeOH puis analysée par LC-MS. La solubilité est déterminée comme le ratio des aires des signaux de masses PBS/MeOH.

**Mesure du logD**

40μL d'une solution à 10mM dans le DMSO de l'échantillon est ajoutée à 1,96mL d'un mélange 1/1 octanol/PBS à pH 7.4. La solution est agitée pendant 2h à TA. 20μL de chaque phase est ajoutée à 180μL de MeOH et analysés par LC-MS. Chaque composé est évalué en triplicate. Le logD est déterminé comme étant le logarithme du ratio des concentrations en produit dans les phases octanol et PBS, déterminés par les signaux de masse.

**ACTIVITES BIOLOGIQUES MESUREES**

Chacun des tableaux suivants regroupe les formules des composés testés ou les définitions des divers radicaux variables mentionnés dans la formule de Markush précedent le tableau concerné et correspondant aux composés testés ainsi que les valeurs de $\Delta T_m$, $EC_{50}$ mesurées expérimentalement selon les protocoles précités etHA qui correspond au nombre d'atomes constituant chaque molécule et différents de l'atome d'hydrogène.

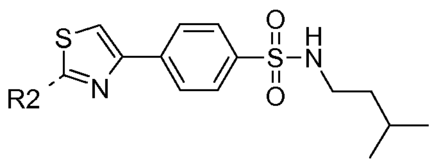

Tableau 1

| **Composé** | **R2** | **$\Delta T_m$(°C)** | **$EC_{50}$(μM)** | **HA** |
|---|---|---|---|---|
| **6** | $H_3C \rightarrow$ | 6.1 | 5.7 | 21 |
| **17** | 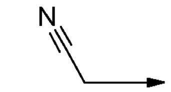 | 5.3 | 7.9 | 23 |
| **21** | 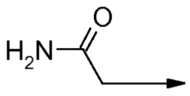 | 4.6 | 5.0 | 24 |

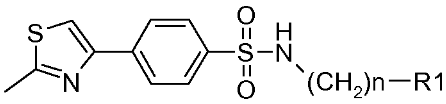

Tableau 2

| **Composé** | **R1** | **n** | **$\Delta T_m$(°C)** | **$EC_{50}$(μM)** | **HA** |
|---|---|---|---|---|---|
| **6** | $-CH(CH_3)_2$ | 2 | 6.1 | 5.7 | 21 |
| **23** | $-C(CH_3)_3$ | 2 | 2.3 | >20 | 22 |
| **24** | $-CF_3$ | 2 | 8.5 | 0.29 | 22 |
| **25** | $-CF_2CF_3$ | 1 | 8.6 | >20 | 24 |
| **26** | $-CF_3$ | 1 | 6.3 | 7.9 | 21 |
| **27** | $-CF_3$ | 3 | 6.6 | >2.5 | 23 |
| **28** | $-CF_2CF_2CF_3$ | 1 | 7.3 | >2.5 | 27 |

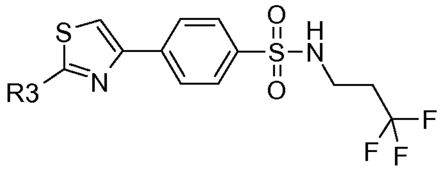

Tableau 3

| **Composé** | **R3** | **$\Delta T_m$(°C)** | **$EC_{50}$(μM)** | **HA** |
|---|---|---|---|---|
| **24** | $H_3C \rightarrow$ | 8.5 | 0.29 | 22 |
| **33** | 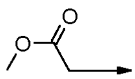 | 9.5 | 0.86 | 26 |
| **34** | 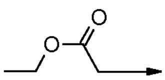 | 9.7 | 0.15 | 27 |
| **35** | 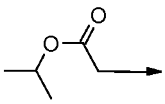 | 11.6 | 0.30 | 28 |

(suite)

| Composé | R3 | $\Delta T_m$(°C) | $EC_{50}$(μM) | HA |
|---|---|---|---|---|
| **36** | 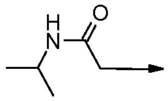 | - | 0.21 | 28 |
| **38** | 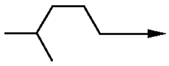 | 12.4 | 0.39 | 27 |
| **39** | 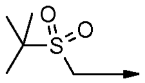 | 7.3 | 0.20 | 29 |

Tableau 4

| Composé | Structure | $\Delta T_m$(◦C) | $EC_{50}$(μM) | HA |
|---|---|---|---|---|
| **41** | 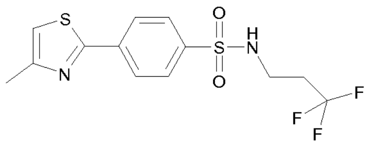 | 6.8 | 0.66 | 22 |
| **42** | 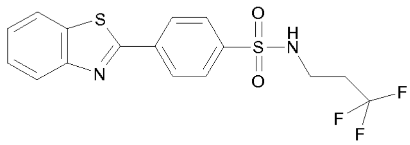 | - | 0.30 | 25 |

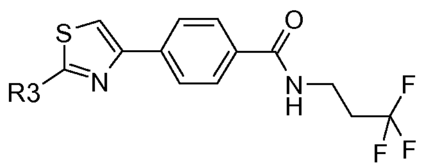

Tableau 5

| Composé | R3 | $\Delta T_m$(◦C) | $EC_{50}$(μM) | HA |
|---|---|---|---|---|
| 43 | $H_3C$- | 11.4 | 0.083 | 21 |
| 52 | 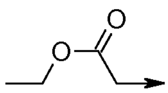 | 10.1 | 0.19 | 26 |
| 53 | 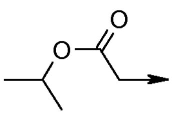 | 13.2 | 0.33 | 27 |
| 50 | 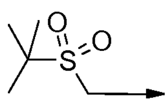 | 9.6 | 0.041 | 28 |
| 103 | 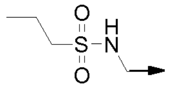 | 6.6 | 0.32 | 28 |

(suite)

| Composé | R3 | $\Delta T_m$(°C) | $EC_{50}$(μM) | HA |
|---|---|---|---|---|
| 104 | 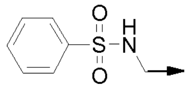 | 1.2 | 0.15 | 31 |
| 47 | 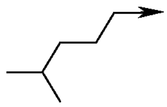 | 13.0 | 0.030 | 26 |
| 54 | 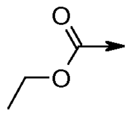 | 9.6 | >2.5 | 25 |
| 55 | 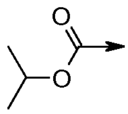 | 8.2 | 0.73 | 26 |
| 49 | 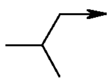 | 12.6 | < 0.040 | 24 |
| 56 | H | 8.1 | 0.36 | 20 |
| 57 | 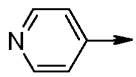 | 12.6 | < 0.010 | 26 |
| 58 | 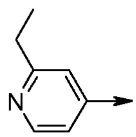 | 13.3 | < 0.010 | 28 |

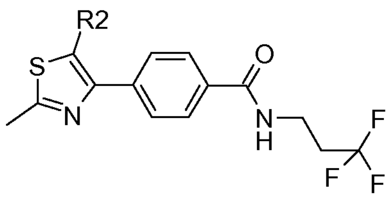

Tableau 6

| Composé | R2 | $\Delta T_m$(°C) | $EC_{50}$(μM) | HA |
|---|---|---|---|---|
| **62** | F | - | 0.055 | 22 |
| **64** | Cl | 8.7 | 0.35 | 22 |

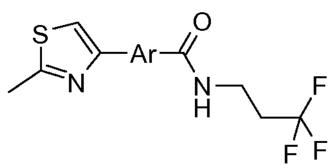

Tableau 7

| **Composé** | **Ar** | **EC$_{50}$(μM)** | **HA** |
|---|---|---|---|
| **67** | 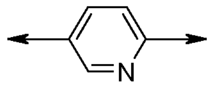 | 0.120 | 21 |
| **71** | 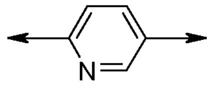 | 1.7 | 21 |

Tableau 8

| **Composé** | Structure | **ΔT$_m$(°C)** | **EC$_{50}$(μM)** | **HA** |
|---|---|---|---|---|
| **92** | 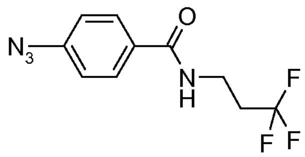 | 6.0 | 1.1 | 18 |

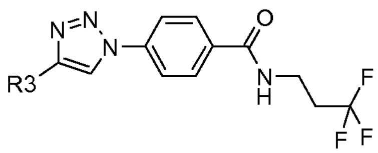

Tableau 9

| **Composé** | **R3** | **ΔT$_m$(°C)** | **EC$_{50}$(μM)** | **HA** |
|---|---|---|---|---|
| **93** | 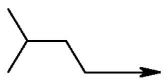 | 3.8 | >10 | 25 |
| **94** | 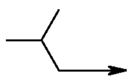 | 4.1 | 1.2 | 24 |
| **95** | 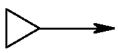 | 3.5 | 1.7 | 23 |
| **97** | 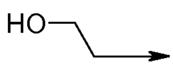 | 1.2 | 2.0 | 23 |

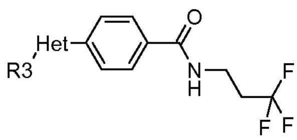

Tableau 10

| Composé | R3 | Het | $EC_{50}$(μM) | HA |
|---|---|---|---|---|
| **72** | $H_3C$→ | 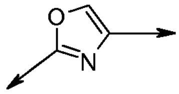 | 0.18 | 21 |
| **75** | $H_3C$→ | 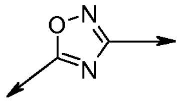 | 0.062 | 21 |
| **78** | $H_3C$→ | 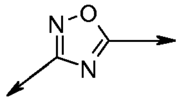 | 0.21 | 21 |
| **80** | $H_3C$→ | 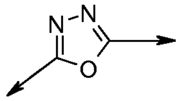 | 0.96 | 21 |
| **83** | $H_3C$→ | 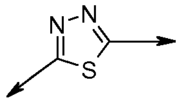 | 1.4 | 21 |
| **90** | 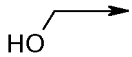 | 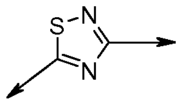 | 0.13 | 22 |

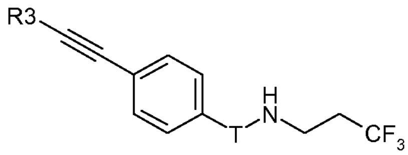

Tableau 11

| Composé | R3 | T | $\Delta T_m$,(°C) | $EC_{50}$(μM) | HA |
|---|---|---|---|---|---|
| **106** | 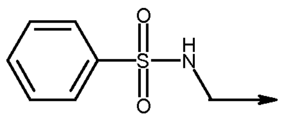 | $SO_2$ | 3.3 | 1.8 | 29 |
| **108** | 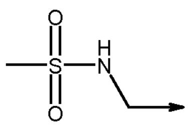 | $SO_2$ | 1.4 | >10 | 24 |
| **112** | 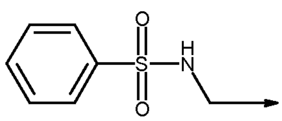 | CO | 4.2 | 0.23 | 28 |

Tableau 12

| Composé | $IC_{50}$(μM) | Solubilité (μM) | LogD |
|---|---|---|---|
| **6** | 4.9 ±0.1 | 20 | 3.55 |

(suite)

| Composé | $IC_{50}$(μM) | Solubilité (μM) | LogD |
|---|---|---|---|
| **24** | 0.55 ±0.02 | 41.4 | 3.12 |
| **35** | - | 2.8 | 3.76 |
| **38** | - | < 1 | >4 |
| **43** | 0.48 ±0.06 | 46.9 | 2.82 |
| **49** | 0.44±0.01 | 4.4 | 3.91 |
| **57** | - | 29 | 3.07 |
| **58** | - | 3.3 | 3.95 |

## Revendications

**1.** Composés de formule générale (I) :

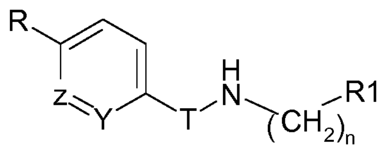

(I)

dans laquelle :

Y et Z sont choisis indépendamment l'un de l'autre parmi CH et N ;
T est choisi parmi CO ou $SO_2$ ;
n est un entier supérieur ou égal à 1 et inférieur ou égal à 3 ;
R1 représente un groupement choisi parmi les chaînes alkyles en C1-C3, linéaires ou ramifiées, les chaînes alkyles en C1-C3 linéaires ou ramifiées et substituées, les groupements cycliques en C3-C6, le groupement cyano, le groupement azido, les chaînes alkoxy en C1-C3, un groupement phényle non substitué, un groupement phényle substitué par un, deux ou trois substituant(s) sélectionné(s) indépendamment les uns des autres parmi les chaînes alkyles linéaires ou ramifiées en C1-C3, le trifluorométhyl, les chaînes alkoxy linéaires ou ramifiées en C1-C3, les halogènes, les hétérocycles à 6 sommets comportant 1 ou 2 atomes d'azote dans le cycle; les hétérocycles aromatiques à 5 sommets ;
R est choisi parmi les groupements azido, cyano, 2-benzothiazolyl, et les groupements (R-1) à (R-10) suivants :

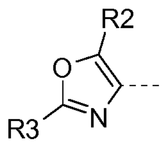
(R-1)

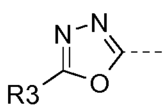
(R-2)

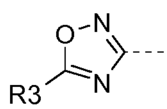
(R-3)

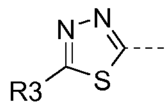
(R-4)

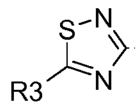
(R-5)

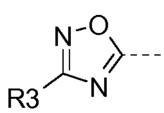
(R-6)

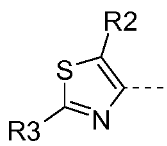
(R-7)

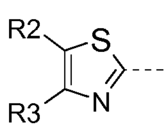
(R-8)

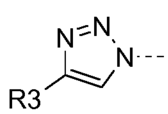
(R-9)

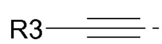
(R-10)

dans lesquels :

R2 est choisi parmi H, F, Cl, Br, I ;
R3 est un groupement sélectionné parmi H, les chaînes alkyles en C1-C6, linéaires ou ramifiées, les chaînes alkyles en C1-C6 linéaires ou ramifiées et substituées par au moins un atome de fluor, les groupements cycliques en C3-C6, le groupement cyanométhyl, le groupement azidométhyl, les chaînes alkoxy linéaires ou ramifiées en C1-C4, les groupements hydroxyalkyle en C1-C4, les groupements alkyle méthyl ester en C1-C4, les groupements alkyle methylcarbonylamino en C1-C4, les groupements alkyle méthylsulfone en C1-C4, le groupement phényl non substitué, un groupement phényle substitué par un, deux ou trois substituants sélectionnés indépendamment les uns des autres parmi les chaînes alkyles en C1-C3, le trifluorométhyl, les chaînes alkoxy en C1-C3, les hétérocycles aromatiques éventuellement substitués, les hétérocycles aromatiques substitués, les hétérocycles aromatiques substitués par au moins un atome de fluor ou par un groupement choisi parmi les groupements (II-a et II-b) suivants :

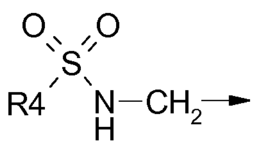
(II-a)

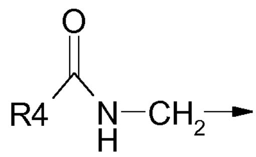
(II-b)

dans lesquels R4 est un groupement choisi parmi H, les chaînes alkyles linéaires ou ramifiées en C1-C4, le groupement phényle, un groupement phényle substitué par au moins un atome d'halogène, un groupement phényle substitué par une chaîne alkyle linéaire ou ramifiée en C1-C4, un groupement phényle substitué par une chaîne alkoxy linéaire ou ramifiée en C1-C4 et un groupement phényle substitué par un groupement trifluorométhyl ;
pour leur utilisation dans le traitement des infections bactériennes et mycobactériennes.

2. Composés selon la revendication 1 pour leur utilisation dans le traitement de la tuberculose, de la lèpre ou des mycobactérioses atypiques.

3. Composés de formule générale (III):

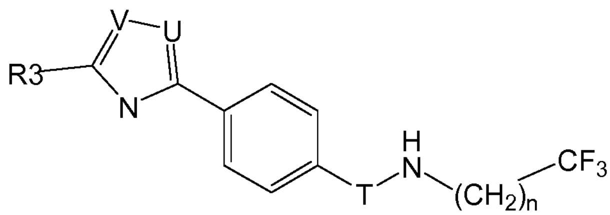
(III)

dans laquelle

T est choisi parmi CO ou $SO_2$;
n est un entier supérieur ou égal à 1 et inférieur ou égal à 3 ;
R3 est un groupement sélectionné parmi H, les chaînes alkyles en C1-C6, linéaires ou ramifiées, les chaînes alkyles en C1-C6 linéaires ou ramifiées et substituées par au moins un atome de fluor, les groupements cycliques en C3-C6, le groupement cyanométhyl, le groupement azidométhyl, les chaînes alkoxy linéaires ou ramifiées en C1-C4, les groupements hydroxyalkyle en C1-C4, les groupements alkyle méthylester en C1-C4, les groupements alkyle methylcarbonylamino en C1-C4, les groupements alkyle méthylsulfone en C1-C4, le groupement phényle non substitué, un groupement phényle substitué par un, deux ou trois substituants sélectionnés indépendamment les uns des autres parmi les chaînes alkyles en C1-C3, le trifluorométhyl, les chaînes alkoxy en C1-C3, les hétérocycles aromatiques éventuellement substitués, les hétérocycles aromatiques substitués, les hétérocycles aromatiques substitués par au moins un atome de fluor ou par un groupement choisi parmi les groupements (II-a et II-b) suivants :

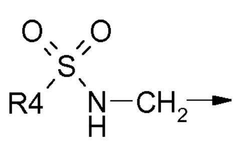 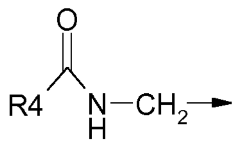

(II-a) (II-b)

dans lesquels R4 est un groupement choisi parmi H, les chaînes alkyles linéaires ou ramifiées en C1-C4, le groupement phényle, un groupement phényle substitué par au moins un atome d'halogène, un groupement phényle substitué par une chaîne alkyle linéaire ou ramifiée en C1-C4, un groupement phényle substitué par une chaîne alkoxy linéaire ou ramifiée en C1-C4 et un groupement phényle substitué par un groupement trifluorométhyl;

U est choisi parmi CH et N ; et V est choisi parmi O ou S.

**4.** Composés selon la revendication 3, **caractérisés en ce qu'**ils correspondent à la formule (IV) suivante :

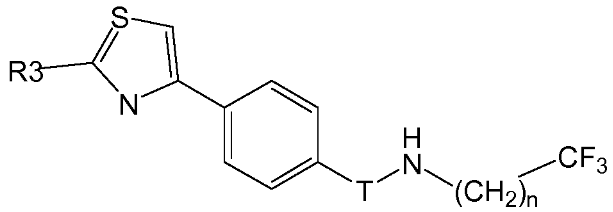

(IV)

dans laquelle n, T et R3 sont tels que définis à la revendication 3.

**5.** Composés selon l'une quelconque des revendications 3 ou 4, **caractérisés en ce que** T est CO et R3 est $-CH_2SO_2R'$ où R' est un groupement tertiobutyle, méthyle, isobutyle, isopentyle, isohexyle, 4-pyridinyle et 4-pyridinyle substitué par une chaîne alkyle linéaire en C1-C4.

**6.** Composés selon la revendication 3 **caractérisés en ce qu'**ils sont choisis parmi les composés suivants :

Composé 24 : 4-(2-méthylthiazol-4-yl)-N-(2,2,2-trifluoropropyl)benzènesulfonamide
Composé 26 : 4-(2-méthylthiazol-4-yl)-N-(2,2,2-trifluoroéthyl)benzènesulfonamide
Composé 27 : 4-(2-méthylthiazol-4-yl)-N-(4,4,4-trifluorobutyl)benzènesulfonamide
Composé 32 : 4-(2-(cyanométhyl)thiazol-4-yl)-N-(3,3,3-trifluoropropyl)benzènesulfon-amide
Composé 33: 2-(4-(4-(N-(3,3,3-trifluoropropyl)sulfamoyl)phényl)thiazol-2-yl)acétate de méthyle
Composé 34 : 2-(4-(4-(N-(3,3,3-trifluoropropyl)sulfamoyl)phényl)thiazol-2-yl)acétate d'éthyle
Composé 35 : 2-(4-(4-(N-(3,3,3-trifluoropropyl)sulfamoyl)phényl)thiazol-2-yl)acétate d'isopropyle
Composé 36 : N-isopropyl-2-(4-(4-(N-(3,3,3-trifluoropropyl)sulfamoyl)phényl)thiazol-2-yl)acétamide
Composé 38 : 4-(2-(4-méthylpentyl)thiazol-4-yl)-N-(3,3,3-trifluoropropyl)benzènesulfon-amide
Composé 39 : 4-(2-(tert-butylsulfonylméthyl)thiazol-4-yl)-N-(3,3,3-trifluoropropyl)benzène-sulfonamide
Composé 41: 4-(4-méthylthiazol-2-yl)-N-(3,3,3-trifluoropropyl)benzènesulfonamide
Composé 43 : 4-(2-méthylthiazol-4-yl)-N-(3,3,3-trifluoropropyl)benzamide
Composé 47 : 4-(2-(4-méthylpentyl)thiazol-4-yl)-N-(3,3,3-trifluoropropyl)benzamide
Composé 49 : 4-(2-isobutylthiazol-4-yl)-N-(3,3,3-trifluoropropyl)benzamide
Composé 50 : 4-(2-(tert-butylsulfonylméthyl)thiazol-4-yl)-N-(3,3,3-trifluoropropyl)-benzamide
Composé 51 : 4-(2-(cyanométhyl)thiazol-4-yl)-N-(3,3,3-trifluoropropyl)benzamide
Composé 52 : 2-(4-(4-(3,3,3-trifluoropropylcarbamoyl)phényl)thiazol-2-yl)acétate d'éthyle
Composé 53 : 2-(4-(4-(3,3,3-trifluoropropylcarbamoyl)phényl)thiazol-2-yl)acétate d'isopropyle
Composé 57: 4-(2-(pyridin-4-yl)thiazol-4-yl)-N-(3,3,3-trifluoropropyl)benzamide
Composé 58: 4-(2-(2-éthylpyridin-4-yl)thiazol-4-yl)-N-(3,3,3-trifluoropropyl)benzamide
Composé 54 : 4-(4-(3,3,3-trifluoropropylcarbamoyl)phényl)thiazole-2-carboxylate d'éthyle
Composé 55: 4-(4-(3,3,3-trifluoropropylcarbamoyl)phényl)thiazole-2-carboxylate d'isopropyle

Composé 56 : 4-(thiazol-4-yl)-N-(3,3,3-trifluoropropyl)benzamide
Composé 72 : 4-(2-méthyloxazol-4-yl)-N-(3,3,3-trifluoropropyl)benzamide
Composé 75 : 4-(5-méthyl-1,2,4-oxadiazol-3-yl)-N-(3,3,3-trifluoropropyl)benzamide
Composé 90: 4-(5-(hydroxyméthyl)-1,2,4-thiadiazol-3-yl)-N-(3,3,3-trifluoropropyl)-benzamide
Composé 103 : 4-(2-(propylsulfonamidométhyl)thiazol-4-yl)-N-(3,3,3-trifluoropropyl)-benzamide; et
Composé 104 : 4-(2-(phénylsulfonamidométhyl)thiazol-4-yl)-N-(3,3,3-trifluoropropyl)-benzamide.

**7.** Composés selon l'une quelconque des revendications 3 à 6 pour leur utilisation en tant que médicament.

**8.** Composés selon l'une quelconque des revendications 3 à 6 pour leur utilisation dans le traitement des infections bactériennes et mycobactériennes.

**9.** Composés selon l'une quelconque des revendications 3 à 6 pour leur utilisation dans le traitement de la tuberculose, de la lèpre ou des mycobactérioses atypiques.

**10.** Composition pharmaceutique comprenant en tant que principe actif au moins un composé selon l'une quelconque des revendications 3 à 6 et/ou au moins un composé de formule générale (I) suivante:

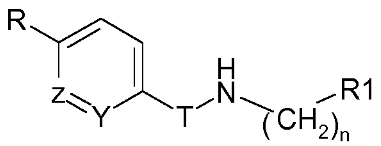

(I)

dans laquelle :

Y et Z sont choisis indépendamment l'un de l'autre parmi CH et N ;
T est choisi parmi CO ou $SO_2$ ;
n est un entier supérieur ou égal à 1 et inférieur ou égal à 3 ;
R1 représente un groupement choisi parmi les chaînes alkyle en C1-C3, linéaires ou ramifiées, les chaînes alkyles en C1-C3 linéaires ou ramifiées et substituées, les groupements cycliques en C3-C6, le groupement cyano, le groupement azido, les chaînes alkoxy en C1-C3, un groupement phényle non substitué, un groupement phényle substitué par un, deux ou trois substituants sélectionnés indépendamment l'un des l'autre parmi les chaînes alkyles linéaires ou ramifiées en C1-C3, le trifluorométhyl, les chaînes alkoxy linéaires ou ramifiées en C1-C3, les halogènes, les hétérocycles à 6 sommets comportant 1 ou 2 atomes d'azote dans le cycle; les hétérocycles aromatiques à 5 sommets ;
R est choisi parmi les groupements azido, cyano, 2-benzothiazolyl, et les groupements (R-1) à (R-10) suivants :

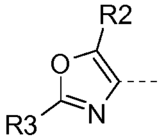
(R-1)

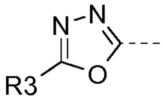
(R-2)

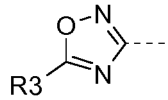
(R-3)

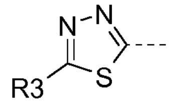
(R-4)

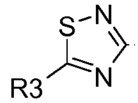
(R-5)

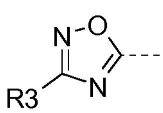
(R-6)

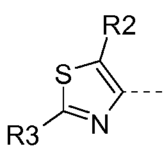
(R-7)

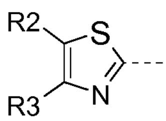
(R-8)

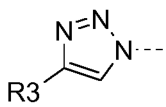
(R-9)

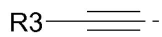
(R-10)

dans lesquels :

R2 est choisi parmi H, F, Cl, Br, I ;

R3 est un groupement sélectionné parmi H, les chaînes alkyles en C1-C6, linéaires ou ramifiées, les chaînes alkyles en C1-C6 linéaires ou ramifiées et substituées par au moins un atome de fluor, les groupements cycliques en C3-C6, le groupement cyanométhyl, le groupement azidométhyl, les chaînes alkoxy linéaires ou ramifiées en C1-C4, les groupements hydroxyalkyle en C1-C4, les groupements alkyle méthylester en C1-C4, les groupements alkyle methylcarbonylamino en C1-C4, les groupements alkyle méthylsulfone en C1-C4, le groupement phényle non substitué, un groupement phényle substitué par un, deux ou trois substituants sélectionnés indépendamment les uns des autres parmi les chaînes alkyles en C1-C3, le trifluorométhyl, les chaînes alkoxy en C1-C3, les hétérocycles aromatiques éventuellement substitués, les hétérocycles aromatiques substitués, les hétérocycles aromatiques substitués par au moins un atome de fluor ou par un groupement choisi parmi les groupements (II-a et II-b) suivants :

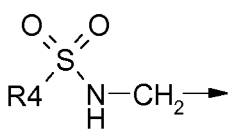

(II-a)

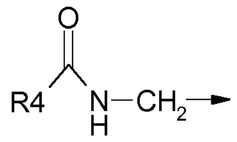

(II-b)

dans lesquels R4 est un groupement choisi parmi H, les chaînes alkyles linéaires ou ramifiées en C1-C4, le groupement phényle, un groupement phényle substitué par au moins un atome halogène, un groupement phényle substitué par une chaîne alkyle linéaire ou ramifiée en C1-C4, un groupement phényle substitué par une chaîne alkoxy linéaire ou ramifiée en C1-C4 et un groupement phényle substitué par un groupement trifluorométhyl ;

et un excipient pharmaceuticalement acceptable.

**11.** Composition selon la revendication 10, **caractérisée en ce qu'**elle comprend en outre, en tant que principe actif, au moins un antibiotique choisi parmi les antibiotiques activables par la voie enzymatique de l'EthA.

**12.** Composition selon la revendication 10, **caractérisée en ce qu'**elle comprend en outre, en tant que principe actif, au moins un antibiotique choisi parmi l'éthionamide, le prothionamide, l'isoxyl et le thiacétazone.

**13.** Produit contenant au moins un composé selon l'une quelconque des revendications 3 à 6 et/ou au moins un composé de formule générale (I) suivante :

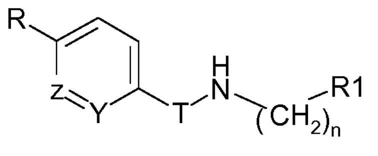

(I)

dans laquelle :

Y et Z sont choisis indépendamment l'un de l'autre parmi CH et N ;

T est choisi parmi CO ou $SO_2$ ;

n est un entier supérieur ou égal à 1 et inférieur ou égal à 3 ;

R1 représente un groupement choisi parmi les chaînes alkyles en C1-C3, linéaires ou ramifiées, les chaînes alkyles en C1-C3 linéaires ou ramifiées et substituées, les groupements cycliques en C3-C6, le groupement cyano, le groupement azido, les chaînes alkoxy en C1-C3, un groupement phényle non substitué, un groupement phényle substitué par un, deux ou trois substituants sélectionnés indépendamment les uns des autres parmi les chaînes alkyles linéaires ou ramifiées en C1-C3, le trifluorométhyl, les chaînes alkoxy linéaires ou ramifiées en C1-C3, les halogènes, les hétérocycles à 6 sommets comportant 1 ou 2 atomes d'azote dans le cycle; les hétérocycles aromatiques à 5 sommets ;

R est choisi parmi les groupements azido, cyano, 2-benzothiazolyl, et les groupements (R-1) à (R-10) suivants :

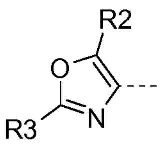
(R-1)

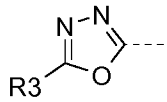
(R-2)

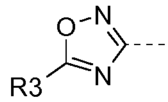
(R-3)

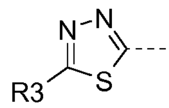
(R-4)

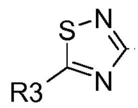
(R-5)

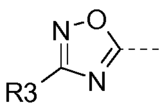
(R-6)

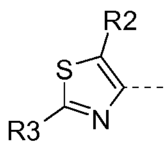
(R-7)

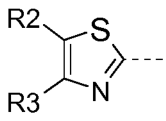
(R-8)

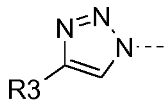
(R-9)

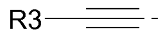
(R-10)

dans lesquels :

R2 est choisi parmi H, F, Cl, Br, I ;
R3 est un groupement sélectionné parmi H, les chaînes alkyles en C1-C6, linéaires ou ramifiées, les chaînes alkyles en C1-C6 linéaires ou ramifiées et substituées par au moins un atome de fluor, les groupements cycliques en C3-C6, le groupement cyanométhyl, le groupement azidométhyl, les chaînes alkoxy linéaires ou ramifiées en C1-C4, les groupements hydroxyalkyle en C1-C4, les groupements alkyle méthylester en C1-C4, les groupements alkyle methylcarbonylamino en C1-C4, les groupements alkyle méthylsulfone en C1-C4, le groupement phényle non substitué, un groupement phényle substitué par un, deux ou trois substituants sélectionnés indépendamment les uns des autres parmi les chaînes alkyles en C1-C3, le trifluorométhyl, les chaînes alkoxy en C1-C3, les hétérocycles aromatiques éventuellement substitués, les hétérocycles aromatiques substitués, les hétérocycles aromatiques substitués par au moins un atome de fluor ou par un groupement choisi parmi les groupements (II-a et II-b) suivants :

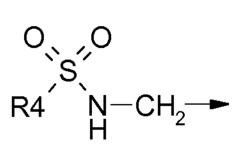
(II-a)

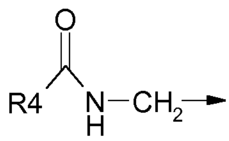
(II-b)

dans lesquels R4 est un groupement choisi parmi H, les chaînes alkyles linéaires ou ramifiées en C1-C4, le groupement phényle, un groupement phényle substitué par au moins un atome halogène, un groupement phényle substitué par une chaîne alkyle linéaire ou ramifiée en C1-C4, un groupement phényle substitué par une chaîne alkoxy linéaire ou ramifiée en C1-C4 et un groupement phényle substitué par un groupement trifluorométhyl ;
et au moins un antibiotique choisi parmi les antibiotiques activables par la voie enzymatique de l'EthA, comme produits de combinaison pour une utilisation simultanée, séparée ou étalée dans le temps en thérapie antituberculeuse, anti-lépromateuse ou anti-mycobactérienne générale.

**14.** Produit selon la revendication 13, **caractérisée en ce que** l'antibiotique choisi parmi les antibiotiques activables par la voie enzymatique de l'EthA est choisi parmi l'éthionamide, le prothionamide, l'isoxyl et le thiacétazone.

**Patentansprüche**

**1.** Verbindungen der allgemeinen Formel (I):

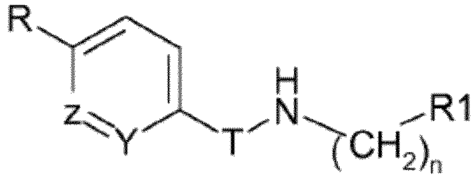

(I),

in der:

Y und Z unabhängig voneinander aus CH und N ausgewählt sind;
T aus CO oder $SO_2$ ausgewählt ist;
n eine ganze Zahl gleich oder größer 1 und gleich oder kleiner 3 ist;
R1 eine Gruppierung, ausgewählt aus geradkettigen oder verzweigten C1-C3-Alkylketten, geradkettigen oder verzweigten und substituierten C1-C3-Alkylketten, cyclischen C3-C6-Gruppierungen, der Cyanogruppierung, der Azidogruppierung, C1-C3-Alkoxyketten, einer unsubstituierten Phenylgruppierung, einer Phenylgruppierung, die durch einen, zwei oder drei Substituenten, unabhängig voneinander ausgewählt aus geradkettigen oder verzweigten C1-C3-Alkylketten, Trifluormethyl, geradkettigen oder verzweigten C1-C3-Alkoxyketten, den Halogenen, Heterocyclen mit 6 Ringgliedern, umfassend 1 oder 2 Stickstoffatome im Zyklus, substituiert ist; aromatischen Heterocyclen mit 5 Ringgliedern, bedeutet;
R aus den Azido-, Cyano-, 2-Benzothiazolylgruppierungen und den folgenden Gruppierungen (R-1) bis (R-10) ausgewählt ist:

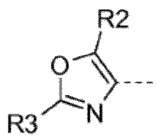
(R-1)
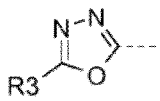
(R-2)
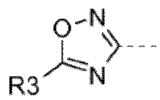
(R-3)
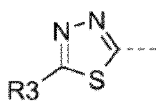
(R-4)
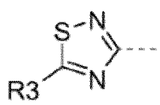
(R-5)

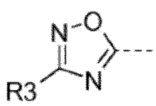
(R-6)
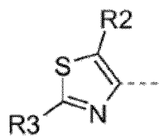
(R-7)
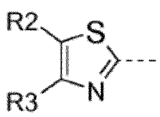
(R-8)
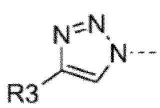
(R-9)
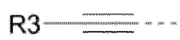
(R-10),

in denen:

R2 aus H, F, Cl, Br, I ausgewählt ist;
R3 eine Gruppierung, ausgewählt aus H, geradkettigen oder verzweigten C1-C6-Alkylketten, geradkettigen oder verzweigten C1-C6-Alkylketten, die durch mindestens ein Fluoratom substituiert sind, cyclischen C3-C6-Gruppierungen, der Cyanomethylgruppierung, der Azidomethylgruppierung, geradkettigen oder verzweigten C1-C4-Alkoxyketten, C1-C4-Hydroxyalkylgruppierungen, C1-C4-Alkylmethylestergruppierungen, C1-C4-Alkylmethylcarbonylaminogruppierungen, C1-C4-Alkylmethylsulfongruppierungen, der unsubstituierten Phenylgruppierung, einer Phenylgruppierung, substituiert durch einen, zwei oder drei Substituenten, unabhängig voneinander ausgewählt aus C1-C3-Alkylketten, Trifluormethyl, C1-C3-Alkoxyketten, gegebenenfalls substituierten aromatischen Heterocyclen, substituierten aromatischen Heterocyclen, aromatischen Heterocyclen, substituiert durch mindestens ein Fluoratom oder durch eine Gruppierung, ausgewählt aus den folgenden Gruppierungen (II-a und II-b) :

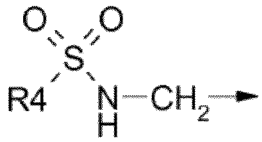

(II-a)

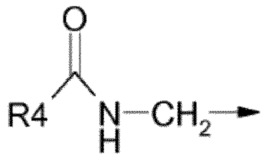

(II-b)

,

in denen R4 eine Gruppierung bedeutet, ausgewählt aus H, geradkettigen oder verzweigten C1-C4-Alkylketten, der Phenylgruppierung, einer Phenylgruppierung, substituiert durch mindestens ein Halogenatom, einer Phenylgruppierung, substituiert durch eine geradkettige oder verzweigte C1-C4-Alkylkette, einer Phenylgruppierung, substituiert durch eine geradkettige oder verzweigte C1-C4-Alkoxykette, und einer Phenylgruppierung, substituiert durch eine Trifluormethylgruppierung, bedeutet;
zur Verwendung in der Behandlung von Bakterien- und Mykobakterieninfektionen.

2. Verbindungen nach Anspruch 1 zur Verwendung in der Behandlung von Tuberkulose, Lepra oder atypischen Mykobakteriosen.

3. Verbindungen der allgemeinen Formel (III):

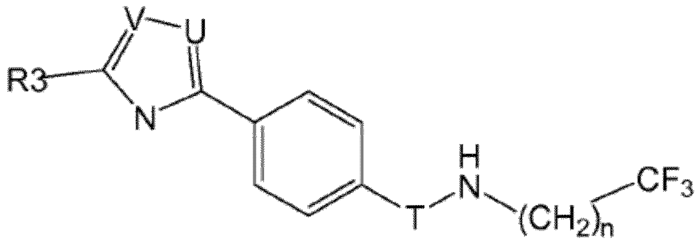

(III)

,

in der

T aus CO oder $SO_2$ ausgewählt ist;
n eine ganze Zahl gleich oder größer 1 und gleich oder kleiner 3 ist;
R3 eine Gruppierung, ausgewählt aus H, geradkettigen oder verzweigten C1-C6-Alkylketten, geradkettigen oder verzweigten C1-C6-Alkylketten, die durch mindestens ein Fluoratom substituiert sind, cyclischen C3-C6-Gruppierungen, der Cyanomethylgruppierung, der Azidomethylgruppierung, geradkettigen oder verzweigten C1-C4-Alkoxyketten, C1-C4-Hydroxyalkylgruppierungen, C1-C4-Alkylmethylestergruppierungen, C1-C4-Alkylmethylcarbonylaminogruppierungen, C1-C4-Alkyl-methylsulfongruppierungen, der unsubstituierten Phenylgruppierung, einer Phenylgruppierung, substituiert durch einen, zwei oder drei Substituenten, unabhängig voneinander ausgewählt aus C1-C3-Alkylketten, Trifluormethyl, C1-C3-Alkoxyketten, gegebenenfalls substituierten aromatischen Heterocyclen, substituierten aromatischen Heterocyclen, aromatischen Heterocyclen, substituiert durch mindestens ein Fluoratom oder durch eine Gruppierung, ausgewählt aus den folgenden Gruppierungen (II-a und II-b) :

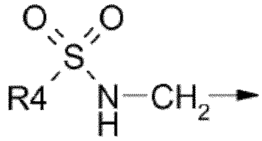

(II-a)

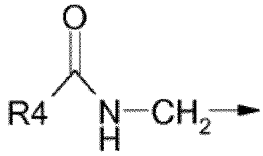

(II-b)

,

in denen R4 eine Gruppierung bedeutet, ausgewählt aus H, geradkettigen oder verzweigten C1-C4-Alkylketten, der Phenylgruppierung, einer Phenylgruppierung, substituiert durch mindestens ein Halogenatom, einer Phenylgruppierung, substituiert durch eine geradkettige oder verzweigte C1-C4-Alkylkette, einer Phenylgruppierung, substituiert durch eine geradkettige oder verzweigte C1-C4-Alkoxykette, und einer Phenylgruppierung, substituiert durch eine Trifluormethylgruppierung, bedeutet;
U aus CH und N ausgewählt ist; und V aus O oder S ausgewählt ist.

4. Verbindungen nach Anspruch 3, **dadurch gekennzeichnet, dass** sie der folgenden Formel (IV) entsprechen:

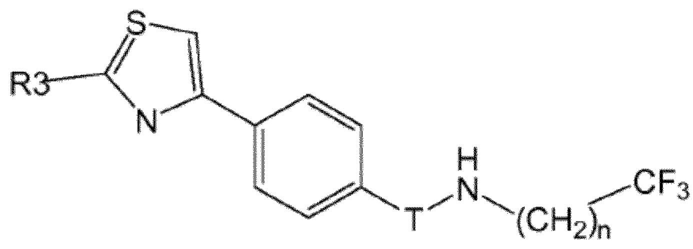

(IV) ,

in der n, T und R3 wie in Anspruch 3 definiert sind.

5. Verbindungen nach einem der Ansprüche 3 oder 4, **dadurch gekennzeichnet, dass** T CO bedeutet und R3- $CH_2SO_2R'$ bedeutet, worin R' eine tert.-Butyl-, Methyl-, Isobutyl-, Isopentyl-, Isohexyl-, 4-Pyridinylgruppierung und 4-Pyridinylgruppierung, die durch eine geradkettige C1-C4-Alkylkette substituiert ist, bedeutet.

6. Verbindungen nach Anspruch 3, **dadurch gekennzeichnet, dass** sie aus den folgenden Verbindungen ausgewählt sind:

Verbindung 24: 4-(2-Methylthiazol-4-yl)-N-(2,2,2-trifluorpropyl)benzolsulfonamid
Verbindung 26: 4-(2-Methylthiazol-4-yl)-N-(2,2,2-trifluorethyl)benzolsulfonamid
Verbindung 27: 4-(2-Methylthiazol-4-yl)-N-(4,4,4-trifluorbutyl)benzolsulfonamid
Verbindung 32: 4-(2-(Cyanomethyl)thiazol-4-yl)-N-(3,3,3-trifluorpropyl)benzolsulfonamid
Verbindung 33: Methyl-2-(4-(4-(N-(3,3,3-trifluorpropyl)sulfamoyl)phenyl)thiazol-2-yl)acetat
Verbindung 34: Ethyl-2-(4-(4-(N-(3,3,3-trifluorpropyl)sulfamoyl)phenyl)thiazol-2-yl)acetat
Verbindung 35: Isopropyl-2-(4-(4-(N-(3,3,3-trifluorpropyl)sulfamoyl)phenyl)thiazol-2-yl)acetat
Verbindung 36: N-Isopropyl-2-(4-(4-(N-(3,3,3-trifluorpropyl)sulfamoyl)phenyl)thiazol-2-yl)acetamid
Verbindung 38 : 4-(2-(4-Methylpentyl)thiazol-4-yl)-N-(3,3,3-trifluorpropyl)benzolsulfonamid
Verbindung 39: 4-(2-(tert.-Butylsulfonylmethyl)thaizol-4-yl)-N-(3,3,3-trifluorpropyl)benzolsulfonamid
Verbindung 41: 4-(4-Methylthiazol-2-yl)-N-(3,3,3-trifluorpropyl)benzolsulfonamid
Verbindung 43: 4-(2-Methylthiazol-4-yl)-N-(3,3,3-trifluorpropyl)benzamid
Verbindung 47: 4-(2-(4-Methylpentyl)thiazol-4-yl)-N-(3,3,3-trifluorpropyl)benzamid
Verbindung 49: 4-(2-Isobutylthiazol-4-yl)-N-(3,3,3-trifluorpropyl)benzamid
Verbindung 50: 4-(2-(tert.-Butylsulfonylmethyl)thiazol-4-yl)-N-(3,3,3-trifluorpropyl)benzamid
Verbindung 51: 4-(2-(Cyanomethyl)thiazol-4-yl)-N-(3,3,3-trifluorpropyl)benzamid
Verbindung 52: Ethyl-2-(4-(4-(3,3,3-trifluorpropylcarbamoyl)phenyl)thiazol-2-yl)acetat
Verbindung 53: Isopropyl-2-(4-(4-(3,3,3-trifluorpropylcarbamoyl)phenyl)thiazol-2-yl)-acetat
Verbindung 57: 4-(2-(Pyridin-4-yl)thiazol-4-yl)-N-(3,3,3-trifluorpropyl)benzamid
Verbindung 58: 4-(2-(2-Ethylpyridin-4-yl)thiazol-4-yl)-N-(3,3,3-trifluorpropyl)benzamid
Verbindung 54: Ethyl-4-(4-(3,3,3-trifluorpropylcarbamoyl)phenyl)thiazol-2-carboxylat
Verbindung 55: Isopropyl-4-(4-(3,3,3-trifluorpropylcarbamoyl)phenyl)thiazol-2-carboxylat
Verbindung 56: 4-(Thiazol-4-yl)-N-(3,3,3-trifluorpropyl)benzamid
Verbindung 72: 4-(2-Methyloxazol-4-yl)-N-(3,3,3-trifluorpropyl)benzamid
Verbindung 75: 4-(5-Methyl-1,2,4-oxadiazol-3-yl)-N-(3,3,3-trifluorpropyl)benzamid
Verbindung 90: 4-(5-(Hydroxymethyl)-1,2,4-thiadiazol-3-yl)-N-(3,3,3-trifluorpropyl)benzamid
Verbindung 103: 4-(2-(Propylsulfonamidomethyl)thiazol-4-yl)-N-(3,3,3-trifluorpropyl)benzamid und
Verbindung 104: 4-(2-(Phenylsulfonamidomethyl)thiazol-4-yl)-N-(3,3,3-trifluorpropyl)benzamid.

**7.** Verbindungen nach einem der Ansprüche 3 bis 6 zur Verwendung als Arzneimittel.

**8.** Verbindungen nach einem der Ansprüche 3 bis 6 zur Verwendung in der Behandlung von Bakterien- und Mykobakterieninfektionen.

**9.** Verbindungen nach einem der Ansprüche 3 bis 6 zur Verwendung in der Behandlung von Tuberkulose, Lepra oder atypischen Mykobakteriosen.

**10.** Pharmazeutische Zusammensetzung, die als Wirkstoff mindestens eine Verbindung nach einem der Ansprüche 3 bis 6 und/oder mindestens eine Verbindung der folgenden allgemeinen Formel (I) :

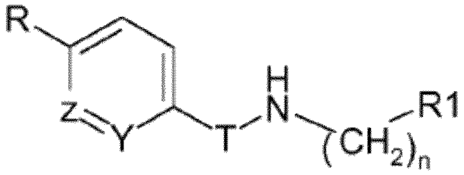

(I) ,

in der:

Y und Z unabhängig voneinander aus CH und N ausgewählt sind;
T aus CO oder $SO_2$ ausgewählt ist;
n eine ganze Zahl gleich oder größer 1 und gleich oder kleiner 3 ist;
R1 eine Gruppierung, ausgewählt aus geradkettigen oder verzweigten C1-C3-Alkylketten, geradkettigen oder verzweigten und substituierten C1-C3-Alkylketten, cyclischen C3-C6-Gruppierungen, der Cyanogruppierung, der Azidogruppierung, C1-C3-Alkoxyketten, einer unsubstituierten Phenylgruppierung, einer Phenylgruppierung, die durch einen, zwei oder drei Substituenten, unabhängig voneinander ausgewählt aus geradkettigen oder verzweigten C1-C3-Alkylketten, Trifluormethyl, geradkettigen oder verzweigten C1-C3-Alkoxyketten, den Halogenen, Heterocyclen mit 6 Ringgliedern, umfassend 1 oder 2 Stickstoffatome im Zyklus, substituiert ist; aromatischen Heterocyclen mit 5 Ringgliedern, bedeutet;
R aus den Azido-, Cyano-, 2-Benzothiazolylgruppierungen und den folgenden Gruppierungen (R-1) bis (R-10) ausgewählt ist:

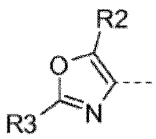
(R-1)

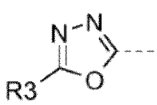
(R-2)

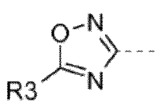
(R-3)

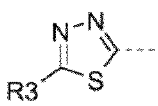
(R-4)

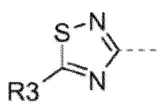
(R-5)

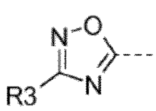
(R-6)

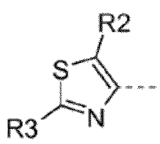
(R-7)

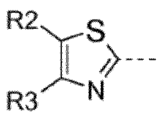
(R-8)

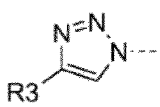
(R-9)

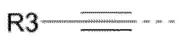
(R-10) ,

in denen:

R2 aus H, F, Cl, Br, I ausgewählt ist;
R3 eine Gruppierung, ausgewählt aus H, geradkettigen oder verzweigten C1-C6-Alkylketten, geradkettigen oder verzweigten C1-C6-Alkylketten, die durch mindestens ein Fluoratom substituiert sind, cyclischen C3-C6-Gruppierungen, der Cyanomethylgruppierung, der Azidomethylgruppierung, geradkettigen oder verzweigten C1-C4-Alkoxyketten, C1-C4-Hydroxyalkylgruppierungen, C1-C4-Alkylmethylestergruppierungen, C1-C4-Alkylmethylcarbonylaminogruppierungen, C1-C4-Alkyl-methylsulfongruppierungen, der unsubstituierten Phenylgruppierung, einer Phenylgruppierung, substituiert durch einen, zwei oder drei Substituenten, unabhängig voneinander ausgewählt aus C1-C3-Alkylketten, Trifluormethyl, C1-C3-Alkoxyketten, gegebenenfalls substituierten aromatischen Heterocyclen, substituierten aromatischen Heterocyclen, aromatischen Heterocyclen, substituiert durch mindestens ein Fluoratom oder durch eine Gruppierung, ausgewählt aus den folgenden Gruppierungen (II-a und II-b):

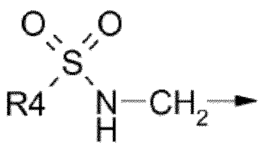

(II-a)

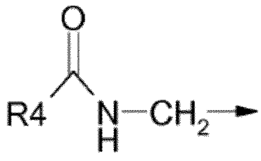

(II-b)

,

in denen R4 eine Gruppierung bedeutet, ausgewählt aus H, geradkettigen oder verzweigten C1-C4-Alkylketten, der Phenylgruppierung, einer Phenylgruppierung, substituiert durch mindestens ein Halogenatom, einer Phenylgruppierung, substituiert durch eine geradkettige oder verzweigte C1-C4-Alkylkette, einer Phenylgruppierung, substituiert durch eine geradkettige oder verzweigte C1-C4-Alkoxykette, und einer Phenylgruppierung, substituiert durch eine Trifluormethylgruppierung, bedeutet;
und einen pharmazeutisch unbedenklichen Grundstoff umfasst.

**11.** Zusammensetzung nach Anspruch 10, **dadurch gekennzeichnet, dass** sie weiterhin als Wirkstoff mindestens ein Antibiotikum, ausgewählt aus den Antibiotika, die enzymatisch über EthA aktivierbar sind, umfasst.

**12.** Zusammensetzung nach Anspruch 10, **dadurch gekennzeichnet, dass** sie weiterhin als Wirkstoff mindestens ein Antibiotikum, ausgewählt aus Ethionamid, Prothionamid, Isoxyl und Thiacetazon, umfasst.

**13.** Produkt, das mindestens eine Verbindung nach einem der Ansprüche 3 bis 6 und/oder mindestens eine Verbindung der folgenden allgemeinen Formel (I):

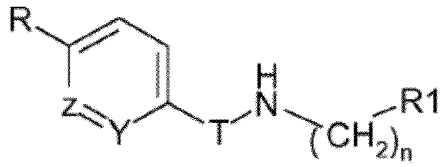

(I)

,

in der:

Y und Z unabhängig voneinander aus CH und N ausgewählt sind;
T aus CO oder $SO_2$ ausgewählt ist;
n eine ganze Zahl gleich oder größer 1 und gleich oder kleiner 3 ist;
R1 eine Gruppierung, ausgewählt aus geradkettigen oder verzweigten C1-C3-Alkylketten, geradkettigen oder verzweigten und substituierten C1-C3-Alkylketten, cyclischen C3-C6-Gruppierungen, der Cyanogruppierung, der Azidogruppierung, C1-C3-Alkoxyketten, einer unsubstituierten Phenylgruppierung, einer Phenylgruppierung, die durch einen, zwei oder drei Substituenten, unabhängig voneinander ausgewählt aus geradkettigen oder verzweigten C1-C3-Alkylketten, Trifluormethyl, geradkettigen oder verzweigten C1-C3-Alkoxyketten, den Halogenen, Heterocyclen mit 6 Ringgliedern, umfassend 1 oder 2 Stickstoffatome im Zyklus, substituiert ist; aromatischen Heterocyclen mit 5 Ringgliedern, bedeutet;
R aus den Azido-, Cyano-, 2-Benzothiazolylgruppierungen und den folgenden Gruppierungen (R-1) bis (R-10) ausgewählt ist:

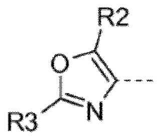
(R-1)

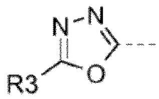
(R-2)

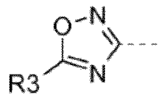
(R-3)

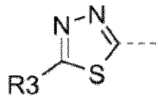
(R-4)

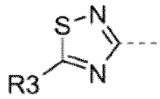
(R-5)

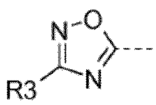
(R-6)

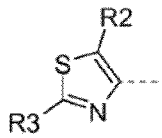
(R-7)

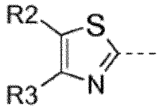
(R-8)

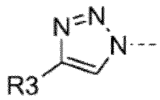
(R-9)

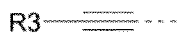
(R-10)

,

in denen:

R2 aus H, F, Cl, Br, I ausgewählt ist;

R3 eine Gruppierung, ausgewählt aus H, geradkettigen oder verzweigten C1-C6-Alkylketten, geradkettigen oder verzweigten C1-C6-Alkylketten, die durch mindestens ein Fluoratom substituiert sind, cyclischen C3-C6-Gruppierungen, der Cyanomethylgruppierung, der Azidomethylgruppierung, geradkettigen oder verzweigten C1-C4-Alkoxyketten, C1-C4-Hydroxyalkylgruppierungen, C1-C4-Alkylmethylestergruppierungen, C1-C4-Alkylmethylcarbonylaminogruppierungen, C1-C4-Alkylmethylsulfongruppierungen, der unsubstituierten Phenylgruppierung, einer Phenylgruppierung, substituiert durch einen, zwei oder drei Substituenten, unabhängig voneinander ausgewählt aus C1-C3-Alkylketten, Trifluormethyl, C1-C3-Alkoxyketten, gegebenenfalls substituierten aromatischen Heterocyclen, substituierten aromatischen Heterocyclen, aromatischen Heterocyclen, substituiert durch mindestens ein Fluoratom oder durch eine Gruppierung, ausgewählt aus den folgenden Gruppierungen (II-a und II-b) :

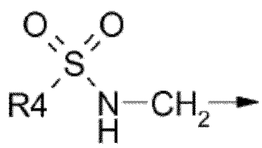
(II-a)

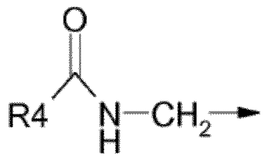
(II-b)

,

in denen R4 eine Gruppierung bedeutet, ausgewählt aus H, geradkettigen oder verzweigten C1-C4-Alkylketten, der Phenylgruppierung, einer Phenylgruppierung, substituiert durch mindestens ein Halogenatom, einer Phenylgruppierung, substituiert durch eine geradkettige oder verzweigte C1-C4-Alkylkette, einer Phenylgruppierung, substituiert durch eine geradkettige oder verzweigte C1-C4-Alkoxykette, und einer Phenylgruppierung, substituiert durch eine Trifluormethylgruppierung, bedeutet;

und mindestens ein Antibiotikum, ausgewählt aus den Antibiotika, die enzymatisch über EthA aktivierbar sind, enthält, als Kombinationsprodukte für eine gleichzeitige, getrennte oder zeitlich gestaffelte Verwendung in der Therapie gegen die Tuberkulose, Therapie gegen die Lepra oder allgemeine Therapie gegen Mykobakterien.

14. Produkt nach Anspruch 13, **dadurch gekennzeichnet, dass** das Antibiotikum, ausgewählt aus den Antibiotika, die enzymatisch über EthA aktivierbar sind, aus Ethionamid, Prothionamid, Isoxyl und Thiacetazon ausgewählt ist.

## Claims

1. Compounds of general formula (I):

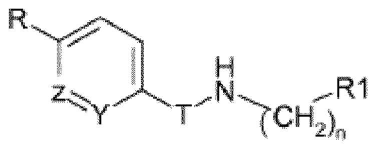

(I)

in which:

Y and z are chosen, independently of one another, from CH and N;
T is chosen from CO or $SO_2$;
n is an integer greater than or equal to 1 and less than or equal to 3;
R1 represents a group chosen from linear or branched $C_1$-$C_3$ alkyl chains, linear or branched and substituted $C_1$-$C_3$ alkyl chains, $C_3$-$C_6$ cyclic groups, the cyano group, the azido group, $C_1$-$C_3$ alkoxy chains, an unsubstituted phenyl group, a phenyl group substituted by one, two or three substituent(s) selected, independently of one another, from linear or branched $C_1$-$C_3$ alkyl chains, trifluoromethyl, linear or branched $C_1$-$C_3$ alkoxy chains, halogens, 6-membered heterocycles comprising 1 or 2 nitrogen atoms in the ring, 5-membered aromatic heterocycles;
R is chosen from the azido, cyano, or 2-benzothiazolyl groups and the following groups (R-1) to (R-10):

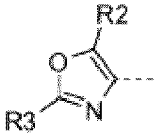
(R-1)
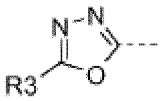
(R-2)
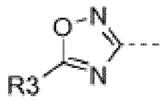
(R-3)
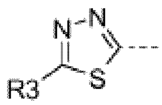
(R-4)
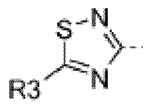
(R-5)

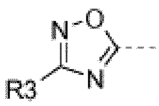
(R-6)
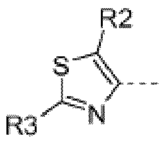
(R-7)
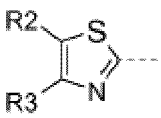
(R-8)
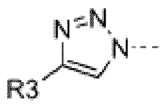
(R-9)
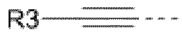
(R-10)

in which:

R2 is chosen from H, F, Cl, Br or I;
R3 is a group selected from H, linear or branched $C_1$-$C_6$ alkyl chains, linear or branched $C_1$-$C_6$ alkyl chains which are substituted by at least one fluorine atom, cyclic $C_3$-$C_6$ groups, the cyanomethyl group, the azidomethyl group, linear or branched $C_1$-$C_4$ alkoxy chains, $C_1$-$C_4$ hydroxyalkyl groups, $C_1$-$C_4$ alkyl methyl ester groups, $C_1$-$C_4$ alkyl methylcarbonylamino groups, $C_1$-$C_4$ alkyl methyl sulphone groups, the unsubstituted phenyl group, a phenyl group substituted by one, two or three substituents selected, independently of one another, from $C_1$-$C_3$ alkyl chains, trifluoromethyl, $C_1$-$C_3$ alkoxy chains, optionally substituted aromatic heterocycles, substituted aromatic heterocycles, aromatic heterocycles substituted by at least one fluorine atom or by a group chosen from the following groups (II-a and II-b):

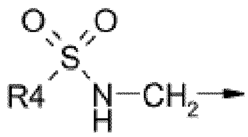
(II-a)
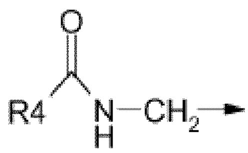
(II-b)

in which R4 is a group chosen from H, linear or branched $C_1$-$C_4$ alkyl chains, the phenyl group, a phenyl

group substituted by at least one halogen atom, a phenyl group substituted by a linear or branched $C_1$-$C_4$ alkyl chain, a phenyl group substituted by a linear or branched $C_1$-$C_4$ alkoxy chain and a phenyl group substituted by a trifluoromethyl group;

for their use in the treatment of bacterial and mycobacterial infections.

**2.** Compounds according to Claim 1, for their use in the treatment of tuberculosis, leprosy or atypical mycobacterioses.

**3.** Compounds of general formula (III):

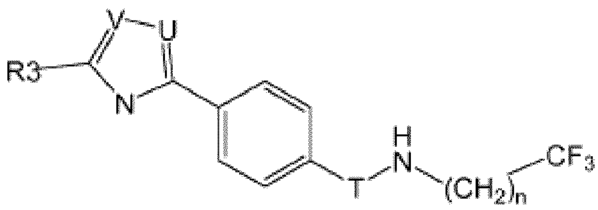

(III)

in which:

T is chosen from CO or $SO_2$;

n is an integer greater than or equal to 1 and less than or equal to 3;

R3 is a group selected from H, linear or branched $C_1$-$C_6$ alkyl chains, linear or branched $C_1$-$C_6$ alkyl chains which are substituted by at least one fluorine atom, cyclic $C_3$-$C_6$ groups, the cyanomethyl group, the azidomethyl group, linear or branched $C_1$-$C_4$ alkoxy chains, $C_1$-$C_4$ hydroxyalkyl groups, $C_1$-$C_4$ alkyl methyl ester groups, $C_1$-$C_4$ alkyl methylcarbonylamino groups, $C_1$-$C_4$ alkyl methyl sulphone groups, the unsubstituted phenyl group, a phenyl group substituted by one, two or three substituents selected, independently of one another, from $C_1$-$C_3$ alkyl chains, trifluoromethyl, $C_1$-$C_3$ alkoxy chains, optionally substituted aromatic heterocycles, substituted aromatic heterocycles, aromatic heterocycles substituted by at least one fluorine atom or by a group chosen from the following groups (II-a and II-b):

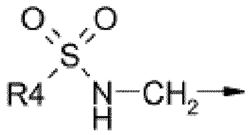

(II-a)

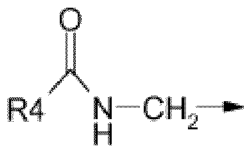

(II-b)

in which R4 is a group chosen from H, linear or branched $C_1$-$C_4$ alkyl chains, the phenyl group, a phenyl group substituted by at least one halogen atom, a phenyl group substituted by a linear or branched $C_1$-$C_4$ alkyl chain, a phenyl group substituted by a linear or branched $C_1$-$C_4$ alkoxy chain and a phenyl group substituted by a trifluoromethyl group;

U is chosen from CH and N; and V is chosen from O or S.

**4.** Compounds according to Claim 3, **characterized in that** they correspond to the following formula (IV) :

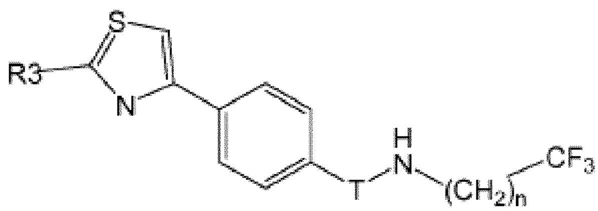

(IV)

in which n, T and R3 are as defined in Claim 3.

5. Compounds according to either one of Claims 3 and 4, **characterized in that** T is CO and R3 is $-CH_2SO_2R'$ where R' is a tert-butyl group, a methyl group, an isobutyl group, an isopentyl group, an isohexyl group, a 4-pyridinyl group or a 4-pyridinyl group substituted by a linear $C_1$-$C_4$ alkyl chain.

6. Compounds according to Claim 3, **characterized in that** they are chosen from the following compounds:

Compound 24: 4-(2-methylthiazol-4-yl)-N-(2,2,2-trifluoropropyl)benzenesulphonamide
Compound 26: 4-(2-methylthiazol-4-yl)-N-(2,2,2-trifluoroethyl)benzenesulphonamide
Compound 27: 4-(2-methylthiazol-4-yl)-N-(4,4,4-trifluorobutyl)benzenesulphonamide
Compound 32: 4-(2-(cyanomethyl)thiazol-4-yl)-N-(3,3,3-trifluoropropyl)benzenesulphonamide
Compound 33: Methyl 2-(4-(4-(N-(3,3,3-trifluoropropyl)sulphamoyl)phenyl)thiazol-2-yl)acetate
Compound 34: Ethyl 2-(4-(4-(N-(3,3,3-trifluoropropyl)sulphamoyl)phenyl)thiazol-2-yl)acetate
Compound 35: Isopropyl 2-(4-(4-(N-(3,3,3-trifluoropropyl)sulphamoyl)phenyl)thiazol-2-yl)acetate
Compound 36: N-isopropyl-2-(4-(4-(N-(3,3,3-trifluoropropyl)sulphamoyl)phenyl)thiazol-2-yl)acetamide
Compound 38: 4-(2-(4-methylpentyl)thiazol-4-yl)-N-(3,3,3-trifluoropropyl)benzenesulphonamide
Compound 39: 4-(2-(tert-butylsulphonylmethyl)-thiazol-4-yl)-N-(3,3,3-trifluoropropyl)benzenesulphonamide
Compound 41: 4-(4-methylthiazol-2-yl)-N-(3,3,3-trifluoropropyl)benzenesulphonamide
Compound 43: 4-(2-methylthiazol-4-yl)-N-(3,3,3-trifluoropropyl)benzamide
Compound 47: 4-(2-(4-methylpentyl)thiazol-4-yl)-N-(3,3,3-trifluoropropyl)benzamide
Compound 49: 4-(2-isobutylthiazol-4-yl)-N-(3,3,3-trifluoropropyl)benzamide
Compound 50: 4-(2-(tert-butylsulphonylmethyl)-thiazol-4-yl)-N-(3,3,3-trifluoropropyl)benzamide
Compound 51: 4-(2-(cyanomethyl)thiazol-4-yl)-N-(3,3,3-trifluoropropyl)benzamide
Compound 52: Ethyl 2-(4-(4-(3,3,3-trifluoro-propylcarbamoyl)phenyl)thiazol-2-yl)acetate
Compound 53: Isopropyl 2-(4-(4-(3,3,3-trifluoro-propylcarbamoyl)phenyl)thiazol-2-yl)acetate
Compound 57: 4-(2-(pyridin-4-yl)thiazol-4-yl)-N-(3,3,3-trifluoropropyl)benzamide
Compound 58: 4-(2-(2-ethylpyridin-4-yl)thiazol-4-yl)-N-(3,3,3-trifluoropropyl)benzamide
Compound 54: Ethyl 4-(4-(3,3,3-trifluoropropylcarbamoyl)phenyl)thiazole-2-carboxylate
Compound 55: Isopropyl 4-(4-(3,3,3-trifluoropropylcarbamoyl)phenyl)thiazole-2-carboxylate
Compound 56: 4-(thiazol-4-yl)-N-(3,3,3-trifluoropropyl)benzamide
Compound 72: 4-(2-methyloxazol-4-yl)-N-(3,3,3-trifluoropropyl)benzamide
Compound 75: 4-(5-methyl-1,2,4-oxadiazol-3-yl)-N-(3,3,3-trifluoropropyl)benzamide
Compound 90: 4-(5-(hydroxymethyl)-1,2,4-thiadiazol-3-yl)-N-(3,3,3-trifluoropropyl)benzamide
Compound 103: 4-(2-(propylsulphonamidomethyl)-thiazol-4-yl)-N-(3,3,3-trifluoropropyl)benzamide; and
Compound 104: 4-(2-(phenylsulphonamidomethyl)-thiazol-4-yl)-N-(3,3,3-trifluoropropyl)benzamide.

7. Compounds according to any one of Claims 3 to 6 for their use as medicament.

8. Compounds according to any one of Claims 3 to 6 for their use in the treatment of bacterial and mycobacterial infections.

9. Compounds according to any one of Claims 3 to 6 for their use in the treatment of tuberculosis, leprosy or atypical mycobacterioses.

10. Pharmaceutical composition comprising, as active principle, at least one compound according to any one of Claims 3 to 6 and/or at least one compound of general formula (I):

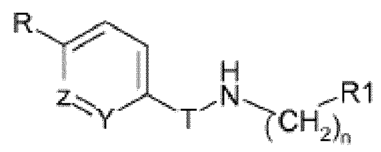

(I)

in which:

Y and z are chosen, independently of one another, from CH and N;

T is chosen from CO or $SO_2$;
n is an integer greater than or equal to 1 and less than or equal to 3;
R1 represents a group chosen from linear or branched $C_1$-$C_3$ alkyl chains, linear or branched and substituted $C_1$-$C_3$ alkyl chains, $C_3$-$C_6$ cyclic groups, the cyano group, the azido group, $C_1$-$C_3$ alkoxy chains, an unsubstituted phenyl group, a phenyl group substituted by one, two or three substituents selected, independently of one another, from linear or branched $C_1$-$C_3$ alkyl chains, trifluoromethyl, linear or branched $C_1$-$C_3$ alkoxy chains, halogens, 6-membered heterocycles comprising 1 or 2 nitrogen atoms in the ring, 5-membered aromatic heterocycles;
R is chosen from the azido, cyano, or 2-benzothiazolyl groups and the following groups (R-1) to (R-10):

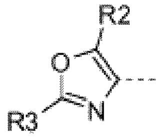
(R-1)
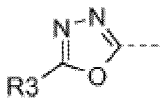
(R-2)
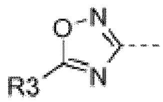
(R-3)
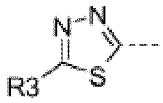
(R-4)
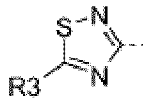
(R-5)

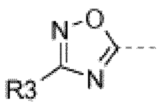
(R-6)
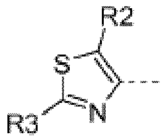
(R-7)
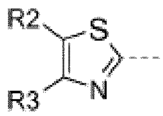
(R-8)
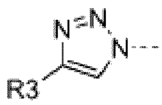
(R-9)
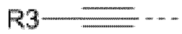
(R-10)

in which:

R2 is chosen from H, F, Cl, Br or I;
R3 is a group selected from H, linear or branched $C_1$-$C_6$ alkyl chains, linear or branched $C_1$-$C_6$ alkyl chains which are substituted by at least one fluorine atom, cyclic $C_3$-$C_6$ groups, the cyanomethyl group, the azidomethyl group, linear or branched $C_1$-$C_4$ alkoxy chains, $C_1$-$C_4$ hydroxyalkyl groups, $C_1$-$C_4$ alkyl methyl ester groups, $C_1$-$C_4$ alkyl methylcarbonylamino groups, $C_1$-$C_4$ alkyl methyl sulphone groups, the unsubstituted phenyl group, a phenyl group substituted by one, two or three substituents selected, independently of one another, from $C_1$-$C_3$ alkyl chains, trifluoromethyl, $C_1$-$C_3$ alkoxy chains, optionally substituted aromatic heterocycles, substituted aromatic heterocycles, aromatic heterocycles substituted by at least one fluorine atom or by a group chosen from the following groups (II-a and II-b):

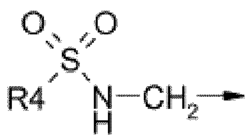
(II-a)
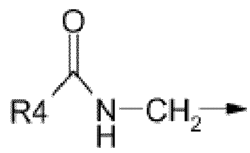
(II-b)

in which R4 is a group chosen from H, linear or branched $C_1$-$C_4$ alkyl chains, the phenyl group, a phenyl group substituted by at least one halogen atom, a phenyl group substituted by a linear or branched $C_1$-$C_4$ alkyl chain, a phenyl group substituted by a linear or branched $C_1$-$C_4$ alkoxy chain and a phenyl group substituted by a trifluoromethyl group;

and a pharmaceutically acceptable excipient.

**11.** Composition according to Claim 10, **characterized in that** it additionally comprises, as active principle, at least one antibiotic chosen from antibiotics which can be activated enzymatically by EthA.

**12.** Composition according to Claim 10, **characterized in that** it additionally comprises, as active principle, at least one

antibiotic chosen from ethionamide, prothionamide, isoxyl and thiacetazone.

**13.** Product containing at least one compound according to any one of Claims 3 to 6 and/or at least one compound of general formula (I):

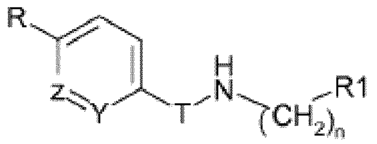

(I)

in which:

Y and z are chosen, independently of one another, from CH and N;
T is chosen from CO or $SO_2$;
n is an integer greater than or equal to 1 and less than or equal to 3;
R1 represents a group chosen from linear or branched $C_1$-$C_3$ alkyl chains, linear or branched and substituted $C_1$-$C_3$ alkyl chains, $C_3$-$C_6$ cyclic groups, the cyano group, the azido group, $C_1$-$C_3$ alkoxy chains, an unsubstituted phenyl group, a phenyl group substituted by one, two or three substituents selected, independently of one another, from linear or branched $C_1$-$C_3$ alkyl chains, trifluoromethyl, linear or branched $C_1$-$C_3$ alkoxy chains, halogens, 6-membered heterocycles comprising 1 or 2 nitrogen atoms in the ring, 5-membered aromatic heterocycles;
R is chosen from the azido, cyano, or 2-benzothiazolyl groups and the following groups (R-1) to (R-10):

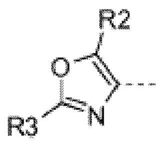
(R-1)

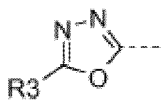
(R-2)

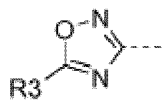
(R-3)

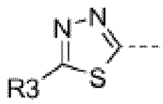
(R-4)

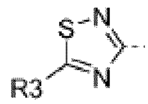
(R-5)

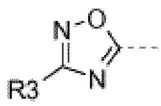
(R-6)

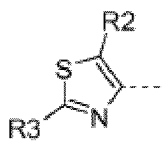
(R-7)

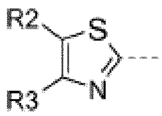
(R-8)

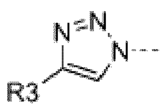
(R-9)

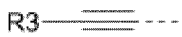
(R-10)

in which:

R2 is chosen from H, F, Cl, Br or I;
R3 is a group selected from H, linear or branched $C_1$-$C_6$ alkyl chains, linear or branched $C_1$-$C_6$ alkyl chains which are substituted by at least one fluorine atom, cyclic $C_3$-$C_6$ groups, the cyanomethyl group, the azidomethyl group, linear or branched $C_1$-$C_4$ alkoxy chains, $C_1$-$C_4$ hydroxyalkyl groups, $C_1$-$C_4$ alkyl methyl ester groups, $C_1$-$C_4$ alkyl methylcarbonylamino groups, $C_1$-$C_4$ alkyl methyl sulphone groups, the unsubstituted phenyl group, a phenyl group substituted by one, two or three substituents selected, independently of one another, from $C_1$-$C_3$ alkyl chains, trifluoromethyl, $C_1$-$C_3$ alkoxy chains, optionally substituted aromatic heterocycles, substituted aromatic heterocycles, aromatic heterocycles substituted by at least one fluorine atom or by a group chosen from the following groups (II-a and II-b):

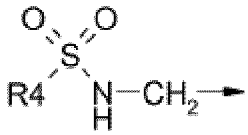

(II-a)

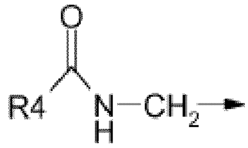

(II-b)

in which R4 is a group chosen from H, linear or branched $C_1$-$C_4$ alkyl chains, the phenyl group, a phenyl group substituted by at least one halogen atom, a phenyl group substituted by a linear or branched $C_1$-$C_4$ alkyl chain, a phenyl group substituted by a linear or branched $C_1$-$C_4$ alkoxy chain and a phenyl group substituted by a trifluoromethyl group;

and at least one antibiotic chosen from antibiotics which can be activated enzymatically by EthA, as combination products for a use which is simultaneous, separate or spread out over time in antituberculous, antilepromateous or general antimycobacterial therapy.

**14.** Product according to Claim 13, **characterized in that** the antibiotic chosen from antibiotics which can be activated enzymatically by EthA is chosen from ethionamide, prothionamide, isoxyl and thiacetazone.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- Activation of the prodrug ethionamide is regulated in mycobacteria. *Journal of Biological Chemistry,* 2000
- Ethionamide Boosters Combining Bioisosteric Replacement and Structure-Based Drug Design to Solve Pharmacokinetic Issues in a series of potent 1,2,4-Oxadiazole EthR Inhibitors. *Journal of Medicinal Chemistry,* 2012
- Remington's Pharmaceutical Sciences